# EUROPÄISCHE PATENTANMELDUNG

(11) **EP 0 953 640 A2**
(43) Veröffentlichungstag der Anmeldung: **03.11.1999**
(21) Anmeldenummer: 99104881.0
(22) Anmeldetag: 11.03.1999
(51) Int. Cl.: C12N 15/29, C12N 15/82, C12N 1/20, C12N 5/10, C12P 21/02, C07K 14/415, A01H 5/00, C12Q 1/68, G01N 33/53

(54) **cDNA-Sequenzen von Genen, die an der Induktion von Resistenz in Pflanzen beteiligt sind**

(30) Priorität: 25.03.1998 DE 19813048
(71) Anmelder: Bayer Aktiengesellschaft, 51368 Leverkusen (DE)
(72) Erfinder: Schreier, Peter Helmut Dr., 50674 Köln (DE); Garbers, Christine Dr., 79540 Lörrach (DE); Langen, Gregor Dr., 35390 Giessen (DE); Kiedrowski, Siegrid Dr., 53604 Bad Honnef (DE)

(57) **Zusammenfassung**

Die Erfindung betrifft allgemein Pflanzenresistenzgene und insbesondere cDNA-Sequenzen, welche verwendet werden können, um bisher unbekannte Gene zu identifizieren, die an verschiedenen Signalketten zur Induktion von Resistenz in Pflanzen beteiligt sind.

SEQUENZPROTOKOLL

<110>	Bayer Aktiengesellschaft

<120>	cDNA-Sequenzen von Genen, die an der
	Induktion von Resistenz in Pflanzen beteiligt sind

<130>	Le A 32 938

<150>	DE 198 13 048.1
<151>	1998-03-25

<160>	213

<210>	1
<211>	207
<212>	DNA
<213>	Nicotiana tabacum

<400>	1

gtgcagtgat aagcattaac taataatggg aggaaaatgg ctctttccac ttgtcgaaag	60

gtatattcaa attaagttga ggtatagaat ggtagcattt tacaatgtta gcgcagtaat	120

gaaacagtat ttccagattt catgcatgtt tcagcacgct tttgctgcga cagttaaagt	180

gcatggttaa ttaccaactg gtgatct	207

<210>	2
<211>	275
<212>	DNA
<213>	Nicotiana tabacum

<400>	2

gtgggaaaag gcaatatcag tgctcagata acaaacaatt cacatctgaa cccggtactt	60

ttcatgcgga ataaatatgc atacaaatta actaatattg caacatacaa tagatatgaa	120

cccataactt taaaaatata atgagttcag gcaagacatg acgcagtttt agattttcgg	180

ggacatgacc cttgatagga aggtgtggag gtcgagcatt agggttgaag gttaggaggt	240

agttgaggct tttctacttt gtttaactgg atcaa	275

<210>	3
<211>	194
<212>	DNA
<213>	Nicotiana tabacum

<400>	3

gtggtgtaac acgtccctga tctggtcatc ctgcgagcaa ctacggagtc tatcatttta	60

ccctatcctt ttgactggta agtcaatggc actattttct agtctcgact ctactaattt 120

ctaacagcaa aagtagacct ttgttggtat gtttgtgcaa tcacaggagg tttcaaatat 180

acagtaataa atgg 194

<210> 4
<211> 646
<212> DNA
<213> Nicotiana tabacum

<400> 4

tttccacaag gaatcgtgat gtctttctac ggaattgcgg gtctctttat tagctcttat 60

ttgtggtgca caatttcctg gaatgtaggt agtggttatg atcgattcga tagaaaggaa 120

ggaatagtct gtatttttcg ttggggattt ccgggaaaaa atcgtcgcat attcctccga 180

ttccttataa aagatattca gtccgttaga atagaagtta aagagggtat ttatgctcgt 240

cgtgttcttt atatggacat ccgaggccag gggtccattc ccttgactcg tactgatgag 300

aatttgactc cacgagaaat tgaacaaaag gctgctgaat tagcctattt cttgcgcgta 360

ccaattgaag tattttgaga aattgagata tcactatcag gaaacaatat tctgaatttc 420

ttcattcgaa gtgaattctt agcttttttc tggattcttt ctagattcaa agactaccac 480

aaatcacaag aaaatgattc attagtccat accttgtata aaactcatgg tgggtaaaaa 540

aatcatccgc tacagtacaa atggggttga ttaaccactt cccaaatgaa aaaaatgggc 600

acaacaaacc aaaaacgggg gcgaaatttc cagccacctg ggccgg 646

<210> 5
<211> 171
<212> DNA
<213> Nicotiana tabacum

<400> 5

ggacaaggaa tgaaatacca tactcaatat atagtacaaa tcacacgtga gaacttatat 60

attgtcaagc cttattgcac agatatccta agtagtggcc ttgtagctag cttattaata 120

atggacaaaa gctaagttcc agtacatttt acagatccat tgctgcgaaa c 171

<210> 6
<211> 198
<212> DNA
<213> Nicotiana tabacum

<400> 6

caaaaggtgt agcattttct gcagattatt cgactaatgc ttctttagca attattattt 60

tcactattac agagaagtag agccactaat aagcatgttt atgaattcat gaatgatcag 120

atgcatccag atctctctta ttcaataact ggaattaccg ctacttctca tctttcttgc 180

ctgcgaaagg cggatccc 198

<210> 7
<211> 259
<212> DNA
<213> Nicotiana tabacum

<400> 7

tttcacagca ggcatctaat tcatagttta aagcaatcac ataggtaaaa tacagcaatt 60

gcctcacagg gtagcttaat aagtatgaaa ttagcattgc aaaaaactct attgaccatt 120

acaaaagctc tgtgtacttc taagcaacag gggtcacctt aacttcatcc acaacaggac 180

cacaaagaga tccatagtca ttgatctttg tatggtaata agagctgtag aaagtaattc 240

ttgtcctgtc tgcgaaaac 259

<210> 8
<211> 455
<212> DNA
<213> Nicotiana tabacum

<400> 8

gatcaatcgc ccaaagggat gaagactatg agcactggcc ttttcttgac aaccctttcg 60

cttggtttct tcattagcag cttcttagta tcggttatca agaaggtgac cggaggcaat 120

ggtactgatg aaggctggct tgcagacaac ataaacaatg gaaggctaga cttgttctac 180

tggcttcttg ctgtgttggg agttataaac attgtgattt acctaatttg cgcaacatgg 240

tacaagccaa ggaagcctaa gtcagccata cagatggaga atccgcacac taaaaatgca 300

gctgaagaga agtgctagga tttatgctgt tcaagttgcc cttttcttaa ttttattagc 360

caatatacag tgaaagagct gttgtttcag tttagtttct gttctctata tatcgtgttt 420

ggatcagtaa attggttttc ggttttgcag gaaaa 455

<210> 9
<211> 440
<212> DNA
<213> Nicotiana tabacum

<400> 9

cctgataagc tttgaggttg tagctgcgga attggctgag acttccaact caatgaaatc 60

ggataacgag aatgaagtac actttgacgg acgtagcgga tacaatggca ttggcaatga 120

tggatattat gggggtggcc atggtggtgg ttataaacgt agaggatgcc gctatggttg 180

ctgcaggaaa ggttacaatg gttgcaaaag gtgttgctcc tatgcaggtg aggccatgga 240

taaagttact gaagttcagc ctcacaactg atcactatat gtaaagagtt taagttatat 300

ataccettag tatgtataac cttgtacgta ctatgtagtg acaagatgta ataatcttgc 360

tactttagac cttgcttgta acaagtatga ataaagccat tcggttcttg tggatggttg 420

gtcatgtaat gttttgttgg 440

<210> 10
<211> 287
<212> DNA
<213> Nicotiana tabacum

<400> 10

cctaaaacat gtatatcaaa cttcaaatgc tccttttcat gtttgaaatt gaaataacag 60

ctaatttgat tagatccttg agaaataaac gtcgtaagct gcactatttt tgccgattaa 120

cctttaacga tcagattctt aagaaaacaa tctacatccg tacaagttat aacatatatc 180

atcgatctgg agtacatgtt aattacttaa attactacta tcgtaattcg taaaccttca 240

caaaataaat ataacacgtt ataagtgata attcagattt gctttgg 287

<210> 11
<211> 251
<212> DNA
<213> Nicotiana tabacum

<400> 11

ccgcagaata aatgcacgct gtgcaatcgg ttgtgtcttt actttttctt ctagttaagc 60

cttgtttgtc tttttatata tgatatctta agttttgagg gtgaaaacta agaagtactt 120

gaattatttc tagaatgtta aggaatagtt tatacattgg aggcgcgtga ataacttaaa 180

gattaaatct cttattttat gttcattatc tttacttttt atgcctataa aaggtcatgt 240

aattgcaatg g 251

<210> 12
<211> 477
<212> DNA
<213> Nicotiana tabacum

<400>	12

cccacacggt tggtaccacg caaagcgcga atatatatac tagttaaata tatgtctggt	60

ataaattttc gaaaagagat tcacatatta aataacaagc atgcttatga agttattcgt	120

acaccaattt ccaaacattt atgaataaga taactaatac atactattaa gatgcctaaa	180

aatgaaagat ctaagttata catacaaaca acgtaacaac actaccactc tagctaattt	240

aattgattat aacacgtgcc gtgttcttat tctagaatat taccccacga atgttaatat	300

aaatagactt caaaacaatt taattgagta aatattaatt ctttatagta ttagtgaggg	360

agcaatattt tgttgaactg ttcattttgg atttaagact tcacatattt gatcaaaggt	420

taaagataat attctaaagg aaagggaaga gatttgcatc ccttttaagg tagtcgg	477

<210>	13
<211>	180
<212>	DNA
<213>	Nicotiana tabacum

<400>	13

cagtactaca agaggtggag tgtcgattga tgatagtccg cgaatgaatt cttcaggaaa	60

aattcttttc cataatttag gaagaagttc gagcagtcca agcagtttta atggtggtgg	120

agcaaggtat aaacatgagg aggaggaatg gagagatctt attcagctaa tgttcgtgtt	180

<210>	14
<211>	220
<212>	DNA
<213>	Nicotiana tabacum

<400>	14

gcgtgtggct tgtggaaggg ggttaagatg tttcctattt taaattaaaa taagaaataa	60

aatagtcaga tccataacta aagcgagtgg gtaacatttt tgagcacatg gatttcatgt	120

ggaagagaat ttaatgaaag gttgtcctga ttaattatga taatttaaac tttgagtaaa	180

tactaacgtg atgaaatcga ggagacggtg cagattctca	220

<210>	15
<211>	397
<212>	DNA
<213>	Nicotiana tabacum

<400>	15

atcaggatgc agaaggagtg gtaggcacgg agatacgcaa caaacaagac attgttccac	60

tcctggtggt gttgctgctt ctgtgactgt tgctgcagct aacgaagagt cacacataaa 120

cgaaaggagt caacaacaat cttaatttct gtaactagaa tcgatcgtaa ggatagtttt 180

gctgctttat ctcccacttt taagagtata ttccatgtgt tgtgctttac tgaccacttt 240

ttgttttgta ttggttgcca cttgtaagca acccgacaca ttgctagttt aatgtttgct 300

ttagcttgta taatagtgga gtcagttata gtcatggaga tgttgtatgt tatcccttgt 360

gtttttaagg ccgcgcagca actactgttt agtgtcc 397

<210> 16
<211> 384
<212> DNA
<213> Nicotiana tabacum

<400> 16

atcaggatgc agaaggagtg gtaggcgcgg agatacgcaa caaacaagac atgtccacta 60

ctcctggtgg tgttgctgct tctgtgactg ttgctgccag gctaaacgaa aggagtcaac 120

aacaatctta atttctgtaa ctagaatcga tcgtaaggat agttttgctg ctttatctcc 180

cacttttaag agtatattcc atgtgttgtg ctttactgac cactttttgt tttgtattgg 240

ttgccacttg taagcaaccc gacacattgc tagtttaatg tttgctttag cttgtataat 300

agtaggagtc agttatagtc atggagatgt tgtatgttat cccttgtgtt tttaaggccg 360

cgcagcaact actgtttagt gtcc 384

<210> 17
<211> 203
<212> DNA
<213> Nicotiana tabacum

<400> 17

gatctaaccg gcgtgaaggt tacagaatga tattttgttc tttacgtctt cgtattatgt 60

tgatttctat atgtatatcg tgtgattata ctttatcatg ccttatgttt tggcaagtgt 120

tgttacatac tggttgtctg atgtaaaatg tgtcttcagt aatgactaga taaagcggtg 180

ttcctcgttg gtttggtatg ggt 203

<210> 18
<211> 254
<212> DNA
<213> Nicotiana tabacum

<400> 18

ggccagtgtg atggatatct ggcaggaatt cggctgatct gactggaaag ctcttgaaga 60

caattagtga tctggaccat gcagcaaaga ccaagaacag ccctgaagcg gagaagtact 120

atcctgatac tgtatctacc ttaaatgatg ttttggccaa ccttggttaa aaaagctttt 180

ctgtactagt atgttattac ttcctgtaag ttatcgaata cttcttgaat ccaattgtga 240

agaatgattt tggt 254

<210> 19
<211> 384
<212> DNA
<213> Nicotiana tabacum

<400> 19

gatctcagac ggcactgtat taaatggaca aggcttcaat aactgtgaat ttgagactct 60

ccaatgcttg ggtaatcaaa cttctggaat ggaggaggtt ccttgtcctg tggataatgg 120

cacacctact cctactgcca acacttctgg tcgaagcaag agaaaaggag gcgcacgtac 180

aacaacagca agttgatgaa ctgcacagga gaagcatctt tgttcttggg aataagtacg 240

ttttgttatg tgctctctct tgggatttct tcccagctta tctactttgt tgagttcaat 300

tataatctcc ttcgggctgt tgagttggtg gcctactact tatttatcaa gagtcatgtt 360

aagcaggaac aaattgaagg aata 384

<210> 20
<211> 270
<212> DNA
<213> Nicotiana tabacum

<400> 20

gatctcagac aaagaaaaag gtttcattga attattaaaa caattgactg tttgtgattc 60

tgtaacagac gaaaaattca aggaaagatt cgaagagatt acaaaatatg gagatgaaca 120

tattatttgt gtgattgaag atgttaaatc aggaaaaatt gttgcaacag gaagtgtatt 180

tattgagaaa aaattcgtaa gaaattgtgg aaaagctggt catattgagg atgttgttgt 240

tgattcaagt acacgtggca tgcaattagg 270

<210> 21
<211> 188
<212> DNA
<213> Nicotiana tabacum

<400> 21

agccatacac cagaaaccca tattgatgtc caaaagacct cccgatcagt atccgagaag 60

tgccaagtcg ggcaaatgga agtctggagt aacactctgg tccccagcag aaaggaaatg 120

agagaatctt atcagtaaca ccccatcccc agcgaaaaga aataaaatga gagtctattg 180

gtgaaacc 188

<210> 22
<211> 551
<212> DNA
<213> Nicotiana tabacum

<400> 22

ctgcttgatg aaaatactct cccaattcat ctgcttcatt ctcttcttga tcgaatgagt 60

caaagaatga cctcttccct ccattaacac tgccgaaact gaccatagaa ctagatcctg 120

aatgataaaa tggccataga atagattact acgatggtgt caatcttgac aaaattcatc 180

cattagatag tctgaaattt taaaaacagc tgtaatttct gtatactact cattgcactt 240

cttttccttc ttcccattaa aatttgctat aagaattcaa tttcataaat tgatgtgtta 300

tcatctacct tacctgatcc aaacttagag ccaatcaacc aaatggcgca cacatgatca 360

accaccaaaa atgatataac tagttatccc aacagagtga ttcaattcta tatattacaa 420

gggaattatc ataaattcat ttgaagaatt aagattcaaa tttataccaa aatactccac 480

caaatacaga tattcatctt cttctgcaag aattcatctt ttaacatata atcacagcac 540

tcatcaagca g 551

<210> 23
<211> 276
<212> DNA
<213> Nicotiana tabacum

<400> 23

caccaccaca aaacaagtat atagcaacga taaacaacct ttatcacaat aattattata 60

tattgcactt cttagcagta catcatattt aaccagaaag cacaaaactc cacatcgaca 120

cacgcaccca cacccatgtg tatacacaaa agactccctt tttcctaaca gtcacttcac 180

catttgttta tgtaaattgt tgtttggttg cactggttta atgactgcac taccaactct 240

aaggttacaa agcaaagcat cacgtccggt tagatc 276

<210> 24
<211> 207

<212>	DNA
<213>	Nicotiana tabacum

<400>	24

gatcaatcgc tgagggagca tgagaggttg aaaaggaacc atcttgttaa ttcttctccg	60

gagaagacaa agcatagaaa ctatttcttc tcttctttat ccaagtcaat ttcaaaaatt	120

gtaggtttag ttgtatttct tatcctccac tattttcctt gaaagcacaa tgcacctctg	180

catagcttca tgcaagtaat agagtat	207


<210>	25
<211>	242
<212>	DNA
<213>	Nicotiana tabacum

<400>	25

cattagaagg gagtgtttta ttatcctaaa ccaggattta tgccatgtaa cagaatctca	60

agtagtacct ctattacttg catgaaatgc agatgtgcat tatactttca aggaaaatag	120

tggaggataa gaaatacaac taaacctaca atttttaaa ttgactagaa atacagcaga	180

acaaatattt tctatgcttt gtcttctgcg acgaaaacta caaagatggt ccttttcaac	240

ct	242


<210>	26
<211>	186
<212>	DNA
<213>	Nicotiana tabacum

<400>	26

cagaagggag tgtattatta tcctaaatca ggatttatgc catgtaacaa aatctcaagt	60

agcacctcta ttacttgcat ggaatgcata tgtgcatttt ctttcaagga aaatagtgga	120

ggataagaaa ttcaactaaa cctacaattt tttaaattga ctagaaaaac accagaacat	180

atattt	186


<210>	27
<211>	171
<212>	DNA
<213>	Nicotiana tabacum

<400>	27

ctttctaccc tacagatata gcctagtcca aataatacat atattttgtt atataatgta	60

ctgaaaaaca gattatttga gtgtgtggct tgtgctattt catcgttaaa ttgtagataa	120

ttccatatcc acttatagaa cactgtttaa taatagtgct ttgctgtctt g 171

<210> 28
<211> 113
<212> DNA
<213> Nicotiana tabacum

<400> 28

taacgtctaa aattattacc agcactcata tagtttaata tcaacacgaa gtcgaaaata 60

aacttcaccc tcaaaggcat cagtcatgag agtagcaatt cctcttgtca tga 113

<210> 29
<211> 171
<212> DNA
<213> Nicotiana tabacum

<400> 29

ctttctaccc taagtagcat gctgaagcaa taaaatgtcc ttgagcaaaa aaactcatcc 60

tgtttacttt taccttacac cttgaattgc agttgctagg ttgaactttg taatatatat 120

actactcttt taatgtgcta aaaataagaa ctttggactt ccctttacct g 171

<210> 30
<211> 312
<212> DNA
<213> Nicotiana tabacum

<400> 30

gatctaaccg gcgtgaaggt tacagaatga tattttgttc tttacgtctt cgtattatgt 60

tgatttctat atgtatatcg tgtgattata ctttatcatg ccttatgttt tggcaagtgt 120

tgttacatac tggttgtctg atgtaaaatg tgtcttcagt aatgactaga taaagcggtg 180

ttcctcgttg gtttggtatg gtttttaaag atttccgttc tgtattttcg gtattcaact 240

tggatatgtt ataccgatat catactaaat aattagtatg gttcggttat gcatttgtgc 300

gattagtact tc 312

<210> 31
<211> 161
<212> DNA
<213> Nicotiana tabacum

<400> 31

gacacacgca cccacaacca tgtgtatacg caaaagactc cctttttcct aacattcact 60

tcaccatttg tttttgtaca ttgttgtttg gtagcactgg tttaatgact gcactaccaa 120

ctctaagatt acaaagcaaa gcatcacgtc cggttagatc a 161

<210> 32
<211> 152
<212> DNA
<213> Nicotiana tabacum

<400> 32

gacgacagga aatccaaata tgtttataat caagaaactt cataccaaat aaagcaacaa 60

cattttcata cataataaaa ttcaaatgca aaattaacaa caaactctat tttacatcac 120

cacatacttg acactactta ctatcagttt cc 152

<210> 33
<211> 242
<212> DNA
<213> Nicotiana tabacum

<400> 33

ggattggtca gcctagtagg aattatcttt ggatattatg taggaaaaca catcttgacg 60

aagagatata caacatgctg attgagaagg ctaaagaagt aggctatgat gtgagtaaac 120

ttcacaagac acctcagtct gatcctccac cagagggtga cgatacccac aggacaccaa 180

aggaacttgg tggcttaaat caatctttgg aaaataaaca tgcagtaaat atatatatgg 240

tc 242

<210> 34
<211> 99
<212> DNA
<213> Nicotiana tabacum

<400> 34

cattatgctg agtgatatct ggcttaatga ctggtaccct gcctgcagaa catgttacta 60

tctcatatgc acaacattat gaactaacca gtaccccga 99

<210> 35
<211> 286
<212> DNA
<213> Nicotiana tabacum

<400> 35

tggattggtc aaatggtcaa aacatgatga tcagttaaga gacacgagtg acaacaaata 60

cggggaaagg aaagctaaat atgcacgaga gacaattcga ataatacaag tttcgtacct 120

atttaggtca tttaaagcca agagatcaga aggcattgaa gacatggata tgatgacaaa 180

aaggagtcca aattcaatat taaataccca atacgttaga gaattggtat taaataggaa 240

tgattcattg tgtaacgtct tatttaatgt catttattgt ttataa 286

<210> 36
<211> 112
<212> DNA
<213> Nicotiana tabacum

<400> 36

tggattggtc aacaaaagga gataaagtga attggggatg taatcaaacc ttaaaattgt 60

gaagatacat acgttgagtt tatgttcaca atgtaaaatt ttatactatg gt 112

<210> 37
<211> 228
<212> DNA
<213> Nicotiana tabacum

<400> 37

tggattggtc actacacaat tcctgaagag actgatgaag tttgtcggtg gctagctgca 60

aatttgcgtc ttggggggac atgaaagtag ttgctgaagc ttcggtatac gcgatataca 120

cggaaaataa cacccatgta tatgaacagc tagcctaggg agagtggttc atggtatact 180

tgcattattg tgcatcctat tattgtctat tagtctgttg gattggtc 228

<210> 38
<211> 509
<212> DNA
<213> Nicotiana tabacum

<400> 38

cgatacaggt actaagcgaa gacaaagaag agactgcaaa aacagcacat tcttactgct 60

ttaattgtta tgtttttcat gctttggatt ttgttaggac aatagtgtct tgttccttta 120

aactattgtt ggggatattt tgggattttt ggaatgttgt tgctggaaaa gaattggtct 180

catttcatct gatggaagac caaaatgtgc tgaataagtt ttgaggaatg gaggtggtgt 240

gcttgatatt gtcaatcgat acaggtacta agcgaagaca aagaagagac tgcaaaaaca 300

gcacattctt actgctttaa ttgttatgct tttcatgctt tggattttgt taggacaata 360

gtgtcttgtt cctttaaact attgttgggg atattttggg atttttggaa tgttgttgct 420

ggaaaagaat tggtctcatt tcatctgatg gaagaccaaa atgtgctgaa taagttttga 480

ggaatggagg tggtgtgctt gatattgtc 509

<210> 39
<211> 296
<212> DNA
<213> Nicotiana tabacum

<400> 39

gccgccatgg ccgcgggatt tacaacgagg catgtggact tcctatagct atttggaaaa 60

aataattcat ggattacgta catgtgagaa atacatgtag catttggttg gacttcttcg 120

tgaatgtaat atgaagagcg gagcaagatt ttcagtactt gaatgctctg atgcaaaatt 180

atttcttatt tcaaaactgc agttattttg ttattatgtg acttactgtt gctgatttcc 240

aagtgtcaga tgtatctgtg tccatagaat cgtaataact ttaactcgct cacgtc 296

<210> 40
<211> 343
<212> DNA
<213> Nicotiana tabacum

<400> 40

catcaactcc ggcagccgct tctgcattgc agcaaagctt tggcctcaat gaaaaagtgg 60

gcaaaggtaa gctacttggc ggaggagaaa tagtaaacac caaagaactt gaaaacttgt 120

taactttatc caataggtcc agagtggtac tgtccttttt cggattttct aaattggaga 180

agtttagtgc gtcgaactgc tcttctttac ctgttgatga catagagaaa accatggaaa 240

catctttggg cagatgcttt gggtttggac ccaaaaggtt agtagatgaa atcataacaa 300

cttttgatga ttttgggttc tcttctttct taggacttga tca 343

<210> 41
<211> 463
<212> DNA
<213> Nicotiana tabacum

<400> 41

gattgagaat aagttgattc tccaatagct aacaaatgaa ctcaatagtc agctaataga 60

agaggaactc aaaaagtgga tgcagaatag gtggagaaca acatcacatt aattgaagga 120

ttgtaagaag atcagcgaaa ggtagagaac caattgcaag gtatgtaaac atgtgtacgc 180

ggctataact ggaagacacc attgagatat cctcctaggc tgagcatcat gggggaaaat 240

ataattagaa agagagaagt taaataagtt gaaaagctat atagttggtc actccttaac 300

gtatggataa gcaggagttc ttgaacaaat ttgactgctg aggtgagatg atatttacat 360

tagtggggga ttaaaatgag gatttttcaa gattttagat ttagaagaca agtgaaggaa 420

ttgaagcttg ttagtgccaa ggagctgttg caggacttga tca 463

<210> 42
<211> 370
<212> DNA
<213> Nicotiana tabacum

<400> 42

tgatcaagtc ctaagaaaga agagaaccca aaatcatcaa aagttgttat gatttcatct 60

actaaccttt tgggtccaaa cccaaagcat ctgcccaaag atgtttccat ggttttctct 120

atgtcatcaa caggtaaaga agagcagttc gacgcactaa acttctccaa tttagaaaat 180

ccgaaaaagg acagtaccac gctggaccta ttggataaag ttaacaagtt ttcaagttct 240

ttggtgttta ctatttctcc tccgccaagt agcttacctt tgcccacttt ttcattgagg 300

ccaaagcttt gctgcaatgc agaagcggct gccggagttg atgccggagc tactgacaat 360

ctccggcgca 370

<210> 43
<211> 194
<212> DNA
<213> Nicotiana tabacum

<400> 43

tttgggctcc tgatgattgg acttatggta ctctagttga tggcttgtgt gaaagaggca 60

gagttgaaga agctaacacc atttttagtt ctcaaaagag aaaggtataa aggttaatgt 120

tgcaatgtac actgctctaa ttgatggaca ttgccgagct gaaaaaagtt gaattttgca 180

ccttgacatt gtgc 194

<210> 44
<211> 176
<212> DNA
<213> Nicotiana tabacum

<400> 44

ttttggctcc ctgagattgg acttatggat actcaagttg atggcttggt gtaaagaggc 60

agagttgaag aagctaacac catttttgtt ctctaaaaga gaaaggtata aaggttaatg 120

ttgcaatgta cactgctcaa tttgattgat ggacattgcc gagctgaaaa acgctt 176

<210> 45
<211> 169
<212> DNA
<213> Nicotiana tabacum

<400> 45

ttttggctcc catgactaac tgggatgttg aaccgtgaga tgtagaaatg tttagaactt 60

ttgtttatct taattaatct taagcatgtt ggtttccagg atcacccttt tgtggtttga 120

tctcttttca agtactttgg gaatacaaga gtggattggt cactcacag 169

<210> 46
<211> 225
<212> DNA
<213> Nicotiana tabacum

<400> 46

ctgcttgatg agagctttgt tcttgtagct tacgtgaaat tttgccttag ctagcaatcc 60

ctcagtatca aactctcctg attcaacatc aatgtttatt tcagtaatga tcttgacgag 120

atcaactatt aatccaggtc tatctgctgc ctctacatac agtaagcttc ggtcaggacc 180

atcgtcatag acatggatat gggttgctat gtccacatca agcag 225

<210> 47
<211> 306
<212> DNA
<213> Nicotiana tabacum

<400> 47

ctgcttgatg ttcgaagaga atggcacaat agatggggat gttacacatc caatcaaaat 60

agagtggttt aaatggacat ttgcgtatcc ggagtattat gtaatagagg gataactacc 120

agagtgaaat gcaaattcta taaaacgatt gttagaccac caatattaca tatatgggag 180

taaatgtagg tctctaaagg ctccaagtat ccacaagata agtggcgcaa agatgaatga 240

tcaagatcca aattcagata atgttttact ttctataaac tttcagttac gaaaaagtgc 300

tacata 306

<210> 48
<211> 410
<212> DNA
<213> Nicotiana tabacum

<400> 48

gatccaatac tgtgacaata ttggttgagt ctgctaaata ttttattttc tacaatacca 60

ttcctttaat tccttatcat cattaagaac aaacatgaaa gtagcagtag ttggggcagg 120

aataagtgga ttggtttctg catatgaatt ggcaaaagct ggtgttgaag ttgtggttta 180

tgagaaggaa gattatctcg gcggccatgc taagactgtt actgtcaatg acgttgatct 240

tgaccttggg tttatggtct tcaatcgggt aacttatccg aacatgatgg aattttttga 300

gaatcttgga gtcgatatgg agatctccga tatgtcattt tcagtgagct tggacaaagg 360

ctatggttgt gaatgaggta gccgaactgg actctctggt ttgtttgcac 410

<210> 49
<211> 658
<212> DNA
<213> Nicotiana tabacum

<400> 49

atttccagat ttcatgcatg tttcagcacg cttttgctgc gacagtaaaa aattgcatgg 60

ttaaaccaac tggttatttt ctctcattct gcactgtatt aaaaactaga ctcttctgtt 120

gttggtctcc tcaagtaata gcgtagactg tttgccagaa cctgttgaag gggcttgatg 180

agagctttgt tcttgtagct tacgtgaaat tttgccttag ctagcaatcc ctcagtatca 240

aactctcctg attcaacatc aatgtttatt tcagtaatga tcctgacgag atcaactatt 300

aatccaggtc tatctgctgc ctctacatac agtaagcttc ggtcaggacc atcgtcatag 360

acatggatat gggttgctat gtccacatca agcttttgat ttggttgata agcaccaaag 420

acttcaccca tagctaacag ggcgctagat tccggatgga actcaatcat attgttgata 480

attgtcaaac gaattgcttc tagttgctct ggatcgtcga cctttctacc agtagaagca 540

tttgtgatgc ataatgtagt atgcttccct gacgaatcta gacaaacatt agctttgaca 600

acatttagtc caagattttt tagtgcactc atcgtgtcac aagagcccca atgcgatc 658

<210> 50
<211> 459
<212> DNA
<213> Nicotiana tabacum

<400> 50

gtagaggaaa gatccaagtc caaaggaata aaggaaagag caagtgacat caaccatttt 60

gctctatcaa ggcacaaaca tggtaaagta ttatctcagg ccatcaaaca tatcagtttg 120

atctgattct cgatttagtg tgtaggtgaa tgcaatggtg acaaacagag gagacacaat 180

gaagtaaatt gattttatgt gacaacgaaa cttcttatga ggaaagaaac taatatgttg 240

tctttatcac ccttttacag agatacttct tgtaagatat gtgcagtttt atcaaaggca 300

aaaagcgcaa aaaagctcta actgtctgtt gaggctttaa gcgcaaagca caaataaagc 360

gtgggcttta ataaaaaaag gcgcaaatgg agaaaaacta caaatatgta tgtgtagtcc 420

aagactaata tctataagca tgaataccaa atattggac 459

<210> 51
<211> 329
<212> DNA
<213> Nicotiana tabacum

<400> 51

gtggaaacca agaaataaat agcacgataa tacttggaag ttggaaacag gtgtagtaac 60

tttcaacccg tgacatagat aagataaatc caaaacagca aaatgcaccg acaataccaa 120

tgtgccacgc cgtgaaaact atctctaata attaattccc cacattaaga aaaaagaaaa 180

aacggaaaag gagaaaatcc cattaaaatc taaaagcagt acttcagctg caggtttcac 240

gagcaacgcc gagcttagaa ttaagaatgt cgaagaggaa gcgatgattc tgctgctgcc 300

agttggcgat gacgttgagg acggagttt 329

<210> 52
<211> 469
<212> DNA
<213> Nicotiana tabacum

<400> 52

gtagaatggt gggattggct tgtaaattgg aacaggtggt ggcagaggtt ttacgactgg 60

tggctcgtat actggaactg gaggaggcag tggcttcact actggcggct tgtatactgg 120

aactggagga ggcagtggct ttactactgg tggcttatat actggaactg gtggaggaag 180

tggttttact actggtggct ttggtgttgg tttatagatt ggaactggag gaggaagtgg 240

ctttactact ggtggttctg gtttgggctt gggtttgtaa gtgggaactg caggaggctt 300

tgcaacaaca gacttattcg agcatttgag ttttaatttt tgttgaaata ggtgttcttg 360

tttggtgatg attttgtgtt gaaatagtca ttagggtaag tataagttgg agacaaactg 420

agtgatatgc agtctgtatt ttaaagcatc gggatgtatg gttttgcac 469

<210> 53
<211> 373
<212> DNA
<213> Nicotiana tabacum

<400> 53

ggatttggta aaggggggag aattctgaag cgtttagtca gagaatttgc tgatggacat 60

cgcaattcac caaattttac ttgaaagttg agcagttgcg tcaaagatat tgaaactgcg 120

gcaccttcat tctatataag ttgagttttg tcctggaatg cagccatctt ccttctcgat 180

gagtctcaaa gtccgtggaa aatttatgca ttctttgtac caatgtacag tatccccaat 240

tttgtactat agagattaat tctgtaatat acaacatatc aacaaggcac tgatggccca 300

tttatggtaa ttggtaagta gggtaaaaga atcataaaag gagaaataaa atggaggata 360

acaattattc cac 373

<210> 54
<211> 448
<212> DNA
<213> Nicotiana tabacum

<400> 54

tggtaaaggg caaggatatc accgagctga ttgctgctgg cagagaaaag ttggcttcag 60

taccttgtgg tggtggtggt ggtggtgttg cagtagctgc acctgctggt ggtgctgccg 120

ctgcagcatc agctgctgag gagaagaaag aggagaagaa ggaagagaaa gaggaatctg 180

atgatgacat gggtttcagt ctcttcgatt agaggaatgc agtgttggcc tctgtatgaa 240

atgtcctgtt cccagttccc ctgagtttat tctaggactt cttttgtcaa ctttactgat 300

tttgtttcta gtttagctga aggtacctaa attttgcgag gcaatttgaa attacagtat 360

tgttggattt gtttgcaccc tttagctttg atgtatttgt tgcctgttta agtcgttaca 420

acttccaatt ctaaatccat tatgggac 448

<210> 55
<211> 454
<212> DNA
<213> Nicotiana tabacum

<400> 55

ggtaaagggc gaagcaagga ggacaaggag agctcggagc tgcaatcttt gtccgcaatg 60

gcattggaga aagactataa gcagagagct cccagcaatc tctctgtgct tagttcaaca 120

ttaagaagct tgaaaatcta accatgttag catgtagctt tattggaagc agtaccatac 180

tgttgctgtg tgcccttcaa gatttaagtc gccgcggaac ttttttcttc tcattttcta 240

ctctttgcat tattttgctt atttgtggaa gggctgttta cacttcagta agtaggggag 300

gaaaatggtt atgcaagtta gtgaaaatat tatttttaga caattttggg aaacttcaat 360

gatgaatact atacttgaac ttcatgttgg gtcgttaata aatttctgtt ggaaaagttt 420

gtggagtata ctctattagt taatcgtttg taca 454

<210> 56
<211> 407
<212> DNA
<213> Nicotiana tabacum

<400> 56

gtggtagttc ctttatgtat agatgttagt cttgcactaa caagacttct tatctatatt 60

acaagaagaa aaccgaacgc aaaaattaaa tcagaaagac attatttaag ctctgattaa 120

acgttacata ttacattatg ctttagtcat aataaagttg cacaaatcat aagcatgata 180

taaatttcag gctgcagcaa cagcttctgc aggcttttcg tctgcgggct ttgcttcagg 240

ttcagctggt gtatctgcgg gcttttcttc tgtaggcttt acttcagcag gctttgtttt 300

gggttctgct ggtgctggtg ttgttgcttc tgcttcccag aaagaagtct tctttccagt 360

ctttgaaaca gtctggaaaa cagcttctgg ctccacattt cccttta 407

<210> 57
<211> 234
<212> DNA
<213> Nicotiana tabacum

<400> 57

tggtaaaggg caacccagta taataaactc ccgttatgcg gaggatgcag ggaatggccg 60

gatcacaagg gtctaatttc tgcgagagac tgttttcgca attcaaatcc gtgacctaca 120

aaagtgttgg gaagaagaaa gaaagtaaaa ataactacag ttaagttaaa ttcgattttt 180

acgagtttta aactatatat ctaaacagac aatcagaata aatccaccgt ccac 234

<210> 58
<211> 520
<212> DNA
<213> Nicotiana tabacum

<400> 58

ttttggctcc agagtacatg cagagtggta gagctacaga aaagactgat gtttatagtt 60

ttggagttct ggttcttgaa gtagtaagtg ggaagcggcc cactgatgca acattcattg 120

agaagggcct caacattgtt ggctggttga attttctagc tactgagagt agacaacggg 180

agatagttga tccacattgt gaacgggtac agacggaaag cttggatgcc ctgctttcag 240

ttgccacaca atgcgtctct tcgaatcctg aggagaggcc cacaatgcac agagttgtac 300

agattcttga atctgaagtc atgacacctt gccccagtga cttctatgat tcaaactccg 360

attgaaggtt gtagctaaaa ttagaagaga atttaatgtg gagtgtgtct gctgcgtcat 420

gcaacaatac tgaagaatgg taccagcata gtctggggat caagatctgc aacaattagc 480

agtctactct gtttattcat tctttctgca gatttaccac 520

<210> 59
<211> 528
<212> DNA
<213> Nicotiana tabacum

<400> 59

ttttggctcc agagtacatg cagagcggta gagctacaga aaagactgat gtttatagtt 60

ttggagttct ggttcttgaa gtagtaagtg ggaagcggcc cactgatgca acattcattg 120

agaagggcct caacatcgtt ggctggttga attttctagc tactgagagt agacaacggg 180

atatagttga tccacattgt gaacgggtac agacggaaag cttggatgcc ctgctttcag 240

ttgccacaca atgcgtctct tcgaatcctg aggagaggcc caccatgcac agagttgtac 300

agattcttga atctgaagtc atgacacctt gcccaagtga cttctatgat tcaaactctg 360

attgaaggtt gtagctaaaa gtagaagaga acttaatgtg gagtgtgcgt ctgctgcgtt 420

atgcaacaat actgatactg aagaatggta ccagcatagt ctggggatca agatctgcaa 480

caatttgcag tgtactctgt ttattcattc tttctgcaga tttaccac 528

<210> 60
<211> 558
<212> DNA
<213> Nicotiana tabacum

<400> 60

tggtctatgg cgaaatgaat gatcgagtga gagatgctgt gatatctttg attgataagg 60

aacgcgaagg agagcaaata gatcaggcct tagtgaaaaa tgttttgaat atatatgtgg 120

agatggatga agactctatg aagtattacg cgaaagactt tgaagaatcg atgctgaatg 180

atactactat attttactca aagaaagcct cagattggat tgcaagcaaa tcttatgcgg 240

attacatgtt gaaggtcgag gaatgcttaa agaatgagga agcgaggagt cagagttatt 300

taagatacgg aaccaaacat aagctattgg gggttgtaga atatgagtta cttactgtac 360

atgcaagcaa actggaagaa aagaaacaaa tcaatcttgc agctgatgct tagaaaatga 420

acacatttga gaacattgtg caagtttgtc ccttcttgta ttactaattg ggacgtgcct 480

ttatgtgatt tgagtgcgag tactatttga tcaaagtttt aattgttgat tttcatcaaa 540

taaaaccata tattgtac 558

<210> 61
<211> 331
<212> DNA
<213> Nicotiana tabacum

<400> 61

tggattggtc gcaccaactg cagatgccac agctccgcca aaaacacgag tttgggatat 60

gtggaagatt gaaggaagca agaaggcaag aaaaatttgc cttctgaaga aaggatgttg 120

caaaatgggt atgtgagtag tcaagatgat gagcaaaaga gcatgaacag aaagatgaag 180

cgcctactcc tctatagtgt ttgggataca tgtaacatgt ggccttcaac aaatgtacta 240

gtcgtattca atttgagatg tcgtctcagg gattaattgt agagtaatct tactttattt 300

atgctcgagg tctcttgagc tttctgtgta c 331

<210> 62
<211> 274
<212> DNA
<213> Nicotiana tabacum

<400> 62

tcggtcatag ggatcatatg gtcaaagaca aggttttaca ggaattcttt gatttattgc 60

aatgtgatta aagtgaactg ccaatacaag agcattggca cctcttcggt ggctagcaaa 120

actggattct aactgtttgt tcctctgcta ttccttcggc aacatctgat catcgtggaa 180

tggggactgt tagttgctgt gctctgtttc tcaaatcata gaaggtttga ggtgaacaat 240

aacattaaac cttctcatgc actctacctt gcca 274

<210> 63
<211> 525
<212> DNA
<213> Nicotiana tabacum

<400> 63

gtgagtaatg tgagtgaaaa ttttgaaaaa tagattttgg tggagttttt caaattttcg 60

agaaatttta aaattcattt tccaatgaaa attgaaattt tatggccaat cactgatttc 120

gaaaagaagt aaaaaaattc ttatgtccaa acggctctta aagtaatcag taagatttaa 180

cccaaataat cgctcaccta accacttaaa ctaaaaatag ccggtggatg tataatttat 240

gtattacatg tgtataatta acgtatatgg atacaaaaag taaataataa atatggtagg 300

ttatttgtgt aaaatccatc ggaaagaaaa cgtaccgttg cagtgatcaa actttagggt 360

gccatctttg caatagcaca aggactcgtt ggttgaattg tcgtaaccac aacgcccctc 420

tgatgcctca cacttgccgc actccgttgc gaccccccag tccagcacga acccctcctt 480

catagccgca acaagtcgtc tattcaactc ctcatctatg accga 525

<210> 64
<211> 211
<212> DNA
<213> Nicotiana tabacum

<400> 64

tacaacgagg agaccaacaa gaagcccatg aaaccccgga tatgggagaa caagagatgc 60

ttgctagaag aatatgggaa acatcaaagt aggctggcta cgacaatatt caaggttttg 120

gcatcagacc tgaacctggg accattacag actcagtctc atctctcagt agccactggt 180

ttactgaggg tgtatcgtta ccctcgttgt a 211

<210> 65
<211> 204
<212> DNA
<213> Nicotiana tabacum

<400> 65

tgtggtactg ttgattttga ctttgatttt ggggcaattc agttatcttc ccatggggat 60

atctcatgga aggcttagag tacttgagag ttcttattag ttagtgttac atgttactat 120

tactttagta aagatctttt ccgcagtaaa agttttgttt ggattaaatt ttgaatgcca 180

aatcttcaat atttggttgt tgac 204

<210> 66
<211> 377
<212> DNA
<213> Nicotiana tabacum

<400> 66

gtcaacaaat atttttagtt ttgtacttct gagtgcttta attaagaata ttacagtcta 60

tgattctacg cactagttag tattcaaacc gaataaaccg aagttaccga accgaaagga 120

gaaaactgaa tcatatcgaa tttaattagg tacgattttg gtatagcatt ttaagaaacc 180

gaataccgaa ccgaaatgtc taaataccgg actgtaccga ccaacgaaca cccctagtta 240

gatccaacaa tcattcatca tcacttgtat cataatcgcg cagccacatc ataaaagcca 300

ttgcttcctc aaacaaagct ctgaagcttt ccatatctcc atcagagccg ctttccgttt 360

catctgcgtc tgagatc 377

<210> 67
<211> 186
<212> DNA
<213> Nicotiana tabacum

<400> 67

acccgcaaca ttatgacttt tcatccttag tcaaggatgc aagaagtagg catgacttaa 60

cattacaaaa atcaactagc taatacaaaa ttgtaacaat accaagtttt cccctctttt 120

actccccctc tacttttgat ctttcataat tatctagtac taagatactt atatgctatt 180

agtagt 186

<210> 68
<211> 214
<212> DNA
<213> Nicotiana tabacum

<400> 68

tacctaagcg taaacagtat tttggatgga ccccttaagt tgaggtcaat ggtacttcca 60

gatagataca tagatagatc atggatcacc acttgatgag attgaagcag ctggtttgtc 120

atctaggcat atatctgcta cagtattaac actattggga agaccaaagg aagctcttca 180

tgtaaattag atttgctagt gaactctatt aatt 214

<210> 69
<211> 250
<212> DNA
<213> Nicotiana tabacum

<400> 69

gatccagtac agtttcaaag aagaagtccc aacaaaaggt ggaatcagtg gatagttttc 60

tacaatcatt gggtcttgag aagtatgcaa ttacatttca agctgaagaa gttgatatgg 120

ctgctcttgt gcacatgaca gatgaagatc ttaaagctat gggaatacca atggtcgagg 180

aagaagataa tattagcact ggagactaga gtctgatatt tggtgcatct ccgtgctggc 240

tgctcctatc 250

<210>	70
<211>	218
<212>	DNA
<213>	Nicotiana tabacum

<400>	70

tacctaagcg gaggagaaga aattaactaa gattgcccta gtaacggcga gtgaagcggc	60

aaaagcccaa aatgtaaatc ttgtgcacat cgtgtacacg aattgtaatc tagagagagt	120

ttactcaacg ggcggggtcg cagaagtgac cacgaacggt ccgccagaga gggtgaaagc	180

ccgtatgtgg tggcctgcta acgagtaggt ttcgaaga	218

<210>	71
<211>	138
<212>	DNA
<213>	Nicotiana tabacum

<400>	71

cgagaaagtt gcaattattt catgaacgtg aaatagcagt gcagttgcat ttctgcattg	60

ctgtaccctg taggttacaa aataaagctc aaaacatcta gtagtagagg tatgagggaa	120

cagccaaccg gtgttctt	138

<210>	72
<211>	244
<212>	DNA
<213>	Nicotiana tabacum

<400>	72

ttgatcaagt ccctactaga cttgttcatt gaaggtcctg aagatagcca tggacaagta	60

gctaccaacc agacttcagt aattggctcc aaaatatgaa gcagcatttg gtgtttttca	120

ggtgtcattg ctacatgaat gaaccatgaa cattcatttg aagatttgct gaaacttcta	180

agaccgacct catttctgtc taaacatatg aactatttgg gctcaagaat acttcgattt	240

ggac	244

<210>	73
<211>	228
<212>	DNA
<213>	Nicotiana tabacum

<400>	73

gatcacgtac accagcggaa acgaatcagt ggaagagttt gccaatgcat tgaggcaatt	60

gtctagatta ggcaaataag atggtactca atgctattcc ttacaagaag tcacatacta 120

tacatcagtc ttattttttt cttttggcaa gtttaaggaa tgcaataatt tgcatgtcaa 180

agcattctga cttcaggtta tgcagaatat agctcattca catccttt 228

<210> 74
<211> 185
<212> DNA
<213> Nicotiana tabacum

<400> 74

ttgatcacgt accaaaggaa gaagtggaga gcaggataga gaatatgcaa gaggtcactg 60

ccaagaactt ggagaaacta gaggaagaga aggaaacagt agtttctcag atggctgaac 120

tgaagaagat attatatgga aaattcaagg attccaattc aatcttgagg aggactagta 180

aattt 185

<210> 75
<211> 242
<212> DNA
<213> Nicotiana tabacum

<400> 75

ttctttctac cctgaaagga tttccttctt ctccagctaa ccgggattct tggaagcaac 60

taaattatcc ggaaatggtc tcgtctaatt tcaggttttt cttaagttgg tgcagaaaca 120

gcgaacttga gggattaata acccaaaaaa taactttact tggaggatgg gtgacgatgc 180

ttttgatggc cagctttggc tgaaaagttg tctaagctta acgttcacag caaagcatag 240

aa 242

<210> 76
<211> 369
<212> DNA
<213> Nicotiana tabacum

<400> 76

tcgatacagg cagagaggct gttttcaatt agtgaattct gatccaagca tataatacta 60

ctgcggtcat gggcaaggat aatagcttgt tctattttct tgagaaatgg catctggagg 120

aagaggttat tattatgaaa tgatctgttg ttgtaggttt ggtccccctg gaggtggcct 180

ggaccgagga gcgttgtgtc cctcgataca ggcagagagg ctgttttcaa ttagtgaatt 240

ctgatccaag catataatac tactgcggtc atgggcaagg ataatagctt gttctatttt 300

cttgagaaat ggcatctgga ggaagaggtt attattatga aatgatctgt tgttgtaggt 360

ttggtcccc 369

<210> 77
<211> 224
<212> DNA
<213> Nicotiana tabacum

<400> 77

tcgatacagg tgggcccata tccaacaaaa cctttcttgc acttatgaag gggcgctcat 60

cttggtaaat gttttgctga taaacttcat ctccaagtaa tactaaactc tcttggtgac 120

agaattgaat tatttgctta agatttgcca cactgagaca ttgtccagtg ggatttccag 180

ggtttataat caccatagcc cgtacagtta ttcccctgta tcga 224

<210> 78
<211> 196
<212> DNA
<213> Nicotiana tabacum

<400> 78

ggctcccagt aagacaaagg catcctcttg agtggaggaa tgaatctctg atttttctaa 60

gtataatttc aatgtcccta tagccttagt atcaggagaa ttttgtggtg tttgaagtat 120

tttccatagg aatgtgtttt tttgtaattt ttctatttcg gattttgaag gttgtcattt 180

tgaggcatgt tttccc 196

<210> 79
<211> 185
<212> DNA
<213> Nicotiana tabacum

<400> 79

tggggtatct tagggtatgg aaaaagatga aattaggaaa gtgctgcctt taaagctcaa 60

atgactatgt acttttttaa tttgatgctg cagcttaatg tttattcgga taggccttag 120

ttctttcttg gccagtatat ggaaatcatg tgtttttgct aattaatttt gtagctttgt 180

tggcc 185

<210> 80
<211> 284
<212> DNA
<213> Nicotiana tabacum

<400>       80

aaccaagaag caataattca aataacaatg atcaaatctt catgaaatga taacatgttg       60

tggataacct tgtgacgccc acagaaatca atacattatc caacatctaa ctaataaacg      120

taacatatct catgtgcaca caggccaaat attcctaaga ttcaattaca ggaaacaata      180

atgctcaatt atcaaacagt gttgctccat tcctgtcagt gccctgtttt cctttctcag      240

ggaagcaaaa ataaaagcag ctcgatcaat tgtagaccat gatc                       284


<210>       81
<211>       290
<212>       DNA
<213>       Nicotiana tabacum

<400>       81

acgatgagaa atgtgctctc aatatattcc agacaatata tgtgtgcttt taagctgtat       60

ttacagagaa gccattatgc caacagagca acaaatcaca aacgggacat gaatcttgat      120

accaataacg ttaaacttca ctaataccaa catcaaatca ctatttacaa acaggacatg      180

aatattgata ctgataagaa tagcatcact aataccagca tcaaagtgtg tgagactgca      240

cattccatgg cgtttatgca ctaaaccctg tggagggact cagtatggac                 290


<210>       82
<211>       352
<212>       DNA
<213>       Nicotiana tabacum

<400>       82

gatcaatcgc agaaggaaaa cctgatccgt gcccattaca cactgcatgg cttaaagaac       60

taaaaatagt ctgaggtata aagtgtactt ggtatatcat agttgttaga ggccctctgt      120

tttcttttgg aggatgtaga tgagagctgc tgtatcattt tattgtaatt tggaagtcgt      180

actcttattt catcttggat tcatcttatt tttctgcctt tattattggt ggaaaagctt      240

cttgttatgt gacttgagtc acgtaggata accagcgctt atgtaaattg tttgacgtgc      300

tagatgaatc taggggtcgt ctgtgatgag atattaaaaa tatattctgt gt              352


<210>       83
<211>       479
<212>       DNA
<213>       Nicotiana tabacum

<400>       83

gatcaatcgc ccaaagggat gaagactatg agcactggcc ttttcttgac aaccctttcg 60

cttggtttct tcattagcag cttcttagta tcggttatca agaaggtgac cggaggcaat 120

ggtactgatg aaggctggct tgcagacaac ataaacaatg gaaggctaga cttgttctac 180

tggcttcttg ctgtgttggg agttataaac attgtgattt acctaatttg cgcaacatgg 240

tacaagccaa ggaagcctaa gtcagccata cagatggaga atccgcacac taaaaatgca 300

gctgaagaga agtgctagga tttatgctgt tcaagttgcc cttttcttaa ttttattagc 360

caatatacag tgaaagaact gttgtttcag tttagtttct gttctctata tatcgtgttt 420

ggatcagtaa attggttttc ggttttgcag tatcctaata atcttatttc tatcttggt 479

<210> 84
<211> 141
<212> DNA
<213> Nicotiana tabacum

<400> 84

tgatctcaga ctgaagttgg tgttgctgcc attaatggtg tattttctgc ttgaagaaaa 60

gtaaagcagc tttgattttc gttgtttttg aatttatgta aaaggttgta aatgctttct 120

tgaagtatta ttttgttggc t 141

<210> 85
<211> 220
<212> DNA
<213> Nicotiana tabacum

<400> 85

agagagatga aagaattcac tttattataa aggcataatt ctttttttat tcttctcact 60

tcttgtgcag cattcttgaa ggaagtgatg tcaattacta gcagtgaaaa gcttgatttg 120

tggagagttg gttttataag atttagccta gaagttgagg aaaattaatt cgaattagga 180

gcgatctacg tacttatact tgaaattact atctttatga 220

<210> 86
<211> 243
<212> DNA
<213> Nicotiana tabacum

<400> 86

agggaaaagg gacaagagca tatgagttca atatattggc tttgaactca gctagttagc 60

ttccctcatg tcaaacatag tgaaaactat agtatatatc agctctcatt aaaatcggac 120

atcaaaaaaa tgaaagaatt tctagctata tattgcagaa acttcacaag acatttactt 180

caatctactc tgatttcaaa ctcaggttct ggtgctgcct cattgtcttc gtcgtctgag 240

atc 243

<210> 87
<211> 331
<212> DNA
<213> Nicotiana tabacum

<400> 87

aggacatgga gaaatgaaaa ggtctgaaat ttgcgaaaat tagatgaaaa tgcgaataat 60

ttcttctagt tgttttttaa ttaatttagt aggacctatt taagatgtat ttatgtaaaa 120

aaaactggta tggaagggtt attttgtatt tatatgggag gcaaagggag ggaaggagat 180

tgaagaaagg gcagtgaaga attaagtagt atggttaaat actatattgt tggtgtatga 240

ttgtgactta tgactaatga tcatgatgaa ttagcttgta agcttttatt ttgaatgtta 300

atgattagta atgagaagta tgttttaggc t 331

<210> 88
<211> 342
<212> DNA
<213> Nicotiana tabacum

<400> 88

acggcatcac aaatttctca attgttcctt gacaatatct gtaaattaca tggcaatcca 60

gtttctatta cttctgatag agacccgatt tttacaagcc tattttggaa agagttgtta 120

atggattggg ggtacaaatt aagctcagct cagcatacca tctgcagacg aatgggcaaa 180

cggaaaggct taacaggtgt ttggaaggtt accttcgatg catgactggt cacaagcctg 240

cagcttggag caaatggtta agcttggcag agttctggta caatacaact tttcattcag 300

cgattcaaat gtctcccttc aagacgttat atggctatga tc 342

<210> 89
<211> 345
<212> DNA
<213> Nicotiana tabacum

<400> 89

acgtaaggat gaagtaagat taaaccacac gaggaagaca atttaagcct taatgaggta 60

ttttccctcg atatgggctc ataacaacaa gcactgacga acgaaagcag aaaaactaat 120

aacagaaaaa ataatgatat attgccttgg aatgcatggt accctccccc ccttatgaat 180

taccagaccc tctttatata gtagaggaat cctactttcg gtataattct ataaaaggta 240

atttttttta ttcactgact gccagttcat tttcggtacg tgccgagatt ctcgccgtaa 300

tatccggccg gtcacggata tttcggtctt tcgttggcta tgatc 345

<210> 90
<211> 345
<212> DNA
<213> Nicotiana tabacum

<400> 90

acgacaagat attgtatgga cttgtgctcc accaacgtca acaacgatat tggcaatgta 60

aggtgaatta ctacatttga gggtatgaac accataggga atagccacac cagagactaa 120

taatcaacta tcgatcttga tgttagacaa atatcagagt cttcacgcat gatttaggat 180

tagctttaaa aaggtgcaac cacctgaatt ggtttctccc tttcccccta tttcaatctc 240

agctatcttt tttcttcaag ggctgttgga tgggttctta tctggtattt cgcgaggtca 300

ttttttttcc ggtgagagaa ctggagggtg gatggggcta tgatc 345

<210> 91
<211> 228
<212> DNA
<213> Nicotiana tabacum

<400> 91

ctgcttgatg atgaccaaat ggctgatgga tttgttctaa cctgtgttgc ttacccccag 60

tctgatgtta ctattgagac tcacaaggag gaggagctca ctgcctaaac ccctcaatct 120

cattatttta aaaaagtttg atgagcattt attttcatat gtaactcttt tgttcgcata 180

aaagatgcta tgttgcaact tctagtcaat gactttggtt tgcttagg 228

<210> 92
<211> 250
<212> DNA
<213> Nicotiana tabacum

<400> 92

atcaatcgct ggaacattca agagatatga tgcttgagaa tgtgaaagga aaagaacaga 60

gaatagcaaa tttgatgggg tggagagtta gtttttactg tgttattttg tttagttgag 120

tgttcatgca aatgtttgta catcttcaag tgaattatgg tccggggaat aattatgggt 180

atatcttgta tgctagtgta tacgttcaag aattgatctc gttaattttc attgttatgt 240

cattcatgga 250

<210> 93
<211> 354
<212> DNA
<213> Nicotiana tabacum

<400> 93

gatcaagtcc agcagctatg gctttagtgg atgaaagaat gagcacggaa ggaacaggct 60

tccatttggt ttgagcaata atcttcttgg ttggattctt tttggtgtct ttggtttgat 120

ttggtctctt tacactgttt acacttctag cctcgacgag gatgaagatt ctggattgtc 180

tctttaattt gagcttgtaa ttaagcagtt cccgcaatcc atcaatcctt aattcttaat 240

tgagtgaatt tatgtaaaat tactatggtc accaattaat taattgtaca ttggtgctgt 300

atcaattaat ttgttcaatt gctggtagta tatatatatt ccttttttgc tcaa 354

<210> 94
<211> 459
<212> DNA
<213> Nicotiana tabacum

<400> 94

gatctcagac ccacaattca tcaaagctca ccacattatc catgcccaca aaaaagaaga 60

aaaactcatt tatatctctg tttctcatgc ttttcacact atcactttca ggaattaatc 120

tactctaggc acaatacaag ttaagagaga tgtatacaaa gccttatcaa aatcgagttg 180

tacacaaagg cctgcatctc gatatgttta atcccaatag tggtttgtat aatgtgatga 240

agtatacaaa ggtagtagtt gagacatgca tttgtgtttg gtatattttg aagcctgcaa 300

aggcagtagt agcaacgtgt ggagaaaggg tcccttttga tcagaccctc atacttgaga 360

tttgatcgtt catatctcag ttgagtttta atagatgatc tgagtagcga taaacgcgca 420

tttaacaggt tgttttacat caagtcttct cctcttgtc 459

<210> 95
<211> 282
<212> DNA
<213> Nicotiana tabacum

<400> 95

gatcgcattg gatgaggatt atgattttaa ggggaccatt gtcgctattg gcaataccaa 60

agttgcaaga gttatacccc aaaagactcc atttattact gtatatttga aacctcctgt 120

gattgcacaa acataccaac aaaggtctac ttttgctgtt agaaattagt agagtcgaga 180

ctagaaaata gtgccattga cttaccagtc aaaaggatag ggtaaaatga tagactccgt 240

agttgctcgc aggatgacca gatcagggac gtgttacacc gc 282

<210> 96
<211> 195
<212> DNA
<213> Nicotiana tabacum

<400> 96

gttttcgcag caacccaacc aattgagcaa agttacccac aaccagagaa gaagaaaatt 60

gccttcacaa gttcaaaatt tttcttgtct aattgtttga ttctgtgtat gaggaatgta 120

cgaaccattc tctttttgtt cgtttggtac agtttaatct ttaatgttag cattggatgt 180

gtatttattt ggtga 195

<210> 97
<211> 235
<212> DNA
<213> Nicotiana tabacum

<400> 97

tggtaaaggg caaaggatcc tggaacagtt tttgttgctg gtgctactgg acaacctggt 60

gtccgcattg cacagacatt gcttcgtgaa ggttttagtg tcagagctgg agtttctgac 120

cttggtgcag cccaagagct agctcagcta gccgttagct acaaggtaat ctccaacgat 180

gagtcgaaac gccgaatgct gttgcatcta catttcaaga tgcagaatca atcgc 235

<210> 98
<211> 217
<212> DNA
<213> Nicotiana tabacum

<400> 98

gcccaaaaaa ctagtaaggc ttatgattag taatacgaaa tatttatcac tgagcatagg 60

catgagacta aaacctacag gatcagcgat cagacgttac aactgcaaaa aagagtagct 120

attcacacac ctcagtcttt attagcatca acaaagagga cgagtttccc tactttccat 180

tttttttcct tctttgttcc ccctttgccc tttacca 217

<210> 99

<211>       472
<212>       DNA
<213>       Nicotiana tabacum

<400>       99

gccagaagaa acagatcaaa taagagaaat aagcgaaaca gttgtaaagg atacagtttg       60

taccttcacg ttcaaatgcc agtataaatg cgaaagaggt aaaattaagg aagccttaaa      120

ccaaaaatat gctttttcat acatacatga ttagctatta gcaaaataga attactctgt      180

ggatcaatga caaactgcag tcacttctag gccttcactt tccaagtcat cctacgcctg      240

ttgtgccagc tgttcctatc accatccatt tggttcttct ttgctgcaaa tgagcactga      300

tcaccatgtc tacctttacc attcatttcc agttgatgct tacgccttct cactctgttc      360

tctaacctta aacctcttag cacttggacc cgctgctcga tctccaaagc ttgagagaag      420

ttccagatcc tgacaacacc ttcttcacca gcacacacaa tacggttaga tc              472


<210>       100
<211>       204
<212>       DNA
<213>       Nicotiana tabacum

<400>       100

ggaacaatca agccgcatat ctaagtgtaa aatataaatg agagaattac aggataagca       60

acagaattta ttgtacaact gaaccatgta taagtacttg catgcaatct actggaaact      120

agtgtaaaat tccatggatt atgagctgta cttgtaatgc tacactagta accatatcag      180

tatatgcata atccttcatg tggc                                             204


<210>       101
<211>       107
<212>       DNA
<213>       Nicotiana tabacum

<400>       101

gccacagtaa acaactatac cagtattatt ccacattgtg aggtctggag aggaagataa       60

acaaatacac ttagcacgaa atgtacaata agtagttgat tggttcc                    107


<210>       102
<211>       605
<212>       DNA
<213>       Nicotiana tabacum

<400>       102

ggaaccaatc aaacatccaa aatggtagta aaggcttcac taaaagatgt aggtgcagtt 60

gttgttgcta cagctgcaag tgcaatacta gctagcaatg ccttagctat tgaagtattg 120

cttggtggcg atgatggcag tcttgctttt gttcctggaa acttcagcgt tagcgccggt 180

gagaaaatta cgttcaagaa caatgcaggg ttcccacaca acgttgtatt tgatgaagat 240

gaaattccag ctggtgttga tgcatctaag atttcaatgt ctgaagagga ccttctcaat 300

gcaccagggg agacttactc tgtcactttg agtgagaaag gaacctacag tttttactgt 360

tcacctcacc agggagctgg aatggttggc aaagttactg ttaactaatt ctagttagcc 420

tctttatcat gtgtgagagt taacttgttt ctattccaat cccctgaatc ttttgttgta 480

tgtctttttg aggatataat cataagttcg tggagttaca tagttcttta tatagcttgg 540

gatttaagag gtttgtgtgt ttcacaattg agacggaaaa caataaaatt gtactactac 600

taggc 605

<210> 103
<211> 240
<212> DNA
<213> Nicotiana tabacum

<400> 103

ggaaccaatc ataacaaaat gcaaagcgag cgagaattgg acaaaagttt catttaagcc 60

agacctggca aagtttaaca tggaacacct tgaagaagat gtagttgcgt taatgagaaa 120

gagagtgatt gatttgggtg gatgccttgg aaagactgtg aaggtcaaac tcaatgaaca 180

acgcattcct gtcaaatcat ttgaagagta ttgcaagctt ttcttagatt ctactgatgc 240

<210> 104
<211> 348
<212> DNA
<213> Nicotiana tabacum

<400> 104

gcagcaagcg attcaaaccg ttaattaaat tccaccaaat ttaaaacata caagcaactg 60

ataagtggta aggaagatag atctatatca gaagcagtag gaaatacatt ggagaaaggc 120

taataaggaa ggatcattat ttagggatcg aggattagat ctaatcgcta tcgctgtcgc 180

tgctgctgtc atgcccatcg tgcttcttct cctttttctc ctttttctcc tttttcttct 240

tcttctcctc tccctccttg tgatggtgtt ctccgccttc ttctccatgg atcttttcct 300

ttatcttctc aacaatccct tccttgtgct ctccgctgct atgaccga 348

<210> 105
<211> 337
<212> DNA
<213> Nicotiana tabacum

<400> 105

gtggaagaat aatgccatgt ctatatattt agacagctaa attaaaaatt gtatacagat 60

gctcagggga ggaggggtgt gattactcat aattgttgca aaagactgag gaacatgtat 120

atcaaatttg cagttaaaaa aatgaaggga acagattccc actgaattat tcctgttata 180

ggaacataaa acaacaacaa tcataacctt gtactcaaat ccaaagaaga aacttcaaga 240

ggaatgggct gttgctctca acagtgtgga agacatctat gagagattta tgaactactg 300

tcttgggaag ctgagaagtt gtccttggtc tgagatc 337

<210> 106
<211> 451
<212> DNA
<213> Nicotiana tabacum

<400> 106

tacaacgagg agacatatac aaagaaagga ttggaatggg tgaatacaac tgaaagcttg 60

aaagatgtat tagatcgaca ttaccctgaa ataactgaga aatggatgaa ctcaagtagc 120

gcattctctg tttgggactc tactccacaa cctcacaatc ctattccact ctactttcgc 180

gttcctccgc agtaaatcca tcatttgact tattaaggca accgcatatt gggattctac 240

aatattactg catgttttat caaatcttaa tacgaaaata atgtgcttga tttaatttta 300

cctgcaagtt tgtaataaac tgagatgatt atagccggtc aaatttatta tttacttttt 360

ctaaccggta tatatagact atatattgat tatacacagt tatacatata ctatacgtga 420

attatatata ttcatcggat atttttagct c 451

<210> 107
<211> 142
<212> DNA
<213> Nicotiana tabacum

<400> 107

aacaaaattt aaggtatcta caggactcta gtggagcaat tttaactcca tagcatcttc 60

cctacaaagg gatgacaaat acttacttga tagataccat accttgaatt tttttttcta 120

gcattcctat ggcgattgat ca 142

<210> 108
<211> 243
<212> DNA
<213> Nicotiana tabacum

<400> 108

gatcaatcgc gcggggttag gaactgttgt ttccgaaaag tcaacatgaa gttcttcata 60

agattcttta gaaggtatct gatttatcaa attttttgtt gtatatattt gagtcttggc 120

aagtattatc atagcaattt tatactgcga atcgccttct cttatcgcta gcttcatgta 180

ctatttcttc tcatacaact gttggttctg aagcaagatg aaattcaata tccattatac 240

tac 243

<210> 109
<211> 289
<212> DNA
<213> Nicotiana tabacum

<400> 109

gatcaatcgc aacctcatcc tcggcctcaa ccatagtatc cccaagaatc atcattacaa 60

tctcagcata cctcgcaaca gcttccacaa tctcctttac aacctgaact gaaccaacaa 120

cctagaactc agctagaact tcattctcag cctcaacaat taccgccacg tgctcagact 180

caattcccac agtactctgc ttatccgcct ccaccttggg catcaactcc cggatatttt 240

agcaatccga atcattcaac tagaccttcc tacatgtact caactccac 289

<210> 110
<211> 175
<212> DNA
<213> Nicotiana tabacum

<400> 110

gttttcgcag aggacaagga gggccagttc agaaccgaat gttttcattt gctttggctg 60

ttgtatcaat gtatcactct ttaagttcag atattatgat aatgagttct gtgggcagaa 120

cttgaaaatt ataatactct gtggaccctg tagaaataga acatactcat tccac 175

<210> 111
<211> 254
<212> DNA
<213> Nicotiana tabacum

<400> 111

gatcgcattg gatatgtggg cgaagatgga gaagttagga gtaaacccag attctattac 60

ctgtgtcctt gtgatatcag cttatagaca tacttccaga aatttagttg attgttgcca 120

aaagttcttt tcctcaatgc aaacgttata taacatagaa cccacctcgg agcattatgc 180

aggctttata gctgtgttag gttattgggg tctgcttgaa gaagcagagc agattattag 240

tgcaatgcga tcaa 254

<210> 112
<211> 234
<212> DNA
<213> Nicotiana tabacum

<400> 112

gtggacggtg gatttattct gattgtctgt ttagatatat agtttaaaac tcgtaaaaat 60

cgaatttaac ttaactgtag ttatttttac tttctttctt tttcccaaca cttttgtagg 120

tcacggattt gaattgcgaa aacagtctct cgcagaaatt agacccttgt gatccggcca 180

ttccctgcat cctccgcata acgggagttt attatactgg gttgcccttt acca 234

<210> 113
<211> 145
<212> DNA
<213> Nicotiana tabacum

<400> 113

tacaacgagg catgataaac aacattgcca gaatataact ttgcttttat acgtagttgt 60

ttctccttat gttgattggt tttgtcactt attgttaaga cacttctaaa tatgtgaccc 120

atatatagtt aatgttgtat tccac 145

<210> 114
<211> 324
<212> DNA
<213> Nicotiana tabacum

<400> 114

gatcaagtcc catagtggat tgcattgtct gcatttaaac agcagaaaaa ataggaggcc 60

gttctttaaa taagcatgga aacaagtgaa caaatccata ctgagaagga agcttctgct 120

gaaggacttc catcactcat cagattcata agttctgaaa tggatcacga ctttgatagt 180

atagtgaatg accatattaa aaatcagact gcgcctgcac ctgaatctca ctttccaagc 240

tcatcaatta tggtatctga atttgctgct gagcgagaga gtgacagaat tcgtgcagtt 300

tccattagca tgcccccctc tccg 324

<210> 115
<211> 378
<212> DNA
<213> Nicotiana tabacum

<400> 115

ccgatgagaa atgtgctctc aatatattcc agacaatata tgtgtgcttt taagctgtat 60

ttacagagaa gccattatgc caacagagca acaaatcaca aactggacat gaatcttgat 120

accaataacg ttaaacttca ctaataccaa catcaaatca ctatttacaa acaggacgtg 180

aatattgata ctgataagaa tagcatcact aataccagca tcaaagtgtg tgagactgca 240

cattccatgg cgtttatgca ctaaaacccc tgtggaggga actccaagta ttggacggga 300

cgctgggaca agcttgtgct gatgcctcca ccatctgctc catcttaatt ccatcattac 360

agatattggc gattgatc 378

<210> 116
<211> 225
<212> DNA
<213> Nicotiana tabacum

<400> 116

gatccagtac catcatcacc accaccacca ccactacctc gctagtgctt tgtcctttca 60

ctcctgttaa gtagcactca actagtaggg cttccttgta attaaagatc tagtttagtt 120

ttttcttgag aaatttgaac ttaccgtatg tgagatatcg acaaattttg actttgatta 180

ttgtgatact tggttataag tacatttaat cttcttttgc tcatg 225

<210> 117
<211> 194
<212> DNA
<213> Nicotiana tabacum

<400> 117

gatccagtac tatgctatgt gtctatgtga tatgttatct tcttacccag tggataataa 60

tctaatgatg taataatggg tggttttatt gttatctacc ttgtttacgc acattttttt 120

cttcgtatta tgggggtgtg tatgtatatg ttttgatgaa taaataaaaa gtatacggtg 180

atgagtaaac tgtg 194

<210> 118

<211>       450
<212>       DNA
<213>       Nicotiana tabacum

<400>       118

gagcaaaagt ttaatttaag cattacgatt gtcgatgtaa caaatagcaa ttccccaaac       60

atgtctctat atatagagta cccagcgttg ataaaacaaa actaacactc aacttaaaca      120

aaatacagta tttagaaaat tgttaagatg atgaaactga atctagccgt ctacataatt      180

ggaacaggaa cctcaatgtc aacaactggc accactggtg gaggaatggt gtattcttct      240

tcatccttgg gagtatggat tgtgacaaga tctggaaggg gtgttgttgg accttgcttt      300

cctttgggat cccaatcaag cataatcttg accttgatgc ccagtactcc ctgtctcatg      360

agaatgtgcc taacagcaga gtcaatatac tctttcactg gctgcccaga agaaatcatg      420

taaccatctt tgaacttcat ggacttgatc                                       450

<210>       119
<211>       300
<212>       DNA
<213>       Nicotiana tabacum

<400>       119

gatcatagcc acaaagatga aagacaaagg ttacaatcgt agccctgaac agtgtaaatg       60

caagtggaaa aatctcgtta cgcgctataa gggatgtgaa acgcttgaac cggaagctct      120

acgacaacag tttccatttt acaacgagtt gcaagcgatt ttcgcggcta ggatgcagag      180

aatgctgtgg atagaggcag aaggtggcag cgggtcgaag aagaagacta tttctcagct      240

atcttctgat gaagaggaag aaaatgagga tagcgaagga gaaaaagtag ttggtatcac      300

<210>       120
<211>       361
<212>       DNA
<213>       Nicotiana tabacum

<400>       120

gatcatggtc aaaagcaatt aatttaatca ataagatcat caactttgca attcagaggt       60

tgattcctag cgacttggac cactttgctt ctacttctct ggtggccttc ttacacattg      120

ttttctttgt gtcttcacgt ggaagtggct tcaacagctt agttttttat tagaatatta      180

attatttagt tgatctttga agtgtatttg agttttcatg ttttttgatg ttgcttcttt      240

ataatttatt attgtaagta tatatctttg tttggtttgt ttcgtgttaa ttattgttgg      300

ctgtctacta gtgcctactt gtgctttgtg caccaaatca ataaagagat gaattttgta 360

c 361

<210> 121
<211> 616
<212> DNA
<213> Nicotiana tabacum

<400> 121

gatcgcattg acgaggagga tggtaacagg ctcactctgc ggcatggcac gattcccgtg 60

tttgtgggta agcctgcggt cagggaggtc attcagcgtg gctgctgcac gtcaccgccg 120

cgtgtcattc aactcagcta caaagcactg cttgatacgt cagtacagac ggtgtgattg 180

tgataccaca agtggtggcg agaaacccag gctggtggcc ggttctttcg taggggaagg 240

gtggctacgg agctgtttaa ctgaacaatt ttggacggta ggaaaactgc ggatcgcttg 300

caggcctgcc ggatgattct tgtcggcagg cctgcaaggg gagatcaatg ggcggcggca 360

gtcttgccct tttccgagca ggcacctggc gtcatataca gcagataatt gcccatccca 420

tgttgggctt caatgttttg tagagaatgg tggttagacc atcaccatct catagtcctc 480

gtctttggaa atagggaatc cctggccgtc tttatagact tgatggggga ggaattcccg 540

aagcgttcca tcgctctcag catccggaat agtccggagg aggtctgctt cttggtctta 600

ttctccaatg cgatca 616

<210> 122
<211> 576
<212> DNA
<213> Nicotiana tabacum

<400> 122

ggcatggcac gattcccgtg tttgtgggta agcctgcggt cagggaggtc attcagcgtg 60

gctgctgcac gtcaccgccg cgtgtcattc aactcagcta caaagcactg cttgatacgt 120

cagtacagac ggtgtgattg tgataccaca agtggtggcg agaaacccag gctggtggcc 180

ggttctttcg taggggaagg gtggctacgg agctgtttaa ctgaacaatt ttggacggta 240

ggaaaactgc ggatcgcttg caggcctgcc ggatgattct tgtcggcagg cctgcaaggg 300

gagatcaatg ggcggcggca gtcttgccct tttccgagca ggcacctggc gtcatataca 360

gcagataatt gcccatccca tgttgggctt caatgttttg tagagaatgg tggtagacca 420

tcaccatctc atagtcctcg tctttggaaa tagggaatcc ctggccgtct ttatagactt 480

gatgggggag gaattcccga agcgttccat cgctctcagc atccggaata atccggagga 540

gggtctgctt cttggtctta ttctccaatg cgatca 576

<210> 123
<211> 208
<212> DNA
<213> Nicotiana tabacum

<400> 123

gatcgcattg gtacattgat ttgcagacct catttccagt agaaagttgt agaaaaaaga 60

taatccataa tcaagtatta caaagatgct gatctatgat ttgtatatat tgcttattta 120

ttgttgttgc tgcagctaca gcaaaagatt atagaaggat tattgttctg ctttgtctaa 180

ttatataaat aatccttgtt tcttggtt 208

<210> 124
<211> 262
<212> DNA
<213> Nicotiana tabacum

<400> 124

gatcgcattg caattcaaga tgaaaaagaa gatggaagag agtgggcagt aacttttcat 60

tcgaaagcaa cacttctcca aatcaggcta caagaagttc aaagaagggt gatctctaac 120

ctatttgtgt gtcatatctt tgtaccctag caaacggcta caataagatg tcagcagttt 180

caaggaagta atctttgtcc ctttaatgtg tgtcatatat ttgtacctac gcaatagatg 240

actcagtttc agtttcttgg tt 262

<210> 125
<211> 617
<212> DNA
<213> Nicotiana tabacum

<400> 125

gatcgcattg acgagggagg atggtaacag gctcactctg cggcatggca cgattcccgt 60

gtttgtgggt aagcctgcgg tcagggaggt cattcagcgt ggctgctgca cgtcaccgcc 120

gcgtgtcatt caactcagct acaaagcact gcttgatacg tcagtacaga cggtgtgatt 180

gtgataccac aagtggtggc gagaaaccca ggctggtggc cggttctttc gtaggggaag 240

ggtggctacg gagctgttta actgaacaat tttggacggt aggaaaactg cggatcgctt 300

gcaggcctgc cggatgattc ttgtcggcag gcctgcaagg ggagatcaat gggcggcggc 360

agtcttgccc ttttccgagc aggcacctgg cgtcatatac agcagataat tgcccatccc 420

atgttgggct tcaatgtttt gtagagaatg gtggtagacc atcaccatct catagtcctc 480

gtctttggaa atagggaatc cctggccgtc tttatagact tgatggggga ggaattcccg 540

aagcgttcca tcgctctcag catccggaat aatccggagg agggtctgct tcttggtctt 600

attctccaat gcgatca 617

<210> 126
<211> 405
<212> DNA
<213> Nicotiana tabacum

<400> 126

gatcgcattg ctgaagggaa tagagctact gggggtttga attttgttgc taaagtttat 60

ggcattgctg gttgcattaa atttttggag tcatattatc ttgtattggt gacgagaagg 120

cgccaaattg gatgtgtatg tggtcatgcg atatacagta tagacgagac ccaaattata 180

acgatacctc atgtctctgt ccaaactgat gttgctcatt cgaaaactga gttaaggtac 240

aagaagcttt tgtccagtgt tgacttggca aaagattttt tctacggtta tacctatcca 300

ataatgcgaa gcttgcagaa gaatatcttg tctacgggtg aggatgggat gccgtatgac 360

aatatttttg tctggaatag ttatctaaca catacaatgc gatca 405

<210> 127
<211> 430
<212> DNA
<213> Nicotiana tabacum

<400> 127

gatcgcattg gctattgggg caaaatttgt ccccatgagg aaacccaaga agttgccggg 60

ggaggttatt tcagaagagt attctttgga gtatggaaca gacaagattg agatgcatgt 120

aggtgccgtg caagccggtg aacgtgcact tgtggtagat gatcttatcg caactggagg 180

gaccctagct gctgcaatta ggcttctaga gcgtgttgga gttgaggtgg tcgagtgtgc 240

atgtcttatt gagttgccag aattgaaggg ccgggacagg ttaggtgata agccactatt 300

tgttcttgtg agctcaacct gatcctcaat taggaaatgg atctagacag agagcttcat 360

tgaggtggaa ctgtattgga gtgacatttt gaatttggag aggagaaatc tcattattac 420

aatgcgatca 430

<210> 128

<211>       453
<212>       DNA
<213>       Nicotiana tabacum

<400>       128

gatcgcattg gtcataggat gttgagattt tgtctccata gtcagcatct ttagagatat       60

ttttcgtaga taatctggct gacttatagc agcagtatag atcttttcct caaacctcaa      120

tgcaattttc ttgagctcct gaactccttc ttctccagtg acaggaagat gcctctttaa      180

ggtctcggtt atcttgttga caatcctttg acgagaatca ggtagaagtt gagtccgcca      240

atcaacggcg tccatatctc cggctgcagc tccagctggg gcggtacctg ggtcgtgagc      300

attctgacca ctacttgcag cattggccga attggtcata ggattttgag agtctgtctc      360

catagtcagc atttttagag atatttttcg tagataatct ggctgactta tagcagcagt      420

atagattttt tcctcaaacc gtcaatgcga tca                                   453

<210>       129
<211>       462
<212>       DNA
<213>       Nicotiana tabacum

<400>       129

gatcgcattg aagtggagaa ctttccttct gattagatgt ggatttggaa tttgcaggca       60

cagctacatc aaaagaaaat ccttcatctt cgaatttatc ccaatcttca tcccaaacgg      120

ctgatatctc cggaatacca ggttgccagc caggggggag ttcaattaat gcagttgact      180

taatattaag ctcatgtttc ttgcaacgat cagccagggc cttcagtagc tcctcaagat      240

catgctgtac gcgatcagcg cgaacctgaa gaataccatc agcgctgcca ccttgttcca      300

ttttactgat tgcctgttgt aactccagct tcctctcctg agtatcacga aatgacgcct      360

cttcaattgt cagtttagaa gcaatttctg caacttgctt gtatttctct tcatattttt      420

ttgcaagcat ctcagcctcg cgtttatcag ccaatgcgat ca                         462

<210>       130
<211>       445
<212>       DNA
<213>       Nicotiana tabacum

<400>       130

gatcgcattg gaaaacgaac agcagcagca cgctaacatc acgattcata actccctcca       60

ccttagtctc ctactacctc acgtttttgc catgaaataa gcaatcaaaa cactacaaag      120

tctctgttaa aaatccaaac ttcgcccaaa acttagtctt gtaaacccct gaaacttagc 180

tgaaaaccaa ccccgtgaac cacccaaaac ccatccaaag gtgcagaaac aaagcagata 240

cgaacagctt cagaaacacc ctcaaatctg tccctaaaac cagctctaaa atcacccttt 300

aacaccatgg aacagcccca aggtcttcat aatccttctc caaggcagca agatcctcac 360

gtgcttcact gaattcacct tcctccatgc cctcaccaac ataccaatgg acaaaagcac 420

gcttggcata catcagatca aactt 445

<210> 131
<211> 616
<212> DNA
<213> Nicotiana tabacum

<400> 131

gatcgcattg acgaggagga tggtaacagg ctcactctgc ggcatggcac gattcccgtg 60

tttgtgggta agcctgcggt cagggaggtc attcagcgtg gctgctgcac gtcaccgccg 120

cgtgtcattc aactcagcta caaagcactg cttgatacgt cagtacagac ggtgtgattg 180

tgataccaca agtggtggcg agaaacccag gctggtggcc ggttctttcg taggggaagg 240

gtggctacgg agctgtttaa ctgaacaatt ttggacggta ggaaaactgc ggatcgcttg 300

caggcctgcc ggatgattct tgtcggcagg cctgcaaggg gagatcaatg ggcggcggca 360

gtcttgccct tttccgagca ggcacctggc gtcatataca gcagataatt gcccatccca 420

tgttgggctt caatgttttg tagagaatgg tggtagacca tcaccatctc atagtcctcg 480

tctttggaaa tagggaatcc ctggccgtct ttatagactt gatgggggag gaattcccga 540

agcgttccat cgctctcagc atccggaata atccggagga gggtctgctt cttggtctta 600

ttctccaatg cgatca 616

<210> 132
<211> 477
<212> DNA
<213> Nicotiana tabacum

<400> 132

gatcgcattg atgaggatag ccttctcagt gaagaagatc tgacgaaacc tcaattgcca 60

agtgttggtg attgtgaagt tggaaagaca aggaaagcat gcaaaaattg tacgtgcgga 120

agggctgaag ctgaaacaaa agtgcgactt ggaccaacgg cggaacagct gaacaatcca 180

cagtcagcct gtggcagttg tggtctaggt gatgctttcc gttgcagtac gtgtccttat 240

aagggtcttc ctccgttcaa actaggcgaa aaggtaacgc tttctgggaa ttttcttgat 300

gctgacatct aattgcagat gataacagtc gatatgagat tgcagaaact gttataatta 360

gttcaaaatt agtttgttca ttttggttga ggtgagataa gtgaatgtgt aataagtgag 420

cgaaatagca gtggactgtg aacataaagg tctctctctc accccttgct ctggtta 477

<210> 133
<211> 580
<212> DNA
<213> Nicotiana tabacum

<400> 133

gatcgcattg acgaggagga tggtaacagg ctcactctgc ggcatggcac gattcccgtg 60

tttgtgggta agcctgcggt cagggaggtc attcagcgtg gctgctgcac gtcaccgccg 120

cgtgtcattc aactcagcta caaagcactg cttgatacgt cagtacagac ggtgtgattg 180

tgataccaca agtggtggcg agaaacccag gctggtggcc ggttctttcg taggggaagg 240

gtggctacgg agctgtttaa ctgaacaatt ttggacggta ggaaaactgc ggatcgcttg 300

caggcctgcc ggatgattct tgtcggcagg cctgcaaggg gagatcaatg ggcggcggca 360

gtcttgccct tttccgagca ggcacctggc gtcatataca gcagataatt gcccatccca 420

tgttgggctt caatgttttg tagagaatgg tggtagacca tcaccatctc atagtcctcg 480

tctttggaaa tagggaatcc ctggccgtct ttatagactt gatgggggag gaattcccga 540

agcgttccat cgctctcagc atccggaata gtccggagga 580

<210> 134
<211> 436
<212> DNA
<213> Nicotiana tabacum

<400> 134

aggcaccaca ggaagattgt ccagcaccag tatgttccca aaaccagcat caaactccaa 60

ggggtcctcc tccattaagt catcatcatc actgggtatg ccacaatctt caccaggagg 120

aagatggatg gagtcaagat cgatttggga aagttccaca ccaatcctag cggctatgat 180

catttacata ttcggatatc atttcaaacc gtataatgac aatgcatcaa aaagaataga 240

aaccaattcc tggcaaaaaa tgaaatacac gaaatatcct tctatacatg gccagttttc 300

atatcagact tgccattaag tggtcctttc tttttcccat tatcttttct tcttagtaat 360

atcaaaacaa agatatcaaa atataaacac taattctaac caaagtttgg ttgaatgatc 420

tgggtaggct atgatc 436

<210> 135
<211> 547
<212> DNA
<213> Nicotiana tabacum

<400> 135

tgatcatggt cagaactcaa ctgtgggtct agtaattggg tatacttccc acaagtcaga 60

attggctttg cccaaataac caggctctct tgacccttcg gacaattgtt acttatcggc 120

tttcttcctg aaagaagctc aagaagtact actccaaatg catagacatc aatcttgtca 180

ttaacctttc catacatgaa atattcggga gccaagtaac cgaaagttcc agctacatca 240

gtgcatgtaa tatgagatga ggtggttgtt gcccatttag caagtccaaa gtcagagagc 300

tgcggctcaa aatcatcaga gagtagtata tttgacgatt taacatcacg gtggatcact 360

ggttgagcat ccggcaaata ctaggagaaa tcttaggcac attatgcttt gactaatctt 420

tttagctttt aagcaaaatg agcgataatc aagtgcgaag cattagagtt ggggcactta 480

tcatgatgca atgttcttcg actttcctgt tcttcgtcca ttagtcttat gattagcacc 540

gagttca 547

<210> 136
<211> 669
<212> DNA
<213> Nicotiana tabacum

<400> 136

gatctaaccg ggatcacagg aagagtcaag tcgcacagca atggggatat atggcgcgaa 60

ctacatatgt tctcctctta aagatattcc cgggcccatg aattcctcct ccttatgaag 120

gaaaatggca acattacagt tcctccaatg aaatctgact tctatccctt taggaaaaag 180

taaaactaaa atggcacctt gagaaaatgt ttcttttttt acattttaaa agttctatat 240

tgaatcaggt aagtaaatag aaaacagtaa aaaggattga agttgagcag cttgatcttg 300

ataagtcgcg agtgtagcat ttctgcagtg atagaaaaat tcccttcatc tgtcatgtct 360

actttatgtc gcatgctgat gctccaagtc agtcactatg gtgatatttt catcttcaaa 420

attgtcttcc tccactttgg catgttcatt tgaattgtct tcctccactt tcacgtgctc 480

gtctgaatca accgaaggct gatattctga gtctgtcacc atggcaatat tttcatgtcc 540

aaagttttct tccgcttttg cttctctgta atctttcgga agtttattac agttaatggt 600

atgccatact ggcctctctt cctggaagag agggtgtttg cagagggttg gacagtgtac 660

ggttagatc 669

<210> 137
<211> 320
<212> DNA
<213> Nicotiana tabacum

<400> 137

gatctaaccg cacaaacgca accaaatgaa caaccgaacc atactgagtt tattttagta 60

tgatatcggt ataacatatc taaatggaat accgaaaata caaaaagaaa atctttcaaa 120

accataccaa accaaccgat gaacaccact atatctagtc attactgaaa ggcacatttt 180

acatcagacg gccagtatgt aacaacattt gctaaaacat aaggcacgat aaagtataat 240

cacacgatat acatatagaa atcagcataa tacaaagaca gaacaaaata tcattcagta 300

accttcacgc cggttagatc 320

<210> 138
<211> 420
<212> DNA
<213> Nicotiana tabacum

<400> 138

gatctaaccg gaaatgacat tttcaagggc gatactcctc cagcatcagc tgagaagcaa 60

ctgagttcaa caaagttgcg agaaatgagt ggaagtaata tatttgcgga tggaaaagta 120

gaatctcggg acttctatgg cggggttcgt aagcctccag gtggtgagag cagcactgca 180

ttggtgtaac ttgctaggtc taactcgatt tacatcatta ttaattgtgt ttccttttta 240

tccattatta tttgtagtgg gggtgtttgg ttctgatgtg atttgaccag tgttctggaa 300

tttggtatgt cctaggtctt atttgacatg tcgaccagag cttaagaact tttggctaat 360

tgggttttat gttcataaat ctgtgtttct ggttaaaaaa aaaaaaaaac aaaattatgc 420

<210> 139
<211> 565
<212> DNA
<213> Nicotiana tabacum

<400> 139

tggattggtc gaggcctcct ttaccttcac agggattcaa gactaaggat tatccacagg 60

gatttgaagg caagcaacat tttgttggat gaatacctga accccaaaat ttcagatttt 120

ggcatggcaa ggattttttc aggcaaccaa gatcaggcca atacaagtag ggttgttgga 180

acctatggct atatggcacc tgaatatgca atggaaggaa gattctcaga aaaattagat 240

gtttacagtt tcggagtttt attattggaa attataagtg gaaggaggaa cgcttagctt 300

tcaccaagat gattgtgcaa taagccttct agcatatgca tggaagcctt ggaacgaaaa 360

cagatagtcg aattgggttg acccccaaga taattgacct gcagctcgag aaggaaattc 420

taagatgtgt acatgttgga ctattgtgcg tacaaaaata tgcagaagac agaccaaatg 480

tctccacagt tttgtctatg ctcaccaggg aaattgctta tttaccaagt cctaaacaac 540

ctgcatttac aacaggacca atcca 565

<210> 140
<211> 195
<212> DNA
<213> Nicotiana tabacum

<400> 140

gatcacgtac acacacaaat tggaaggggg tggaagttga attaagaagg aaaaaaaaca 60

gataggagaa agtagatcta tgttgttctg ctatgtaact taaattgttc cgttctttaa 120

atccgcagtt tgttgttaaa caatagatgg ctggacgagc tccttgtata cactcgacac 180

actgtacgtg atcaa 195

<210> 141
<211> 315
<212> DNA
<213> Nicotiana tabacum

<400> 141

gatcacgtac cccataaacc actcccacct ctccctttga atttgatcct ttcctttttc 60

ttcttcttct tcttcttctc tctctaaatc tctctctttt cagaagatct aattttttcc 120

tgggtttgca tagtggttta ttattaagat cagcttcatt tccggtagaa tggatttttt 180

ctttaggaat gtaaatgatg attcttcaac tatccagcaa gatattgtta ggtgcccatt 240

tttgcggaac atcaatgagc caacaaactt ttccttctca agctccatgg ctttcccact 300

tcctgtacgt gatca 315

<210> 142
<211> 354
<212> DNA
<213> Nicotiana tabacum

<400>       142

gatcatagcc atataattaa ttgagtgaaa aataaattaa aaaaaagaga aagaaaaatt       60

gagcatacaa attcaagact aattacattc gacaaaagaa agccacatgc aaagatatct      120

acagcttcag cagcaaaacc tgaaatacat ttaaaggaaa caaaacgact acattttcac      180

ttcagttttc ctcttcaata tctgtaaaac cctcatccaa ttcaccagaa cccaatcgtc      240

tgtcaacctg atcaactatg ttccctgtgc tgaactgcca tttgctaacc cttccaagag      300

gcctctcgga aagtgacatc aaacctcttg tcgcaaaacc attggctatg atca            354

<210>       143
<211>       341
<212>       DNA
<213>       Nicotiana tabacum

<400>       143

tggtaaaggg caagataatg gtcaagtgca ttgagaatca gccttcagtt gaggtggttc       60

tgggaaagca tgtcttcttg agtgttggag attatttctt gaacaggaaa tcacagtaac      120

agagactaat actctgccaa tataattttt gacctgagaa atccaaagtt acaaattttc      180

ccaatttttt ttttaaaaat tattgtgcat ttgcctcatc cctgcatact tctattgcaa      240

cagatctgta atgtaattgc cttctgggcc attttaacaa ggaatagtgt aattggtgaa      300

aaacaatttt gattaattaa gtgtcttctg attggtggta a                          341

<210>       144
<211>       342
<212>       DNA
<213>       Nicotiana tabacum

<400>       144

tggtaaaggg gggagggcac acagctcctg agtacaaacg tgaagatagc tttcatatgt       60

ttaaaagatg gatagctcag ctacctttgt gaacttttta attaacttcc cgtttacttc      120

tttgaattac tatgaaatat tatacatctc tagacttcag aacttctgaa tcagaagagc      180

ataaaatctg ggaaaagttg ttcaattgtc tcatcacaca ctctattgat agggagaaca      240

cattgtcaat tgttcagctt cgtactcatg taaaatagaa taatatctca agtggtagta      300

cagatacact gaaaaatgat atagtatact tgtattgggt aa                         342

<210>       145
<211>       241

<212>	DNA
<213>	Nicotiana tabacum

<400>	145

tacctaagcg ggagagggac aagagacaga agcctctatt tgaaatgtat ggaatgtatt	60

tgtattagcg aggttgtctt tagataggct attgttgtta ttagattaaa tttattttca	120

gaacagagtc tggtctttgt cgtgaggttt tcacggcctt ctggggttcc gacgagacat	180

ggaaatattt ctctctgact gcatcgctgt tatctctctt agagccatac atgtacgtaa	240

a	241


<210>	146
<211>	463
<212>	DNA
<213>	Nicotiana tabacum

<400>	146

gatctaaccg gcgtgaaggt tacagaatga tattttgttc tttacgtctt cgtattatgt	60

tgatttctat atgtatatcg tgtgattata ctttatcatg ccttatgttt tggcaagtgt	120

tgttacatac tggttgtctg atgtaaaatg tgtcttcagt aatgactaga taaagcggtg	180

ttcctcgtaa aaaaaaaaaa gaaaacaaaa ttatgcatat tcccgcccaa aacttaaact	240

ttgttcactc atatttccta tacctactat gctcccaaaa agaatgaaga tcagcagaaa	300

atcaaattag catcctgcac tcaactgtgt agaaccaact gcagtcctgt gctgaagatg	360

caaagcagcc aaaaggcctt gccatgtttc tccaggtcct ccagccactt gaaaatcaag	420

gagctttctc tgtttcctgt agtattcttc tagcggttag atc	463


<210>	147
<211>	246
<212>	DNA
<213>	Nicotiana tabacum

<400>	147

gatctaaccg ctagaagaat actacaggaa acagagaaag ctccttgatt ttcaagtggc	60

tggaggacct ggagaaacat ggcaaggctt ttggctgctt tgcatcttca gcacaggaat	120

gcagttggct ctacacagtt gagtgcagga tgctaatcga tttgctgctg ctcagccttc	180

ttttgaagcc tcgaacattc cttcctgtac gaggcgctcc gaaccttgtc taggaatcac	240

ataccg	246

<210>	148
<211>	219
<212>	DNA
<213>	Nicotiana tabacum

<400>	148

acctgatgtt tacctcatga gttgcctgtc gaatgccgat gtagataaca tgcctctgtt	60

ttcttcagct ctcctcataa ggtagtgcat aatctggtca acaggtccaa atggcaaata	120

cttcacttga aatactgcat ttctcagccc aaaagtaagt ccctctgcca taccatatag	180

ctgagcaaat tggagctttt gactatcctt gattccata	219

<210>	149
<211>	276
<212>	DNA
<213>	Nicotiana tabacum

<400>	149

gatctaaccg gacgtgatgc tttgctttgt aatcttagag ttggtagtgc agtcattaaa	60

ccagtgctac caaacaacaa tgtacaaaaa caaatggtga agtgaatgtt aggaaaaagg	120

gagtcttttg cgtatacaca tggttgtggg tgcgtgtgtc gatgtggagt ttgttgcttt	180

ctggttaaat atgatgtact gctaagtagt gcaatatata gttattgttg tgataaagtg	240

tgcgtgctat atacttgtta tgtgctgttg ggtagg	276

<210>	150
<211>	203
<212>	DNA
<213>	Nicotiana tabacum

<400>	150

acgtagaaaa ttcagacttt cttttttatg ttgcaatcac ataaaaacat aaactttgag	60

gctcaatagc taaatacatg gtaattgaat cataccgaga tcataaagat gttttctcaa	120

tctcaaggtt caatcggcaa atcttggttt cggagcatat ttgtttcata agggtttaag	180

ttacatttct aaataggaag aaa	203

<210>	151
<211>	450
<212>	DNA
<213>	Nicotiana tabacum

<400>	151

cgtgatgtca cttgcgatga agatgagtga atagataact ttttgaccaa tgcaatacta	60

agaagatcat gccttacttc aatttgacga tgcttttgct gaaatgacaa cagtgcatcc     120

tctgtcagca gccaacaacc cctgagatca agaattctca tagatggtgg agggttgaat     180

acgatgagcc cagcattagt gagcacaccg tcacgaatcc ctagtgttac caagcttcta     240

atagatgaca gctgatgcaa agaggtatca gtaagggaac cactccgtaa aaacaaataa     300

ctcaatttac gaaagctcgg caaaggagtc aaagctgaat ccttgattcg agtctcctcc     360

aaatccaatc tctccaaatg atcaagatta cataacccag acagtgatga aatgcaatta     420

ggatcaaaat ccgcctcgtt gaccaatcca                                      450

<210>       152
<211>       454
<212>       DNA
<213>       Nicotiana tabacum

<400>       152

agaattgtac accgaggggt caatgcacct ttggtcctcg attgcaagat gatgaaatta      60

aacttttagc tgagtttgtg aagtctcaag ccgatcaagg ttggcctaaa atagaaaata     120

gtggagattg aaaataattc ctacatcgtt aagtctagtt caagaaatag aagaaattag     180

ctcaattgta aaggtctgtg taaacttcac cctccccaga ccccccacta gtgggatcac     240

acaaggtaga agtacatcat acacctcacc atccccatac ctcactagtg ggatcatatt     300

gggtatgttg ttgttgtaca aaatgtgtat ttcatgcttc tcattccttt gtcttttcat     360

ctccccttcc cattcgacgt tcctgctcac acatccctac cactaaaggg gatgcagata     420

gaaatagtca gtcatctcaa gcagaccaat ccaa                                 454

<210>       153
<211>       224
<212>       DNA
<213>       Nicotiana tabacum

<400>       153

gacaacgagg cacagatgag aggcatggta tcaactattt tacaaaatag gcagcgaatt      60

ttgctgaact gaaacttttg accactcctg aattatgctg gaacgaataa agagctgacg     120

tttattgcgg tggataagct tattttgaag gttgtcttgt gactgcttga catcaaagag     180

tcaaaaatac cttcaaaggg ttgccaacgt tggttcatgg taaa                      224

<210>       154
<211>       380

<212>        DNA
<213>        Nicotiana tabacum

<400>        154

acccaacaac taatgaatag aatggaagtt tgaaaaacac ctaaaacttg ccatactact        60

tataacttct ctcataacac taaagtacat tcacatgtag tacctaagtc ccatggtcat       120

tatgtactat agacatgatt atagtccctt tttttttttt tacccacaat gagacaacta       180

accatctttt gttggtggac aagttgacat agtgtaaata cataaaccta ttagcattta       240

ttcatggaac acacttcaca caccacacag taatagatac agagtcaaca gagagctctt       300

tctcttaaat aaagtacagc attcactgag cagcagaagt tccttattgt atagcttctg       360

cctcttactt gtctgagatc                                                   380


<210>        155
<211>        545
<212>        DNA
<213>        Nicotiana tabacum

<400>        155

tacccaacaa ctaatgaata gaatggaagt ttaaaaaaca cctaaaactt gccatactac        60

ttataacttc tctcataaca ctaaagtaca ttcacatgta gtacctaagt cccatggtca       120

ttatgtacta tagacatgat tatagtccct tttttttttt tttacccaac aactaatgaa       180

tagaatggaa gtttgaaaaa cacctaaaac ttgccatact acttataact tctctcataa       240

cactaaagta cattcacatg tagtacctaa gtcccatggt cattatgtac tatagacatg       300

attatagtcc cttttttttt ttttttaccc acaatgagac aactaaccat cttttgttgg       360

tggacaagtt gacatagtgt aaatacataa acctattagc atttattcat ggaacacact       420

tcacacacac acagtaatag atacagagtc aaacagagag ctctttctct taaataaagt       480

acagcattca ctgagcagca gaagttcctt attgtatagc ttctgcctct tacttgtctg       540

agatc                                                                   545


<210>        156
<211>        256
<212>        DNA
<213>        Nicotiana tabacum

<400>        156

accctggcct aaacaagata ctcacataac caaccacata aatatcgatc atgaaaatat        60

ttaagacaat aagttctaac cgcacttttg ctataaagtt ttagtttctt tcttaactag       120

tcttacatag tactagttta catttgtcac ccttttcttt ttggccaggg cgaataaagc 180

tagctatgcc tttttaacta atcataattg attgccacaa tttgactaat taggaagact 240

ctaaatgaga ctactt 256

<210> 157
<211> 219
<212> DNA
<213> Nicotiana tabacum

<400> 157

gatctcagac aagtaagagg cagaagctat acaataagga acttctgctg ctcagtgaat 60

gctgtacttt atttaagaga aagagctctc tgtttgactc tgtatctatt actgtgtgtg 120

tgtgaagtgt gttccatgaa taaatgctaa taggtttatg tatttacact atgtcaactt 180

gtccaccaac aaaagatggt tagttgtctc attgtgggt 219

<210> 158
<211> 236
<212> DNA
<213> Nicotiana tabacum

<400> 158

acccaatcgc atgaacatga actagattaa cacctttgaa cagacgaaat gttcatggat 60

acatctaacc tattacaatg ccattagaag acctaacaat tcctgaaaga aatagaaaat 120

tggataagga cctatgagaa acatagaaga ctgatctaac gagtatccgt tgttagcaac 180

gaggtcattt aactgtcctg cagctcaagt acacaagact caaagtttta ttgccg 236

<210> 159
<211> 271
<212> DNA
<213> Nicotiana tabacum

<400> 159

gatctcagac ctgaagaaga ttgaacgagg tgtaaggagg catttccatg atgatatcac 60

ggtcgtggtt gtgtttatgg atccatattc catgaataga actcttctca aagtccaatc 120

ctccattaga ggaggagagg agaggaggag gtatcccgcc ctctcccaaa cgatagtttc 180

ccaagactta acgaattctc actccaatcg atccacttgg aaagactgca gaagatactg 240

taaccaccac atgtgccaaa acaacatcga a 271

<210>	160
<211>	437
<212>	DNA
<213>	Nicotiana tabacum

<400>	160

aggcaccaca gggaagattg tccaccacca gtatgttccc aaaaccagca tcaaactcca	60

aggggtcctc ctccattaag tcatcatcat cactgggtat gccacaatct tcaccaggag	120

gaagatggat ggagtcaaga tcgatttggg aaagttccac accaatccta gcggctatga	180

tcatttacat attcggatat catttcaaac cgtataatga caatgcatca aaaagaatag	240

aaaccaattc ctggcaaaaa atgaaataca cgaaatatcc ttctatacat ggccagtttt	300

catatcagac ttgccattaa gtggtccttt ctttttccca ttatcttttc ttcttagtaa	360

tatcaaaaca aagatatcaa aatataaaca ctaattctaa ccaaagtttg gttgaatgat	420

ctgggtaggc tatgatc	437

<210>	161
<211>	266
<212>	DNA
<213>	Nicotiana tabacum

<400>	161

aggctatgat catttacata ttcggatatc atttcaaacc gtataatgac aatgcatcaa	60

aaagaataga aaccaattcc tggcaaaaaa tgaaatacac gaaatatcct tctatacatg	120

gccagttttc atatcagact tgccattaag tggtcctttc tttttcccat tatcttttct	180

tcttagtaat atcaaaacaa agatatcaaa atataaacac taattctaac caaagtttgg	240

ttgaatgatc tgggtaggct atgatc	266

<210>	162
<211>	268
<212>	DNA
<213>	Nicotiana tabacum

<400>	162

gatcatagcc tacccagatc attcagccaa actttggtta gaattagtgt ttatattttg	60

atatctttgt tttgatatta ctaagaagaa aagataatgg gaaaaagaaa ggaccactta	120

atggcaagtc tgatatgaaa actggccatg tatagaagga tatttcgtgt atttcatttt	180

ttgccaggaa ttggtttcta ttctttttga tgcattgtca ttatacggtt tgaaatgata	240

tccgaatatg taaatgattg ttgcctaa	268

<210>	163
<211>	275
<212>	DNA
<213>	Nicotiana tabacum

<400>	163

gatcatagcc ctgaagccaa atcaaagcca tggtatggat tgctgatgct tttaagaaaa	60

tacccggagc attttgtcat caatacgcgg tccaagggac gggtgacttt ggagtttgtt	120

tctcttgtct cactactttc atgagagctt ataagaattt taatcattta gttgtaattt	180

agctttaagt agggtgtgaa atatgggtcc tgaaacatcg ctgtgaattt cgatactgga	240

gatcaaattt gtcgatgaca tgtacttgtg gctaa	275

<210>	164
<211>	191
<212>	DNA
<213>	Nicotiana tabacum

<400>	164

cgaggagaat agatgacttg tagtaattcc tgtttagtgc tgagctgtta tgcatatttt	60

tcagacgcca caatctggag acccttgcgc tggagtttca atacaatcta cataagcttt	120

aatttacgct ggccatactc gtgcgcttgt ttaacccttc aagaatatgt attttggtga	180

catcaagcag a	191

<210>	165
<211>	456
<212>	DNA
<213>	Nicotiana tabacum

<400>	165

gatccagtac ccaaaaccaa caacaagatt tagatccaat tccaatatta tcttcttcta	60

ctcgctcaaa gcctacaatt tcctctctac ttttagcacc attttctcct ccaagccaat	120

tgaatagcaa cagcacccca agttctccac cctggtgaat aatatcttgc acttctcttc	180

actttttctt tcacaaatgt atacctgaaa tgttgtgtga catacttgac gtcttcggcc	240

tcgagcccga acgcttcggt ggtctcgagc gtcacgagtg tcgacgatga cggtggtagc	300

cgctctacaa aaggccgccg gaggcaccac gacagaagtt catctccgct gaagttgccg	360

ggacctaaca tgcaacaact tttactccca tctctcaata attggctatt ttgtagatga	420

cctcttacta taaatagcat tctctgtact ggatca	456

<210>        166
<211>        240
<212>        DNA
<213>        Nicotiana tabacum

<400>        166

gatcgcattg gatgagatag tctggctgct gaatgatgtt ttgggctcca tctcgtaggc        60

ttgactagaa atgatgagga ggctatttag aaccaggtta gacttagatt gtattgtatc       120

ttgccaagtc taaagtaact tcctagaaaa gaatgacctg cctacagata tacatgcttt       180

tccgcttaat tgttcaaaca cgaggtgtct cgaaattcta tggaagatat ttatacaaga       240

tacataatag aaaaaggatt ataaacaatg tcaatatgtg attctactgt ctctcagcgt       300

cagattgaaa aaggattatt agcaatgcga tc                                     332


<210>        167
<211>        761
<212>        DNA
<213>        Nicotiana tabacum

<400>        167

gttttcgcag acgatgtata taacttaaaa tcaaaaatta tatcgacaaa ctaagaatac        60

gtttaatttg tcagaattaa ataattttaa atctttttat tttttttttt ttttagcaga       120

atccatctct tttatgctta tacattataa acacaccaca tacagacatg tcttaagttt       180

tacacaactc agcagaattt taataagtga aataagttca gaaggctttg agaaactgga       240

tataaatcaa accaagttgg aaacggctgg actctaaagc attatacttg tgcttagcgg       300

cccatggtct ggtcacccgg gggaatcata acgaccactt gttgggcgtt aaacccggga       360

tgagggagcc tacgacgaag ctcacggcct tcctggcaaa gagcacaagg gtggcagaag       420

aaatgagttg caaagtcaca agctgactca cattgctcgc gctgcacctc gtcctcaaca       480

aagctcccac agcagccgca agatctgttg agtgcctcgc agctcccctc cagattaaac       540

tttcggcgtt atagctgtac gggtgggata tgtaaagaag ggtgccacac agttcgagcc       600

caaaaaggat tgttccaagc atgtaaagcc agtgttaagc aagcatgatt ggcaaaatcc       660

agacaaaaca attctctcgg cattgctccc gtaagccacc gggcccccaa taaacaaact       720

tcaatatccc ggctgcaaaa tcatctcccg caaaacaacc g                           761


<210>        168
<211>        325

<212>	DNA
<213>	Nicotiana tabacum

<400>	168

gttttcgcag caacccaacc aattgagcaa agttacccac aaccagagaa gaagaaaatt	60

gccttcacaa gttcaaaatt tttcttgtct aattgtttga ttctgtgtat gaggaatgta	120

cgaaccattc tctttttttt ttttcacaaa tgtacaaaat gtagctgcat taactgttgc	180

tacatttaca gaaagctaaa atcaaccata tatgcttttt ttgttctgaa tccaactcta	240

ctaagctcag gttataactc cacaaagtcg gattcaaata tcggtttaat cattcaccat	300

cttacctttt tatcctgcga aaaca	325

<210>	169
<211>	180
<212>	DNA
<213>	Nicotiana tabacum

<400>	169

gttttcgcag gataaaaagg taagatggtg aatgattaaa ccgatatttg aatccgactt	60

tgtggagtta taacctgagc ttagtagagt tggattcaga acaaaaaaag catatatggt	120

tgattttagc tttctgtaaa tgtagcaaca gttaatgcag ctacattttg tacatttgtg	180

<210>	170
<211>	380
<212>	DNA
<213>	Nicotiana tabacum

<400>	170

cagggtttgt gaaaaatcta gggcttaaaa agagatctcg aaaacctgta ctttgcaagt	60

caacaatggc gcctcttccc ggaccttact ctggtaccag cacccttgct ctggtggctc	120

gtacctcggc ttcacattcg gacttgttta tggtagcatg aagctcaagt atctcagggc	180

taaggccaag tctcaccaga aggccgaagc taaggcacat cactgatgag agcagtatgc	240

tgcatttgct gtttgataaa aataatatat gacaatcata taatatcaat tttcattctg	300

gcctgttgct taggaccatc attttcgact tgtaactgtt caattttgtg ttttgagagt	360

tattagatat ttttatggtg	380

<210>	171
<211>	362
<212>	DNA
<213>	Nicotiana tabacum

<400>       171

tggtaaaggg gggataccac cgagattttg tgtttgatga ggatggtaat tggatgttac       60

agggttggaa aggccgggta gtatgtgcta gctcgtgttg ggtgccagca tagaagtatc      120

tggaacttgt ttgttgtcaa actgggaatc ctgaggtctc cagaaaatat cacaggagga      180

ctgcattcat gcatgagatt gcacttctgt atgcagctgc ttgggtggtg gcacaatttt      240

cgttttagtt cataggatgc tgttatgtag ccaaaaccga cgtgttatct agctattctg      300

tacataatgt tgttcctaga aaggattggt ctttgctaat cttgattaag cttagtatgg      360

tg                                                                     362


<210>       172
<211>       309
<212>       DNA
<213>       Nicotiana tabacum

<400>       172

tggtaaaggg caaaggggga acaaagaagg aaaaaaaatg gaaagtaggg aaactcgtcc       60

tctttgttga tgctaataaa gactgaggtg tgtgaatagc tactcttttt tgcagttgta      120

acgtctgatc gctgatcctg taggttttag tctcatgcct atgctcagtg ataaatattt      180

cgtattacta acataagcct tactagtttt ttgggcatga agttctttta cattttcagt      240

actatatttt actagctcta acaggttatc ctatccacct tcggttcatt tactaactca      300

tatttggtg                                                              309


<210>       173
<211>       185
<212>       DNA
<213>       Nicotiana tabacum

<400>       173

ttggtaaagg ggggatacca ccgaattttg tgtttgatga ggatggtaat tggatgttac       60

agggttggaa aggccgggta tatgtctagc tcgtgttggg tgccagcata caagtatctg      120

gaacttgttt gttgtcaaac tgggaatcct gaggtctcca gaaaatatca caggaggact      180

cttca                                                                  185


<210>       174
<211>       233
<212>       DNA
<213>       Nicotiana tabacum

<400>       174

ggattggtct acaggggaat tgactggttg atagggtcta tgttagttgt atgatgatga       60

cgatcatttc tccaattact ttggtctcag cttttagtac ttgaaattga aacaaaacac      120

tcagctttcc ttcagcttgt acttgaagac aagcatgtta tatatcttag gcattatgtt      180

tttccttacc aaaatcatgg tttctataga ctagttttgt attgtcttgg tga            233


<210>       175
<211>       228
<212>       DNA
<213>       Nicotiana tabacum

<400>       175

ggtctacagg ggaattgact ggttgatagg gtctatgtta gttgtatgat gatgacgatc       60

atttctccaa ttactttggt ctcagctttt agtacttgaa attgaaacaa aacactcagc      120

tttccttcag cttgtacttg aagacaagca tgttatatat cttaggcatt atgtttttcc      180

ttaccaaaat catggtttct atagactagt tttgtattgt cttggtga                   228


<210>       176
<211>       301
<212>       DNA
<213>       Nicotiana tabacum

<400>       176

tggattggtc gacacacaga aaccaccaga gatgatatca ggcaacatga cttcagcaat       60

gtgtgtgtat tctgcattgt tcatgagatt tgcatggatg gtacagcctc ggaattatct      120

tcttctggca tgccatgcct cgaatgaatc ggtgcaactc tatcaactat cacgttgggc      180

aaaaagtcaa ggatatttgc agcagaatgc agccaaagca gagtgattta gtgttctctc      240

ttgtttattt ttggacaatt ttcgcttgtc atttctgagt gctttcatca attgttgcag      300

g                                                                      301


<210>       177
<211>       287
<212>       DNA
<213>       Nicotiana tabacum

<400>       177

tccacagagt ttggtcttgg atgcagcaga cacagttgct cttcacataa attggggaat       60

gacattattg ataaacaatc aaaatgcctt gatgaaagca caagaagaga tagacacaaa      120

agttggtaag gatagatggg tagaaagcca aattttccat atctagaatg gaagaaatag 180

aagaaacaac atttacagta ccaaacttat cgtggcattt caagatcagg tctttgatcg 240

agtgcctgaa ttgttgtatt atctggaagt gcatcagggt agaaaga 287

<210> 178
<211> 146
<212> DNA
<213> Nicotiana tabacum

<400> 178

ctttctaccc actgtatgtt aactgaaatg gttccctata tcttcttctc acattttcaa 60

ataagtttct cttagagcga ttgagaagtt gagtatatgt ttttgtgaac ttgaaacttt 120

taggcagagg cgtctattaa caggga 146

<210> 179
<211> 466
<212> DNA
<213> Nicotiana tabacum

<400> 179

ctgcttgatg ccgtccggtg catgtcgacg aagcttttcg ttggtggtct ttcatgggga 60

actgatgatc agtctctgag agatgccttt gctacctttg gtgatgttgt tgatgcaagg 120

gtaattgttg acagagattc tggcagatca aggggatttg gatttgtgaa cttctcagat 180

gatgaatgtg ccaatgaggc tattaaggca atggatggtc aggaactcca gggaaggaat 240

attcgtgtta gtattgccca agagagagct cctcgaagcg gaggttttgg cggttccggt 300

ggtggatttg gtggcggcta tggtcaagct agagacaatg atggatacta agtcactttt 360

atttttgcta agctgtcagt gtgtgaatga taacgtttat ctaagtagag gacttttggg 420

ggatagcttt gttgagttta tctatattaa gacttctttt cgtgcg 466

<210> 180
<211> 178
<212> DNA
<213> Nicotiana tabacum

<400> 180

ctgcttgatg gggcatactt aaatggaaac tggcaaagat aattaggttg atttagtgtg 60

ctgctctgat ttaacaaccg ccctgtaaga tactccagta tacatagcag cttaactaaa 120

agcttgctga cctgtaaagc tcatgagtaa cgaccaaatg ttgagttttt gttgccga 178

<210>	181
<211>	356
<212>	DNA
<213>	Nicotiana tabacum

<400>	181

cggggggaaa aagaatatgg aatacaatta ctagttagat ttctatacac aggtgattac	60

aatttagagc atctctaatc gtaaaaagaa agacaaaagg tgaagtagca aattatacat	120

ccttaattat aaattcagga tttcctgagg ctagtacaat aacaagtgct ggagtagatg	180

aatcttacat cagacagtgg gagattaatc aaggtcaaat gaagctccca ttactttact	240

tcagtaaatt gtaaaggtag gccacaattc ctagaagaat acaaataagt attatatcga	300

taaagaagtt actgctggac cgtatcttgt taatattttc cttgagtctg agatca	356

<210>	182
<211>	398
<212>	DNA
<213>	Nicotiana tabacum

<400>	182

gatcatagcc aggtattgaa gataacatac ttgagcagag ttaaagcggg atcgtaagga	60

ttcaaggtag agctgcttgg tcgtcgcatt cccttagcca tgtattgaag ataacggact	120

tgagaagagt taaagcggaa tcgtaaggat ttaagcttca tttcaggaaa aggaacgaaa	180

caatctcaaa gattcctcgt ggattagtta agaaggcatt ctattgggat tctttgattg	240

atagagaagt gaagcaccta cggtgaagta aggcagcgag aaggtactat gattttatat	300

gcaaataaaa aaagataagg atatagttca accagatttt gctccaaaag atgtgtgagg	360

caattgaatg gaactatgga aggatcctaa gtgtgtca	398

<210>	183
<211>	141
<212>	DNA
<213>	Nicotiana tabacum

<400>	183

ctgcttgatg atcagttaca caatgaatag tgttctttct cttgtcaaat gtgcataata	60

tgtaaaaatg aagccgctag acttggattt cgggtacctt agtctcaaaa tttcatgaat	120

aaaaagtgaa cgagttagtc a	141

<210> 184
<211> 192
<212> DNA
<213> Nicotiana tabacum

<400> 184

tgatgaagag gggcttcttt tagcaaactg ataaaacaac aagtcaagtg tacaattgca 60

aatcaggaaa taacttgcag cagtttcagc aaaaaggaaa ttcagattta aatggttcaa 120

acattaatgc ctggagagta accccaacct ataaaataaa acctcttgag atttagtata 180

ccatcaagca ga 192

<210> 185
<211> 194
<212> DNA
<213> Nicotiana tabacum

<400> 185

ctgcttgatg ataaggtgga gaggtatgct aatcgttctg agtgagatgg tttgtaagtc 60

tagaaaactc ttaatttcgt tttaaatgtt tacagtaatt gaggtttaag gagcctcttc 120

ttgtgctttg gacgagcgta ttttatactt tgggatatgt tcttccgtat attgtagttt 180

tgattttctg gtca 194

<210> 186
<211> 190
<212> DNA
<213> Nicotiana tabacum

<400> 186

ctgcttgatg tgagaccaat tcctgctttc cttgatcact acctggatcg tgaactgagt 60

tttagcgagt aaaatctttg tactgtgatg agaaggttgg aatctgtatt gtctttttta 120

gcactcagaa ttttactgta cgtggtaata gctgagtcta caaatagaaa tcaagaagtc 180

tgatgcggtc 190

<210> 187
<211> 192
<212> DNA
<213> Nicotiana tabacum

<400> 187

ctgcttgatg ataaggtgga gaggtatgct aatcgttccg agtgagatgg tttgtaagtc 60

tagaaaactc ttaatttcgt tttaaatgtt tgcagtaatt gaggttaagg agccttctct 120

tgtgctttgg atgagcgtat tttatacttt gggatatgtt cttcggtata ttgtagtttt 180

aattttctgg tc 192

<210> 188
<211> 441
<212> DNA
<213> Nicotiana tabacum

<400> 188

tctcggagga ggactacaaa cagtttgtgg attccttaaa gcaggatgat gaagacaagg 60

agaatgaaat ggcaactgag aacatctcca aaattgatga caacataaag gagaagagag 120

tcggtataaa agactggtgg acaaagagca agtatgcata tctaaaccaa cctgccgtga 180

agtccatgga acgccaaggt tccacttata ttccacaact atattgctac aaggctcctc 240

ctccaccagt tacaagcact ttcggcatat tctaaggcaa ttcaatctac gtacagtcat 300

ataccatata tttgtattgt gcctaagtgt atatcattgt ttgctatgtg tttgattgaa 360

atggttgtat tggttttatg cactactctg ttgcttttgt aaaccaatgt caataaataa 420

tggtttattg ttggatgctc a 441

<210> 189
<211> 486
<212> DNA
<213> Nicotiana tabacum

<400> 189

ctgcttgatg ctattaaggc tgcagcccgt atcagggcac gcaatttctc ggaggaggac 60

tacaaacagt ttgtggattc cttaaagcag gatgatgaag acaaggagaa tgaaatggca 120

actgagaaca tctccaaaat tgatgacaac ataaaggaga agagagtcgg tataaaagac 180

tggtggacaa agagcaagta tgcatatcta aaccaacctg ctgtgaagtc catggaacgc 240

caaggttcca cttatattcc acaactatat tgctacaagg ctcctcctcc accagttaca 300

agcactttcg gcatattcta aggcaattca atctacgtac agtcatatac catatatttg 360

tattgtgctt aagtgtatat cattgtttgc tatgtgtatg attgaaatgg ttgtattggt 420

tttatgcact actctgttgc ttttgtaaac caatgtcaat aaataatggt ttattgttgg 480

atgctc 486

<210> 190
<211> 332

<212>        DNA
<213>        Nicotiana tabacum

<400>        190

gatcgcattg gttgacgggg aggatgaatg aggaagcatc aaggattata gaatatgaag        60

ctcgctgttt gtaagttgat gtagatgtgt tattcttttt gtttgaacgg tcatcttagt       120

ggaggggata agattgtttc tccttttcat tttttggtga gatggagcac tattccttga       180

gcacgctccg ctctatttgt tttattggca ccgagagaaa taagcattct gcatgtgagg       240

ttcacagcaa tgaaagcagc atctctgccg ctgcaacttt tagatggtat aataagttat       300

ttataaaatt gtattgttat gttgtgtggt ca                                     332


<210>        191
<211>        361
<212>        DNA
<213>        Nicotiana tabacum

<400>        191

gatcatggtc aaaagcaatt aatttaatca ataagatcat caactttgca attcagaggt        60

tgattcctag cgacttggac cactttgctt ctacttctct ggtggccttc ttacacattg       120

ttttctttgt gtcttcacgt ggaagtggct tcaacagctt agttttttat tagaatatta       180

attatttagt tgatctttga agtgtatttg agttttcatg ttttttgatg ttgcttcttt       240

ataatttatt attgtaagta tatacctttg tttggtttgt ttcgtgttaa ttattgttgg       300

ctgtctacta gtgcctactt gtgctttgtg caccaaatca ataaagagat gaattttgtt       360

c                                                                       361


<210>        192
<211>        361
<212>        DNA
<213>        Nicotiana tabacum

<400>        192

ggctccaaca agtaatgaga cgaagaaaac atgcagcctt acctacggtt acaccgcatt        60

tgcttgcaag aaaaaaattc agagcatttt ctattaaaaa atctaagtga agtgtatatt       120

aatttcgttt attcaagtta ataagtagta gtatttgtgc tactgttatg aatttcatgc       180

aatgtactat ctgaaggaag catttgaatt cctttcactc tactttgtaa tatccatgtt       240

ctctctctac ataagcagag gcagatctag gatttgaagg ttccggtgcc atattatttt       300

atatgtagat gtattattat ttatacggtc caaataggat aaatatttag tcggtttgat       360

c                                                                 361

<210>    193
<211>    380
<212>    DNA
<213>    Nicotiana tabacum

<400>    193

atctcagact gaagataaca gatttcaaat aggcaatgaa gaggaggaag agaagagacc    60

tttggtctaa atcaaaatag cacttgtttg ctctagcttt agtcttatta tttcatttga    120

ttaggttata gttgtaatat ggcaacaaaa tgttaggctc tttttcacat tgttgcaatt    180

catctaagat ggtaacattt gtgaagtaat ttgtttcctc tgagggcatt tagtccaact    240

ttagatgatg aatatatggg tgcagtaatg ccaagttctt taggttgagg atctcctggg    300

tggcatagta accaataaat cgttactaat gatccttcta tatgaaaact atactgttaa    360

aagtaacaag ttcaaggtca                                                380

<210>    194
<211>    141
<212>    DNA
<213>    Nicotiana tabacum

<400>    194

tggtaaaggg tataaagagt ataggcttgg tgattattct aattggttac agaaaagggt    60

taataatcat tggggtaaga ttcagagttg tttgcaggat agtaagatct gcaaaacttt    120

gattgatgat gggtctagca c                                              141

<210>    195
<211>    233
<212>    DNA
<213>    Nicotiana tabacum

<400>    195

ggtaaagggc aacccagtat aataaactcc cgttatgcgg aggatgcagg gaatggccgg    60

atcacaaggg tctaatttct gcgagagact gttttcgcaa ttcaaatccg tgacctacaa    120

aagtgttggg aaaaagaaag aaagtaaaaa taactacagt taagttaaat tcgattttta    180

cgagttttaa actatatatc taaacagaca atcagaataa atccaccgtc cac           233

<210>    196
<211>    412

<212>       DNA
<213>       Nicotiana tabacum

<400>       196

tgtagtagtt cctttatgta tagatgttag tcttgcacta acaagacttc ttatctatat       60

tacaagaaga aaaccgaacg caaaaattaa atcagaaaga cattatttaa gctctgatta      120

aacgttacat attacattat gctttagtca taataaagtt gcacaaatca taagcatgat      180

ataaatttca ggctgcagca acagcttctg caggcttttc gtctgcgggc tttgcttcag      240

gttcagctgg tgtatctgcg ggcttttctt ctgtaggctt tacttcagca ggctttgttt      300

cgggttctgc tggtgctggt gttgttgctt ctgcttccca gaaagaagtc ttctttccag      360

tctttgaaac agtctggaaa acagcttctg gctccacatt tccctttacc aa              412

<210>       197
<211>       168
<212>       DNA
<213>       Nicotiana tabacum

<400>       197

gatcatggtc cggggaataa ttacgggtat atcttgtata ctagtgtata cgttaaagaa       60

cggatctttt tatttttcat tgttatgtta ctggtttttg tcattcatgg aaacttgtac      120

tagtcatctc ttttttaata taccgttggg ttttaattac tctttcac                    168

<210>       198
<211>       316
<212>       DNA
<213>       Nicotiana tabacum

<400>       198

tgtggtcatt gtggcgcctg gggattgttc gccgtcgcgg agagtggtgt cgaagatgcg       60

aatgtagttc gggtcgggaa tgtggctcgg ggtatattcg ggtcgacggc ggattgcgca      120

gtggataata ggggaacatt tggagattga tggtttgaaa ttgaagagag cttgggtttg      180

aatgagagga ttttggggat ggaatgagat gttagggtta cgggaaaatg aatggtttat      240

ggtgatagac gccattggag gagaagagaa gaacagagga gtcgagttgt gtggagagtt      300

gggaatggtg gagcca                                                      316

<210>       199
<211>       326
<212>       DNA
<213>       Nicotiana tabacum

<400>	199

ggctcctact gaggatgatt atagttactc tgccatctca tgagttccac tcaactattg	60

ttgcatattg tgttctattg cacgtcgact agtaggtata ctcttattgg tgcctctacc	120

tttctacttt ggtcttcttg ttttgatgtc tttgatcatc tgcctgtata gtggagtatg	180

atccgacttc tttgcactat tgtaatcata tatctccatc ttgatgatgg cacaatcttg	240

atgttcatgt tattatatgg gagctaatat ttgtagttaa aatgattgtg gtgggaggcc	300

ttgaatactt tgatttcttt tgtaca	326

<210>	200
<211>	326
<212>	DNA
<213>	Nicotiana tabacum

<400>	200

ggctcccatt tagtgaaaga gctaacagac agagagatct ggaaattctt tttcaaataa	60

aaggcatttc ttcaacagcc gaaattttat taatctaaca ttgttcttat ttttatgctt	120

ctatatttat ttatcttaca tgcgttatgt gatgatagat tgacattcaa gcattaagat	180

gaaagcatac gtggctttga gtcctgttgg tctacttatg tagttatttt tgcttgtgga	240

ccttttcttc cttatcttga aggtgtaaat taaatgtaca ttatcttgat ttatacatga	300

tgtaatgatc ctccactttg accaca	326

<210>	201
<211>	288
<212>	DNA
<213>	Nicotiana tabacum

<400>	201

tgtgggactt aaacctcttt gaggtttacc tcttgaaatt gcctcatctt ctcattaggt	60

tgtaattatc ccccgaagca tatttgaggt aaaagacttc tgattatctg ttgttctcct	120

ggtacaaatc actgctatgt cgaagcatgt tagaaatgga cctcagtgga ctttttttgg	180

atatgacgtt ttagctttcg tgaagatttc agattcctct ctactctttt gtttaaaaat	240

ttggttgacg ttgaagctat tatgcttctg ccatttctct tttgtgac	288

<210>	202
<211>	217
<212>	DNA
<213>	Nicotiana tabacum

<400>	202

gtactaacgg gtaaataact tcttattgac ttgtattttc tgacacatgg cgtatgcttc	60

tacattcatc gcccagtgaa ccagaaaaag agagaacaaa caaaatccac tgctaagcca	120

gatcgtaaaa ttaaatgcac aaagagtttg acgacattat aatcaaccaa tttaaagatc	180

agattcgttc ttcactatta ggttttacgg ttagatc	217

<210>	203
<211>	216
<212>	DNA
<213>	Nicotiana tabacum

<400>	203

tgtagtcggg aagtcaacct cattgaacag agtataatgc tgtgtcagaa attaccgaga	60

gcacaactta aacagttcaa taacaatttt taattattca atcgtcagaa aatgaaaaaa	120

ttatgtagta caaacagaac ctatataaag gaagcaagtt gagaacgcac tcatagaaaa	180

ggatcatgca tacctagaac tttctgcggt tagatc	216

<210>	204
<211>	219
<212>	DNA
<213>	Nicotiana tabacum

<400>	204

tgtgtataaa tttgtcaatc tttaataata attacagcat agaacaagta attcccatgt	60

aaggctaagt ctcaagtatc accaaacata tactaataca taaaaatttg tatctttttc	120

caacctcaag aatctgaaag aaatactaca aagatattat acatgggact attacagtgt	180

taaaaacgaa ccggggttct aaattgagcg gttagatca	219

<210>	205
<211>	135
<212>	DNA
<213>	Nicotiana tabacum

<400>	205

taggatttgc atatactaac cattctagtg tcttttaggc tactaaatgt atttgaaatc	60

ttatggctac cggatgttat aagtttgcag cgggtggcca cctgattttt gtgggggaag	120

tgcacggtta gatca	135

<210>	206
<211>	288
<212>	DNA
<213>	Nicotiana tabacum

<400>	206

gatcaatcgc agagttagag aagttgtgga gcagagctgg actgcagact gattccaagg	60

aagaaatacg tcattgctga ccgcaaatca ggagcaccat ggctcatcta tgttacaatt	120

tacggattga aacctcttat ttgaagtttt attggaacac acttgttttg gccctagtat	180

tctggcttgg tgctgtttgc cttatcgttt gcttgcattg cattgacaag ctcctgtaaa	240

atatgtgaat gttcgttctg gtttaaaaat gcaagtaact ttctgtac	288

<210>	207
<211>	296
<212>	DNA
<213>	Nicotiana tabacum

<400>	207

gatcaatcgc gacaagattg tgatggatgc cattacagga ggtgctgttg caactgctgc	60

tgagtttctt aattatctca catgaatttc ttaccaacta cttagaacat tgctaagcat	120

ttgctctgtt tctcgaattg tatccatttg aaattacaag cttatgacat atgaatctta	180

taatatttga ctatgtttaa gtgaatctat aaattagcca tctttttcaa tcatttgctt	240

taaatatcgt ctatcaagat gaatcaattc atgtattaac aatttaatca gaagag	296

<210>	208
<211>	324
<212>	DNA
<213>	Nicotiana tabacum

<400>	208

tgtatagaaa ttggaactgc ctaacccttc aaagcagctt tattttcttt ttccttttac	60

atatcgttct tttactttta tttgggccgt caaagactaa aacatgggca tcgatggctt	120

cttattcttt gtcaactggg gatagcagaa cattcatatt gtcaccacaa ttaacaaact	180

cgtagttcac aataagatgg ccgcgttgat tccctcttcc gtctgtgtcg agctgccgga	240

aaacattaac gtccaagtct agtccgccat tgctgcattg atccacgatt ctcactgtgg	300

tctgagctcc tgtgcctgta tcga	324

<210>	209
<211>	224

<212>       DNA
<213>       Nicotiana tabacum

<400>       209

tacaacgagg gtgaagccat ggaaaaattc actgaagcta agcctcgcaa ttaattacta       60

acgaatatga agattcaatg gtggctatat attattagtt ttagtaatag ttttgttgtt      120

tggagtatga ataaatatgt atcttttcat ttgcctgtac tgttcatcta tatatttgag      180

tgtaattgtt gagacataat tataattaag ggtgtgtttg gtac                       224

<210>       210
<211>       335
<212>       DNA
<213>       Nicotiana tabacum

<400>       210

tacaacgagg gatctgctga gaaagcaaca gaaggaggaa atggctgcaa gtgcggagca       60

aactgcaaat gtgacccttg caactgctga tgaacatggt cattagctgg cccaaaaata      120

tattatgtgt tttaaaagct ttgtagtatt aataaaacta caaccatgaa actgaccatc      180

tccaaatctt tttgacaagg tttgtttgat gttgtttcat ggtttttctg tttggttttc      240

taatatgata tttgtatgaa aggaagctga tttgggctgt aacacgaact tatcaatata      300

tagaatacaa gttggcttct ccttttatta tgtac                                 335

<210>       211
<211>       300
<212>       DNA
<213>       Nicotiana tabacum

<400>       211

ggtactaagg ggaggaaaag ctaaaaatac caaatacttg agtcaaagtg gggttttggc       60

tgttctgagt gtggttagtt tataaataat gaggatgaat tgagtgaaga ctggtagaat      120

gagttgagta gaagacatgg aaaatgtaaa agcctatgtc tctcaggtgg aatagagtgt      180

tgcggttact tattcgattt gacgaagttc atggagaata tagttcgata gaaactgagt      240

ctcgaagctc aattgaaggc acagattgat tgacttctgc gctataccca ggattggaca      300

<210>       212
<211>       300
<212>       DNA
<213>       Nicotiana tabacum

<400>       212

tgtactaatg aatactatga attactatac acaatttata caaatagaaa acaaggagcc 60

agctttcaat ccacagttcc ctgttttatg gaatgcataa attcactcat tcaccaattc 120

atatggccat ttgtgagaga caaaaacacc gtcttgtttt attctagttc aaacaggcac 180

cgagccatgg caacggtgaa attcgacaga tatatgaaaa gccttggggt ttctatgaaa 240

tctacgtgta atcagcaacc tgaagttcac tggaccaatc ccttgtgctc cttagtacca 300

<210> 213
<211> 239
<212> DNA
<213> Nicotiana tabacum

<400> 213

gtgagttagt tcatgattga attcaaagaa aaatgttcta tttcttctct ttaaattccg 60

gacttcgttt tcaaattatg gagtttgttt agggtcgtat ttggttttga atttccggtg 120

ccgattcgag ttgcgtccag agttcatcgg tttgaggttt gtttcttaat ttcttctatt 180

gtttctgctt gataccatgc ttatattgtg ttattaatct ttgtgctcta tttttgaca 239

## Beschreibung

Die Erfindung betrifft insbesondere cDNA-Sequenzen, welche verwendet werden können, um bisher unbekannte Gene zu identifizieren, die an verschiedenen Signalketten zur Induktion von Resistenz in Pflanzenzellen beteiligt sind.

Pflanzen, die von einem Pathogen (Pilz, Virus oder Bakterium) angegriffen werden, entwickeln - über die direkte Abwehrreaktion hinausgehend - eine transiente Resistenz gegenüber späteren Infektionen mit dem gleichen oder einem anderen Pathogen (Sequeira, 1983; Heller und Gessler, 1986; Madamanchi und Kuc, 1991). Diese induzierte Resistenz (IR) kann entweder auf den Bereich der Erstinfektion beschränkt sein, oder sie schützt die ganze Pflanze. Die betreffende Pflanze kann nach einer Erstinfektion für mehrere Wochen bis Monate gegen ein breites Spektrum verschiedener Pathogene geschützt sein. Die ersten Versuche zur IR wurden mit Tabaksorten durchgeführt, die eine hypersensitive Reaktion auf Infektion mit TMV (Tabakmosaikvirus) zeigten (Ross, 1961). Auch viele andere Pflanzenspezies, wie Weizen, Gerste, Reis, Gurke, Tabak oder Arabidopsis (Görlach *et al*., 1996, Kogel *et al*., 1994, Smith und Métraux, 1991, Métraux *et al*., 1990, Malamy *et al*., 1990, Mauch-Mani und Slusarenko 1994) können durch Erstinfektion mit verschiedenen Pathogenen oder durch Behandlung mit Chemikalien Resistenz entwickeln.

SAR (systemic acquired resistance) ist eine besondere Form von IR, die zuerst im oben erwähnten Tabak-TMV-System beobachtet wurde. SAR wird vermutlich durch ein endogenes Pflanzensignal hervorgerufen, das im infizierten Blatt produziert und dann in andere Pflanzenteile transportiert wird und dort den Resistenzmechanismus aktiviert. Salicylsäure ist für die lokale Ausprägung der Resistenz verantwortlich, ob sie auch für den Ferntransport als Signalmolekül in Frage kommt, wird kontrovers diskutiert (Métraux *et al*., 1990; Malamy *et al*, 1990; Vernooij *et al*., 1994). Daneben konnte eine Salicylsäure unabhängige IR zum Beispiel bei der Interaktion von Arabidopsis / *Peronospora parasitica*, Tabak / *Erwinia* und Weizen / Mehltau nachgewiesen werden (Bowling *et al*., 1997; Vidal *et al*., 1997; Schaffrath *et al*., 1997).

Nicht nur Pathogeninfektion, sondern auch verschiedene Chemikalien wie Polyacrylsäure, Salicylsäure, 2,6-Dichlor-Isonicotinsäure und das kommerzielle Produkt Bion^{R} (Benzothiodiazol-Derivat) (Gianinazzi und Kassanis, 1974; White, 1979; Métraux *et al*., 1991; Görlach *et al*., 1996; Schaffrath *et al*., 1997) können Resistenz induzieren. SAR ist begleitet von der Akkumulation verschiedener mRNA Klassen, darunter die der Gene, die PR-Proteine (PR = pathogenesis related) kodieren. Die Expression dieser Gene wird auch durch Salicylsäure oder Isonicotinsäure induziert (Ward *et al*., 1991). PR-Proteine und ihre Gene werden als typische Marker für IR in Tabak verwendet und sind Bestandteile der Pathogenabwehr. Es gibt transgene Pflanzen, die konstitutiv einzelne PR-Proteine überexprimieren. Außerdem sind Arabidopsismutanten bekannt, bei denen die Mutation Elemente der Signalkette traf und die deshalb konstitutiv mehrere PR-Gene exprimieren. Beide Klassen zeigen erhöhte Resistenz gegenüber Pathogenen (Alexander *et al*, 1993; Bowling *et al*., 1994).

Gene, die Bestandteile der verschiedenen Signalketten für IR sind, eignen sich zur Verbesserung der Pathogenabwehr in transgenen Pflanzen. Ebenso können diese Gene, insbesondere deren Promotoren in Kombination mit einem Reportergen, wie z.B. GFP (green fluorescent protein) oder Luziferase zum Auffinden neuer Wirkstoffklassen in einem HTS (high throughput screening)-System verwendet werden. Jedoch zeigt der Stand der Technik, daß die Expression einzelner PR Gene in der Regel nicht ausreicht um einen zufriedenstellenden Schutz zu gewähren. Gleichfalls ist die Induktion der Expression eines Großteils der bekannten PR Proteine oftmals nicht auf biotische Faktoren, insbesondere Pathogen-Interaktionen, beschränkt, so daß für das oben beschriebene HTS Verfahren keine oder nicht ausreichend geeignete Gene oder deren Promotoren zur Verfügung stehen.

Daher liegt der vorliegenden Erfindung die Aufgabe zugrunde, Produkte und Verfahren zur Verfügung zu stellen, die eine Erweiterung der verfügbaren Pflanzenschutzmittel und Anwendungen darstellen. Diese Aufgabe wird efindungsgemäß durch die Bereitstellung der in den Patentansprüchen charakterisierten Ausführungsformen gelöst. Somit betrifft die Erfindung Nukleinsäuren umfassend eine Sequenz ausgewählt aus
(a) den Sequenzen gemäß SEQ ID NO:1 bis SEQ ID NO: 213,
(b) zumindest 14 Basenpaar langen Teilsequenzen der unter (a) definierten Sequenzen,
(c) Sequenzen, welche an die unter (a) oder (b) definierten Sequenzen hybridi sieren,
(d) Sequenzen, welche eine zumindest 70%ige Homologie zu den unter (a) definierten Sequenzen aufweisen,
(e) Sequenzen, welche zu den unter (a) definierten Sequenzen komplementär sind und
(f) Sequenzen, welche aufgrund der Degeneration des genetischen Codes für dieselbe Aminosäuresequenz kodieren wie die unter (a) bis (d) definierten Sequenzen.

Die vorliegende Erfindung begründet sich auf den überraschenden Befund, daß aufgrund der Expression eines rekombinanten viralen Nuleocoreproteins eine Vielzahl von Genen exprimiert wird, von denen in Experimenten, die im Rahmen der vorliegenden Erfindung durchgeführt wurden, gezeigt werden konnte, daß sie einen bestimmten Status der Pflanze für induzierte Resistenz anzeigen. Bei diesen Experimenten wurde überraschenderweise auch gefunden, daß beim Vergleich von primär infizierten Blättern und nicht infizierten Folgeblättern zahlreiche Gene exprimiert werden, die ebenso den oben erwähnten Status induzierter Resistenz der Pflanze repräsentieren.

Die erfindungsgemäßen Nukleinsäuren eignen sich zum Identifizieren von vollständigen Genen, die für Proteine kodieren, welche im Zusammenhang mit der Pathogenerkennung und -abwehr stehen und an erhöhter Resistenz beteiligt sind. Die Kenntnis dieser Proteine erlaubt die Aufklärung der Induktionswege des zugrundeliegenden Resistenzmechanismus. Die gezielte Beeinflussung des Resistenzmechanismus erlaubt wiederum die Erzeugung von Pflanzen, die gegenüber verschiedenen Pathogenen resistent sind. Daher sind auch die mit Hilfe der erfindungsgemäßen Nukleinsäuren aufgefundenen vollständigen Gene, das heißt, die zur Kontrolle der Genexpression notwendigen Signalsequenzen sowie die Genprodukte (Proteine), Gegenstand der vorliegenden Erfindung. Ferner können die erfindungsgemäßen Nukleinsäuren und deren regulatorische Regionen, wie nachstehend beschrieben, zur Verbesserung der Pathogenabwehr oder für den chemischen Pflanzenschutz zur Auffindung neuer Wirkstoffe genutzt werden.

Gegenstand der vorliegenden Erfindung sind auch Nukleinsäuren, die eine zumindest 70 %ige Homologie zu den erfindungsgemäßen cDNA-Sequenzen aufweisen. Die Bestimmung der Homologie erfolgt beispielsweise mit dem kommerziell erhältlichen Programm bioSCOUT der Fa. Lion, Heidelberg.

Desweiteren sind Nukleinsäuren Gegenstand der Erfindung, die an die erfindungsgemäßen Sequenzen hybridisieren. Dabei kommen die üblichen Hybridisierungsbedingungen in Betracht (Sambrook *et al*., 1989). Geeignete Hybridisierungsbedingungen liegen zwischen 2 x SSC, 60°C und 0,1 x SSC, 65°C. Bevorzugte Hybridisierungsbedingungen sind 0,5 x SSC, 62°C.

Gegenstand der Erfindung sind weiterhin Vektoren, die zumindest eine der erfindungsgemäßen cDNA-Sequenzen enthalten. Als Vektoren können alle in molekularbiologischen Laboratorien verwendete Plasmide, Phasmide, Cosmide, YACs oder künstliche Chromosomen verwendet werden. Für die Expression der erfindungsgemäßen cDNA-Sequenzen können diese mit üblichen regulatorischen Sequenzen verknüpft werden. Die Auswahl solcher regulatorischen Sequenzen ist davon abhängig, ob zur Expression pro- oder eukaryotische Zellen bzw. zellfreie Systeme verwendet werden.

Zur Expression der erfindungsgemäßen cDNA-Sequenzen können diese in geeignete Wirtszellen eingebracht werden. Als Wirtszellen eignen sich sowohl prokaryotische Zellen wie z.B. *E.coli* oder *Agrobacterium tumefaciens*, als auch eukaryotische Zellen, wie z.B. Pilz-, Pflanzen- oder Tier-Zellen.

Zur Herstellung eines Polypeptids, das von den erfindungsgemäßen cDNA-Sequenzen kodiert wird, können Wirtszellen, die zumindest eine der erfindungsgemäßen cDNA-Sequenzen enthalten, unter geeigneten Bedingungen kultiviert werden. Die gewünschten Polypeptide können danach auf übliche Weise aus den Zellen oder dem Kulturmedium isoliert werden.

Gegenstand der vorliegenden Erfindung sind auch die Polypeptide, die von den erfindungsgemäßen cDNA-Sequenzen kodiert werden.

Weiterhin sind Antikörper Gegenstand der Erfindung, die spezifisch an die vorstehend genannten Polypeptide binden. Die Herstellung solcher Antikörper erfolgt auf die übliche Weise.

Die erfindungsgemäßen cDNA-Sequenzen können insbesondere zur Transformation von Pflanzenzellen und zur Herstellung transgener Pflanzen verwendet werden. Wie vorstehend beschrieben, können auf diese Weise Pflanzen erzeugt werden, die eine erhöhte Resistenz gegenüber verschiedenen Pathogenen aufweisen. Die Herstellung transgener Pflanzenzellen, Pflanzen oder Pflanzengeweben erfolgt auf die übliche Weise, insbesondere unter Verwendung des *Agrobacterium tumefaciens*/Ti-Plasmid-Systems oder einer "gene gun"-Methode. Es können sowohl transgene monokotyledone als auch dikotyledone Pflanzen unter Verwendung der erfindungemäßen cDNA-Sequenzen erzeugt werden.

Gegenstand der Erfindung sind auch die Nachkommen oder Ernteprodukte der vorstehend genannten Pflanzen.

Die erfindungsgemäßen cDNA-Sequenzen können auf die übliche Weise hergestellt werden. Beispielsweise können die Nukleinsäuremoleküle vollständig chemisch synthetisiert werden. Man kann auch nur kurze Stücke der erfindungsgemäßen cDNA-Sequenzen chemisch synthetisieren und solche Oligonukleotide radioaktiv oder mit einem Fluoreszenzfarbstoff markieren. Die markierten Oligonukleotide können verwendet werden, um ausgehend von Pflanzen-mRNA hergestellte cDNA-Banken zu durchsuchen. Klone, an die die markierten Oligonukleotide hybridisieren, werden zur Isolierung der betreffenden DNA ausgewählt. Nach der Charakterisierung der isolierten DNA erhält man auf einfache Weise die erfindungsgemäßen Nukleinsäuren.

Die erfindungsgemäßen cDNA-Sequenzen können auch mittels PCR-Verfahren unter Verwendung chemisch synthetisierter Oligonukleotide hergestellt werden.

Die erfindungsgemäßen cDNA-Sequenzen können zur Isolierung und Charakterisierung der regulatorischen Regionen, die natürlicherweise benachbart zu der kodierenden Region vorkommen, verwendet werden. Solche regulatorischen Regionen stehen ebenfalls im Zusammenhang mit der Induktion von Resistenzmechanismen und sind daher Gegenstand der Erfindung.

Die regulatorischen Regionen können zur Herstellung von rekombinanten DNA-Molekülen verwendet werden. Beispielsweise können die regulatorischen Regionen fünktionell mit einer heterologen DNA-Sequenz, d.h. mit einer DNA-Sequenz, die natürlicherweise nicht benachbart zur der regulatorischen Region vorkommt, verknüpft werden. Auf diese Weise kann die heterologe DNA-Sequenz unter der Kontrolle der regulatorischen Regionen exprimiert werden. Beispiele für heterologe DNA-Sequenzen sind Nukleinsäuren, die für Peptide, Polypeptide, Antisense-RNAs oder Ribozyme kodieren. Insbesondere kodiert die heterologe DNA-Sequenz für einen selektierbaren und/oder nachweisbaren Marker.

Die vorstehend beschriebene regulatorische Region oder das vorstehend beschriebene rekombinante DNA-Molekül können in einen üblichen Vektor oder eine Wirtszelle eingebracht werden.

Mit Hilfe der erfindungsgemäßen cDNA-Sequenzen bzw. der damit auffindbaren regulatorischen Regionen können neue Wirkstoffe für den Pflanzenschutz, insbesondere zur Resistenzinduktion, identifiziert werden. Dazu wird ein rekombinantes DNA-Molekül, das eine regulatorische Region eines Gens, das an der Resistenzinduktion beteiligt ist, und ein Gen für einen selektierbaren oder nachweisbaren Marker umfaßt, in eine geeignete Wirtszelle eingebracht. Die Wirtszellen werden in Gegenwart einer Verbindung oder einer Probe, welche eine Vielzahl von Verbindungen umfaßt, unter Bedingungen kultiviert, die die Expression des selektierbaren oder nachweisbaren Markers erlauben. Auf diese Weise kann eine Verbindung oder Probe, die zur Suppression oder Aktivierung und/oder Verstärkung der Expression des selektierbaren oder nachweisbaren Markers in der Zelle führt, identifiziert oder nachgewiesen werden. Eine solche Verbindung oder Probe kann zur Erzielung der gewünschten Eigenschaften auf die Pflanzen ausgebracht werden.

Da die erfindungsgemäßen cDNA-Sequenzen dazu verwendet werden können, Polypeptide zu identifizieren und zu charakterisieren, die bei der Induktion von Resistenzmechanismen in Pflanzen eine Rolle spielen, ermöglicht die Erfindung auch das Auffinden von Inhibitoren oder Aktivatoren solcher Polypeptide. Diese können dann auf Pflanzen angewandt werden, um den gewünschten Effekt einer erhöhten Resistenz gegenüber Pathogenen zu erzielen. Beispielsweise werden Wirtszellen, die die erfindungsgemäßen cDNA-Sequenzen enthalten und das entsprechende Genprodukt exprimieren oder das Genprodukt selbst mit einer Verbindung oder einem Gemisch von Verbindungen unter Bedingungen in Kontakt gebracht, die die Wechselwirkung zumindest einer Verbindung mit dem Polypeptid oder der Wirtszelle erlauben.

Gegenstand der Erfindung sind somit auch die Verbindungen bzw. Pflanzenschutzmittel, die nach den vorstehend beschriebenen Verfahren erhältlich sind.

Die vorstehend beschriebenen erfindungsgemäßen Produkte und Verfahren können verwendet werden, um die Pathogenabwehr von Pflanzen zu verbessern oder transgene Pflanzen herzustellen, die eine erhöhte Resistenz gegenüber Pathogenen aufweisen. Sie können ebenso zum Auffinden neuer Wirkstoffe für den Pflanzenschutz oder zum Auffinden von Genen, die an der Induktion von Resistenz in Pflanzen beteiligt sind, verwendet werden.

Im folgenden wird die Erfindung näher beschrieben. In der US 5,569,823 ist die Erzeugung resistenter Tabakpflanzen beschrieben, die gegenüber dem TSW-Virus (TSWV; tomato spotted wilt virus) resistent sind. Für diese Resistenz ist die Expression eines modifizierten TSWV-Gens verantwortlich. Die Nachkommen verschiedener transgener Tabakpflanzen zeigen neben der hohen, spezifischen Resistenz gegenüber TSWV und anderen Tospoviren auch eine erhöhte Resistenz gegenüber nicht verwandten Viren, wie TMV (tobacco mosaic virus) oder TRV (tobacco rattle virus) und zum Beispiel gegenüber dem pilzlichen Pathogen *Botrytis cinerea*. Sie zeigten dabei eine 20-30%ige Verbesserung gegenüber den Wildtyppflanzen. Dieser Phänotyp breit wirksamer Resistenz ist am besten mit dem solcher Pflanzen vergeichbar, bei denen eine Resistenz durch ein Pathogen induziert wurde (IR bzw. SAR). Da die breit wirksame Resistenz in unabhängigen trapsgenen Pflanzen nachgewiesen wird, muß sie von der Expression des Transgens (TIR = Transgen induzierte Resistenz) abhängig sein. Salicylsäure, die auch als Signalmolekül bei induzierter Resistenz diskutiert wird, konnte beim Vergleich von transgenen und Wildtyppflanzen weder im primär infizierten Blatt noch in nicht infizierten Folgeblättern in erhöhten Konzentrationen nachgewiesen werden. Ebensowenig ist die Expression der PR-Gene in den transgenen Pflanzen erhöht. Das zeigt, daß die hier beschriebene, neu beobachtete TIR durch eine bisher nicht beschriebene Signalkette ausgelöst wird. Beim Vergleich von primär infizierten Blättern und nicht infizierten Folgeblättern von Wildtyppflanzen entspricht die PR-Genexpression sowie die Änderung der Salicylsäurekonzentration den publizierten Werten. In diesen Pflanzen werden, neben den schon bekannten, neue Genprodukte gefunden, die ebenso Gegenstand der vorliegenden Erfindung sind.

Die erfindungsgemäßen Nukleinsäuren wurden durch das folgende Verfahren hergestellt und analysiert. Aus der nachstehenden Darstellung ergibt sich außerdem, daß die erfindungsgemäßen Nukleinsäuren geeignet sind, um einen neuen Induktionsmechanismus aufzuklären und diesen für die Erzeugung resistenter Pflanzen zu nutzen.

### 1. Phytopathologie

Verschiedene transgene Tabakpflanzen wurden zur Überprüfung der erhöhten Resistenz mit TMV und TRV infiziert. TMV ruft bei den Kontrollen (Samsun NN) und den transgenen Tabakpflanzen eine HR (HR; hypersensitive response) hervor. TRV verbreitet sich in den Pflanzen systemisch, so daß neben den primären Symptomen systemische Verbreitung und Schäden auf den Folgeblättern zu beobachten sind. Es wurden transgene Pflanzen und Kontrollpflanzen untersucht. Die Vorgehensweise und die Resultate der phytopathologischen Experimente sind im Detail in der US 5,569,823 beschrieben. Die Experimente zur TRV- und TMV-Infektion wurden mit einer wieteren unabhängigen transgenen Pflanze in gleicher Weise durchgeführt und mit gleichem Ergebnis abgeschlossen.

Da unabhängige transgene Pflanzen den gleichen Phänotyp zeigten, ist die Expression des Transgens und nicht die Integration der T-DNA für den beobachteten Phänotyp verantwortlich. Es handelt sich also um eine durch das Transgen hervorgerufene, induzierte Resistenz (TIR = transgene induced resistance).

### 2. Bestimmung des Salicylsäure (SA)- und Salicylsäureglycosid (SAG)-Gehaltes

Ein typischer Sekundärmetabolit und Teil der Signalkette bei der hypersensitiven Antwort ist Salicylsäure. Sie wird auch als Signalmolekül bei induzierter Resistenz diskutiert. Aus diesem Grund wurde der Gehalt an Salicylsäure (SA) und ihrem(n) Glykosid(en) (SAG) in verschiedenen transgenen Pflanzen und Kontrollpflanzen bestimmt. Der Salicylsäuregehalt in nicht infizierten Pflanzen (Samsun und zwei unabhängige transgene Pflanzen) beträgt in 4 - 5 Wochen alten Pflanzen 30 - 50 ng/g Frischgewicht (FW) (freie Säure) bzw. 200 - 300 ng/g FW (SA-Glycosid). Die transgenen Pflanzen haben keinen konstitutiv erhöhten Gehalt an Salicylsäure. Einen Tag nach Infektion sind in den Blattextrakten noch keine erhöhten Salicylsäuremengen nachweisbar. Nach 3 Tagen ist sowohl in den mit TMV infizierten Blättern der Kontrollpflanzen als auch in den transgenen Pflanzen eine deutliche Zunahme der freien Salicylsäure und des Glycosids zu messen. Dieser Anstieg setzt sich bis zum 6. Tag fort. Die SA-Konzentration in Blättern oberhalb der Primärinfektion erhöht sich bis zum 6. Tag nicht oder nur sehr schwach. Alle Pflanzen reagieren auf TMV-Infektion mit lokalen Nekrosen (HR). Die Infektion wird in diesem Stadium gestoppt, es erfolgt keine systemische Infektion. Bei den transgenen Pflanzen wurden nach Infektion weniger Nekrosen beobachtet. Die Akkumulation von Salicylsäure ist ebenfalls geringer. Eine Korrelation zwischen Anzahl der Läsionen und SA-Gehalt in den TMV infizierten Blättern wurde auch von anderen Autoren beobachtet (Yalpani *et al*., 1991; Enyedi *et al*., 1992).

### 3. Expression von "pathogenesis-related proteins" (PR)

SAR ist begleitet von der Akkumulation verschiedener mRNA Klassen, darunter die der Gene, die PR-Proteine kodieren. Die Expression dieser Gene wird auch durch Salicylsäure oder Isonicotinsäure induziert (Ward *et al*., 1991). Die Gene sind also Zielpunkte in einer Signalkette, die im Zusammenhang mit SAR angesteuert wird oder die direkt für SAR verantwortlich ist. Aus diesem Grund wurde geklärt, ob auch TIR die PR-Genexpression verändert. Die Expression der Gene PR1, PR3, und PR 5 wurde mit Hilfe von "Northern Blots" analysiert. In den primär infizierten Blättern ist nach 2 Tagen PR1, PR3 und PR5 Expression nachweisbar, die nach 6 Tagen ihr Maximum erreicht. In den systemischen Blättern werden die PR Gene später und schwächer exprimiert. PR1 und PR3 mRNAs sind 6 Tage nach Infektion nachweisbar und zeigen nach 12 Tagen maximale Akkumulation. PR5 Expression ist erst etwas später nachweisbar, erreicht aber ebenfalls nach 12 Tagen das Maximum. Der zeitliche Verlauf so wie die Stärke der PR-Genexpression in primär infizierten und systemischen Blättern in den Wildtyp-Kontrollpflanzen ist mit derjenigen in den transgenen Pflanzen vergleichbar. Dabei variieren die absoluten Mengen in unabhängigen Experimenten. In den Mock-inokulierten Pflanzen konnte keine PR-Genexpression nachgewiesen werden. Die niedrigeren SA-Gehalte in TMV infizierten Blättern von transgenen Pflanzen im Vergleich zu infizierten Wildtyppflanzen haben also keinen Einfluß auf den Verlauf der PR-Genexpression. Möglicherweise ist bereits ein geringer SA-Gehalt, der über einem kritischen Schwellenwert liegt, ausreichend für die PR-Genexpression. Da weder der SA-Gehalt noch die PR-Genexpression ursächlich an der beobachteten TIR beteiligt sind, muß eine bisher nicht beschriebene Signalkette vorliegen.

### 4. Differential Display

Mit Hilfe von Differential Display (DD; siehe Abbildung 1) werden alle Genprodukte, in denen sich verschiedene Zelltypen oder Mutanten eines Organismus voneinander unterscheiden, im direkten Vergleich betrachtet und nach Identifikation isoliert. Da die oben erwähnten transgenen Pflanzen bis auf die inserierte T-DNA - die das Transgen trägt- isogen zu den Wildtyppflanzen sind, stehen alle zusätzlich exprimierten oder nicht mehr exprimierten endogenen Gene im Zusammenhang mit TIR. Die TIR kann im Verlauf der Signalkette durch Pathogen unabhängig exprimierte oder durch pathogeninduzierte Gene vermittelt werden. Um beide Möglichkeiten zu erfassen, wurde wie folgt verfahren: RNAs aus Gewebekulturpflanzen sowie von primär infizierten und nicht infizierten Blättern wurden 1, 3 und 6 Tage nach TMV Infektion im DD analysiert.

213 differentiell exprimierte RNAs wurden als cDNA Fragmente identifiziert und vollständig sequenziert. Die isolierten cDNA Fragmente stammen von RNAs, die entweder differentiell zwischen Wildtyp und transgenen Pflanzen oder differentiell zwischen primär infizierten und nicht irfizierten Blättern exprimiert werden.

Die Sequenzdaten wurden mit verschiedenen Methoden der Bioinformatik analysiert und aufgrund dieser Ergebnisse die cDNA Fragmente in drei Gruppen eingeteilt:
1) cDNA-Fragmente, die Homolgie zu bekannten Genen oder cDNAs aufweisen, bei denen eine Funktion vermutet wird, oder bei denen eine Funktion bekannt ist,
2) cDNA-Fragmente, die Homologie zu Nukleinsäuren zeigen, bei denen aber bisher keine Funktion zugeordnet werden kann,
3) cDNA-Fragmente, die keine Ännlichkeit zu publizierten Sequenzen haben.

Zusätzlich zu den erfindungsgemäßen cDNA-Fragmenten wurden cDNA-Fragmente isoliert, die im Zusammenhang mit IR, SAR und Pathogenabwehr den Stand der Technik wiedergeben. Unter diesen sind cDNA-Fragmente, die typische PR-Proteine kodieren wie zum Beispiel: Chitinase, anionische Peroxidase, Glyzin-reiche Proteine, PR1b und Phenylalanin-ammoniumlyase (PAL). Es ist bekannt, daß diese Gene genutzt werden können, um resistentere Pflanzen zu erzeugen ( z.B. WO 95 / 19443). Transgene Pflanzen, die z.B. PR1 unter der Kontolle eines konstitutiven Promotors überexprimieren, zeigen diesen Phänotyp. Außerdem wurde gezeigt, daß die Promotoren dieser Gene genutzt werden können, um Stoffe zu identifizieren, die die Resistenz von Pflanzen gegenüber Pathogenen erhöhen können (WO 95/19443).

Im folgenden werden ausgewählte Beispiele zu den erfindungsgemäßen cDNA-Fragmenten und das weitere Vorgehen zur Nutzung beschrieben. 
#164 (SEQ ID NO: 57)

Ein cDNA Fragment (#164)

TGGTAAAGGGCAACCCAGTATAATAAACTCCCGTTATGCGGAGGATGCAGGGAATGGCCG
1 ---------+---------+---------+---------+---------+---------+ 60
ACCATTTCCCGTTGGGTCATATTATTTGAGGGCAATACGCCTCCTACGTCCCTTACCGGC

GATCACAAGGGTCTAATTTCTGCGAGAGACTGTTTTCGCAATTCAAATCCGTGACCTACA
61 ---------+---------+---------+---------+---------+---------+ 120
CTAGTGTTCCCAGATTAAAGACGCTCTCTGACAAAAGCGTTAAGTTTAGGCACTGGATGT

AAAGTGTTGGGAAGAAGAAAGAAAGTAAAAATAACTACAGTTAAGTTAAATTCGATTTTT
121 ---------+---------+---------+---------+---------+---------+ 180
TTTCACAACCCTTCTTCTTTCTTTCATTTTTATTGATGTCAATTCAATTTAAGCTAAAAA

ACGAGTTTTAAACTATATATCTAAACAGACAATCAGAATAAATCCACCGTCCAC
181 ---------+---------+---------+---------+---------+---- 234
TGCTCAAAATTTGATATATAGATTTGTCTGTTAGTCTTATTTAGGTGGCAGGTG
 welches in unabhängigen DD-Experimenten 1, 3 und 6 Tage nach TMV-Inokulation identifiziert wurde, wurde z.B. in pGEM-T kloniert. Mit Hilfe von quantitativer RT-PCR wurde die differentielle Expression aus den DD Experimenten bestätigt. Die PCR wurde in Doppelansätzen mit RNA-Präparationen aus unterschiedlichen Infektionsexperimenten (1Tag nach TMV-Inokulation) und den Primern 164-GSP1(26-mer, GC-Gehalt 50%, 5'-***CTTCCCAACA CTTTTGTAGG TCACGG***-3', unterer Strang) und 164-GSP2 (26mer, GC-Gehalt 50%, 5'-**GGGCAACCCA GTATAATAAA CTCCCG**-3', oberer Strang) (s.o.) durchgeführt. Dabei zeigte sich, daß bei den gewählten PCR-Bedingungen (wie in Beispiel viii; in diesem Fall 28 Zyklen) mit RNAs aus infizierten Blättern ein Produkt nachgewiesen werden konnte. In nicht infizierten Blättern war bei gleichen RNA-Mengen kein entsprechendes Produkt nachweisbar.

Mit Hilfe eines RACE-Kits ( zum Beispiel Marathon™ cDNA Amplification Kit, Kat.Nr. K1802-1, Fa. Clontech, Palo Alto, CA, USA) kann ein vollständiger cDNA-Klon von #164 erhalten werden, ebenso kann man aus einer käuflichen Tabak cDNA Bank (Kat.Nr. FL1071b, Fa. Clontech) mit dem Fragment #164 den entsprechenden cDNA-Klon isolieren. Diese cDNA kann zum Beispiel unter der Kontrolle eines konstitutiven Promotors, z.B. dem 35S CaMV Promotor, oder unter induzierbaren Promotoren z.B. dem der Stilbensynthase (US 5,689,046 und US 5,689,047) oder z.B. dem Chitinasepromotor (DE 40 31 758) zur Expression gebracht werden. Die Transformation kann zum Beispiel an Blattscheiben oder anderen Explantaten mit Hilfe von *Agrobacterium tumefaciens* ( US 5,689,046) oder mit anderen wohlbekannten Transformationsmethoden (US 5,689,046) erfolgen. Regenerierte Pflanzen werden, nach Überprüfung auf stabile Transformation, mit TSWV und anderen Pathogenen inokuliert und auf erhöhte Resistenz (wie in US 5,569,823) untersucht.

Das cDNA Fragment #164 kann auch dazu verwendet werden, den korrespondierenden genomischen Klon des Gens z. B aus einer käuflichen genomischen Tabakbank (Kat.Nr. 1071d, Fa. Clontech) zu isolieren. Mit Hilfe z.B. des GCG Software-Pakets der Universität Wisconsin, Madison, Wi, USA (GCG) erfolgt die Suche nach typischen Promotorelementen, in der 5' nicht translatierten Region des Gens.

Unterschiedlich lange 5'-untranslatierte Regionen werden in Kombination mit einem Reportergen wie z.B. GUS (Jefferson 1987) oder GFP (Sheen *et al*., 1995) zur Identifizierung eines Promotors eingesetzt.

Solche Promotoren können dann in Kombination mit einem Reportergen in Screening-Systemen zur Auffindung chemischer Induktoren von Resistenz eingesetzt werden (WO 95 / 19443; DE 44 46 342).

### #630 (SEQ ID NO: 135)

Ein cDNA Fragment (#630), das in nichtinfizierten Blättern sechs Tage nach TMV-Inokulation stärker als in infizierten Blättern exprimiert ist, wurde in pCR2.1 kloniert und sequenziert. Sequenzvergleiche (GCG) mit diesem aus 547 Basenpaaren (bp) bestehenden Fragment ergaben eine Ähnlichkeit von 64 % mit dem Pto-Resistenzgen aus Tomate (Martin *et al*., 1993).

Bei dem Pto-Gen handelt es sich um eine Serin-Threonin-Kinase, deren Expression Resistenz gegenüber *Pseudomonas syringae* pv. *tomato* vermittelt. Die Phosphataseaktivität dieses Enzyms ist Teil der Pathogenerkennungs- und Abwehrreaktionen in Tomate.

Um das erfindungsgemäße cDNA-Fragment in ähnlicher Weise einzusetzen wird zum Beispiel wie folgt verfahren. Mit Hilfe eines RACE-Kits (z.B.Marathon™ cDNA Amplification Kit, Kat.Nr. K1802-1, Fa. Clontech, Palo Alto, CA, USA) kann ein vollständiger cDNA-Klon der Kinase erhalten werden. Oder man sucht in einer käuflichen Tabak cDNA Bank (Kat.Nr. FL1071b, Fa. Clontech) mit dem Fragment #630 einen solchen Klon. Diese cDNA kann zum Beispiel unter der Kontrolle eines konstitutiven Promotors, z.B. dem 35S CaMV Promotor, oder unter induzierbaren Promotoren z.B. dem der Stilbensynthase (US 5,689,046 und US 5,689,047) oder z.B. dem Chitinasepromotor (DE 40 31 758) zur Expression gebracht werden. Die Transformation kann zum Beispiel an Blattscheiben oder anderen Explantaten mit Hilfe von *Agrobacterium tumefaciens* ( US 5,689,046) oder mit anderen wohlbekannten Transformationsmethoden (US 5,689,046) erfolgen. Regenerierte Pflanzen werden, nach Überprüfung auf stabile Transformation, mit TSWV und anderen Pathogenen inokuliert und auf erhöhte Resistenz (wie in US 5,569,823) untersucht. Mit Hilfe von GCG erfolgt die Suche nach typischen Promotorelementen, in der 5' nicht translatierten Region des Gens. Unterschiedlich lange 5'-untranslatierte Regionen werden in Kombination mit einem Reportergen wie z.B. GUS (Jefferson 1987) oder GFP (Sheen *et al*., 1995) zur Identifizierung eines Promotors eingesetzt.

Solche Promotoren können dann in Kombination mit einem Reportergen in Screening-Systemen zur Auffindung chemischer Induktoren von Resistenz eingesetzt werden (WO 95 / 19443; DE 4446342).

### #634 (SEQ ID NO: 139)

Ein cDNA-Fragment (#634), das in infizierten Blättern sechs Tage nach TMV-Inokulation stärker exprimiert ist als in nicht infizierten Blättern, wurde in pCRII kloniert und sequenziert. Sequenzvergleiche (GCG) mit diesem aus 565 Basenpaaren (bp) bestehenden Fragment zeigten die größte Ähnlichkeit mit SFR1 (66% Übereinstimmung auf Nukleinsäureebene und 73 % in einem Bereich von 174 Aminosäuren). SFR1 (EMBL accession number Y14285) ist ein Rezeptor der S Gen-Familie aus Brassica oleracea und gehört zu den "plant receptor-like kinases". Einige Mitglieder dieser heterologen Gruppe reagieren auf Pathogenkontakt und/oder Salicylsäure, ihre Beteiligung an pflanzlichen Signaltransduktionswegen bei Abwehrreaktionen ist sehr wahrscheinlich (Pastuglia *et al*., 1997).

Die Isolation eines derartigen Gens, oder der entsprechenden vollständigen cDNA aus Tabak kann wie im folgenden beschrieben erreicht werden. Mit Hilfe eines RACE-Kits (z.B.Marathon™ cDNA Amplification Kit, Kat.Nr. K1802-1, Fa. Clontech, Palo Alto, CA, USA) kann ein vollständiger cDNA-Klon der Kinase erhalten werden. Oder man sucht in einer käuflichen Tabak cDNA Bank (Kat.Nr. FL1071b, Fa. Clontech) mit dem Fragment #634 einen solchen Klon. Diese cDNA kann zum Beispiel unter der Kontrolle eines konstitutiven Promotors, z.B. dem 35S CaMV Promotor, oder unter induzierbaren Promotoren z.B. dem der Stilbensynthase (US 5,689,046 und US 5,689,047) oder z.B. dem Chitinasepromotor (DE 40 31 758) zur Expression gebracht werden. Die Transformation kann zum Beispiel an Blattscheiben oder anderen Explantaten mit Hilfe von *Agrobacterium tumefaciens* ( US 5,689,046) oder mit anderen wohlbekannten Transformationsmethoden (US 5,689,046) erfolgen. Regenerierte Pflanzen werden, nach Überprüfung auf stabile Transformation, mit TSWV und anderen Pathogenen inokuliert und auf erhöhte Resistenz (wie in US 5,569,823) untersucht. Mit Hilfe von GCG erfolgt die Suche nach typischen Promotorelementen, in der 5' nicht translatierten Region des Gens. Unterschiedlich lange 5'-untranslatierte Regionen werden in Kombination mit einem Reportergen wie z.B. GUS (Jefferson 1987) oder GFP (Sheen *et al*., 1995) zur Identifizierung eines Promotors eingesetzt.

Solche Promotoren können dann in Kombination mit einem Reportergen in Screening-Systemen zur Auffindung chemischer Induktoren von Resistenz eingesetzt werden (WO 95 / 19443; DE 4446342).

### #107 (SEQ ID NO: 22)

Ein cDNA Fgment (#107), das in infizierten Blättern von Samsun Pflanzen einen Tag nach TMV-Inokulation stärker exprimiert ist, wurde in pGEM-T kloniert und sequenziert. Sequenzvergleiche (GCG) mit diesem 551 Nukleotide umfassenden Fragment weisen auf eine Ähnlichkeit mit einem Homeoboxgen (levahox1) aus Tomate hin (Tornero *et al*., 1996). Es wurden 87% Übereinstimmung in einem Bereich von 119 bp gefunden bzw. 87% bei der Betrachtung eines 38 Aminosäuren langen Bereichs.

Levahox1 (EMBL Accession Nr. X94947) kodiert ein Homeodomain-Protein, das außerdem ein Leuzin-Zipper Motiv enthält. Diese Kombination wurde bislang nur bei Transkriptionsfaktoren dikotyler Pflanzen gefunden. Die Expression von levahox1 während des sekundären Dickenwachstums, einem Prozeß bei dem große Mengen Lignin hergestellt werden, legt nahe, daß dieses Protein bei der Zellwandverstärkung nach Pathogenbefall beteiligt sein kann, und so der Abwehr des Pathogens dient.

Die Isolation eines derartigen Gens, oder der entsprechenden vollständigen cDNA aus Tabak kann wie im folgenden beschrieben erreicht werden. Mit Hilfe eines CE-Kits (z.B.Marathon™ cDNA Amplification Kit, Kat.Nr. K1802-1, Fa. Clontech, Palo Alto, CA, USA) kann ein vollständiger cDNA-Klon der Kinase erhalten werden. Oder man sucht in einer käuflichen Tabak cDNA Bank (Kat.Nr. FL1071b, Fa. Clontech) mit dem Fgment #107 einen solchen Klon. Diese cDNA kann zum Beispiel unter der Kontrolle eines konstitutiven Promotors, z.B. dem 35S CaMV Promotor, oder unter induzierbaren Promotoren z.B. dem der Stilbensynthase (US 5,689,046 und US 5,689,047) oder z.B. dem Chitinasepromotor (DE 40 31 758) zur Expression gebracht werden. Die Transformation kann zum Beispiel an Blattscheiben oder anderen Explantaten mit Hilfe von *Agrobacterium tumefaciens* ( US 5,689,046) oder mit anderen wohlbekannten Transformationsmethoden (US 5,689,046) erfolgen. Regenerierte Pflanzen werden, nach Überprüfung auf stabile Transformation, mit TSWV und anderen Pathogenen inokuliert und auf erhöhte Resistenz (wie in US 5,569,823) untersucht. Mit Hilfe von GCG erfolgt die Suche nach typischen Promotorelementen, in der 5' nicht translatierten Region des Gens. Unterschiedlich lange 5'-untranslatierte Regionen werden in Kombination mit einem Reportergen wie z.B. GUS (Jefferson 1987) oder GFP (Sheen *et al*., 1995) zur Identifizierung eines Promotors eingesetzt.

Solche Promotoren können dann in Kombination mit einem Reportergen in Screening-Systemen zur Auffindung chemischer Induktoren von Resistenz eingesetzt werden (WO 95 / 19443; DE 4446342).

### #116(SEQ ID NO: 28)

Ein cDNA Fragment (#116), das in Blättern von transgenen Pflanzen einen Tag nach TMV-Inokulation stärker exprimiert ist, wurde in pGEM-T kloniert und sequenziert. Sequenzvergleiche (GCG) mit diesem Fragment weisen auf eine Ähnlichkeit mit einem disease resistance response gene aus Erbse hin (EMBL Accession Number M18250). Es wurden 78 % Übereinstimmung in einem Bereich von 106 Basenpaaren gefunden.

Die Isolation eines derartigen Gens, oder der entsprechenden vollständigen cDNA aus Tabak kann wie im folgenden beschrieben erreicht werden. Mit Hilfe eines RACE-Kits (z.B.Marathon™ cDNA Amplification Kit, Kat.Nr. K1802-1, Fa. Clontech, Palo Alto, CA, USA) kann ein vollständiger cDNA-Klon der Kinase erhalten werden. Oder man sucht in einer käuflichen Tabak cDNA Bank (Kat.Nr. FL1071b, Fa. Clontech) mit dem Fragment #116 einen solchen Klon. Diese cDNA kann zum Beispiel unter der Kontrolle eines konstitutiven Promotors, z.B. dem 35S CaMV Promotor, oder unter induzierbaren Promotoren z.B. dem der Stilbensynthase (US 5,689,046 und US 5,689,047) oder z.B. dem Chitinasepromotor (DE 40 31 758) zur Expression gebracht werden. Die Transformation kann zum Beispiel an Blattscheiben oder anderen Explantaten mit Hilfe von *Agrobacterium tumefaciens* ( US 5,689,046) oder mit anderen wohlbekannten Transformationsmethoden (US 5,689,046) erfolgen. Regenerierte Pflanzen werden, nach Überprüfung auf stabile Transformation, mit TSWV und anderen Pathogenen inokuliert und auf erhöhte Resistenz (wie in US 5,569,823) untersucht . Mit Hilfe von GCG erfolgt die Suche nach typischen Promotorelementen, in der 5' nicht translatierten Region des Gens. Unterschiedlich lange 5'-untranslatierte Regionen werden in Kombination mit einem Reportergen wie z.B. GUS (Jefferson 1987) oder GFP (Sheen *et al*., 1995) zur Identifizierung eines Promotors eingesetzt.

Solche Promotoren können dann in Kombination mit einem Reportergen in Screening-Systemen zur Auffindung chemischer Induktoren von Resistenz eingesetzt werden (WO 95 / 19443; DE 4446342).

### #674(SEQ ID NO: 165)

Ein cDNA-Fragment ( # 674), das in nicht infizierten Blättern sechs Tage nach TMV-Inokulation stärker exprimiert ist, wurde in pCR2.1 kloniert und sequenziert.

Sequenzvergleich (GCG) mit diesem aus 456 Basenpaaren bestehenden Fragment zeigten Ähnlichkeit mit einem Calmodulin-bindenden-Kanalprotein (CaMB-channel protein; U65390). Es wurde eine Übereinstimmung von 43% in einem Bereich von 119 Aminosäuren gefunden. Kalziumionen und Calmodulinmoleküle sind an zahlreichen Signaltransduktionswegen in Tieren und Pflanzen beteiligt.

Die Isolation eines derartigen Gens, oder der entsprechenden vollständigen cDNA aus Tabak kann wie im Folgenden beschrieben erreicht werden. Mit Hilfe eines RACE-Kits (z.B. Marathon™ cDNA Amplification Kit, Kat.Nr. K1802-1, Fa. Clontech, Palo Alto, CA, USA) kann ein vollständiger cDNA-Klon der Kinase erhalten werden. Oder man sucht in einer käuflichen Tabak cDNA Bank (Kat.Nr. FL1071b, Fa. Clontech) mit dem Fragment #674 einen solchen Klon. Diese cDNA kann zum Beispiel unter der Kontrolle eines konstitutiven Promotors, z.B. dem 35s CaMV Promotor, oder unter induzierbaren Promotoren z.B. dem der Stilbensynthase US 5,689,046 und US 5,689,047 oder z.B. dem Chitinasepromotor DE 4031758 zur Expression gebracht werden. Die Transformation kann zum Beispiel an Blattscheiben oder anderen Explantaten mit Hilfe von *Agrobacterium tumefaciens* ( US 5,689,046) oder mit anderen wohlbekannten Transformationsmethoden (US 5,689,046) erfolgen. Regenerierte Pflanzen werden, nach Überprüfung auf stabile Transformation, mit TSWV und anderen Pathogenen inokuliert und auf erhöhte Resistenz (wie in US 5,569,823) untersucht. Mit Hilfe von GCG erfolgt die Suche nach typischen Promotorelementen, in der 5' nicht translatierten Region des Gens. Unterschiedlich lange 5'-untranslatierte Regionen werden in Kombination mit einem Reportergen wie z.B. GUS (Jefferson 1987) oder GFP (Sheen *et al*., 1995) zur Identifizierung eines Promotors eingesetzt.

Solche Promotoren können dann in Kombination mit einem Reportergen in Screening-Systemen zur Auffindung chemischer Induktoren von Resistenz eingesetzt werden (WO 95 / 19443; DE 4446342).

### Beispiel (i)

### Verwendete Pflanzen und Viren

Verwendet wurden 4 - 6 Wochen alte Tabakpflanzen des Typs Samsun NN und transgene Pflanzen in denen ein virales Protein konstitutiv exprimiert wird (US 5,569,823).

Pro Blatt wurden je 100 ml TMV (Kat.Nr. PV-0055, DSM, Braunschweig, Deutschland) mechanisch bei einer Konzentration von 0.075 mg/ml aufgebracht, so daß ca. 100 bis 300 Läsionen entstehen konnten. TRV (Tobacco Rattle Virus) wurde bei einer Konzentration von 0.38 - 1.9 ng/ml aufgebracht.

### Beispiel (ii)

### Klonierung

Zur Klonierung von cDNA-Fragmenten wurden z. B. folgende Vektoren verwendet: pGem-T (Fa. Promega, Heidelberg, Deutschland), pCRII, pCRIITopo, pCR2.1, pCR2.1Tope (Fa. Invitrogen, Carlsbad, Ca, USA)

### Beispiel (iii)

### RNA-Präparation

Gesamt-RNA wurde wie bei Chirgwin *et al*., (1979) beschrieben isoliert. Blätter wurden in flüssigem Stickstoff zu feinem Pulver zermahlen und in Zentrifugenröhrchen überführt, in denen pro 1g Pulver 3 ml GTC-Puffer (4 M Guanidiniumthiocyanat, 25 mM Natriumcitrat, pH 7.0, 0.5% Lauroylsarcosin) vorgelegt worden waren. Nach gründlicher Durchmischung erfolgte eine 20 minütige Inkubation bei Raumtemperatur während der die Röhrchen über Kopf gedreht wurden. Das Homogenat wurde 20 Minuten bei 8000 rpm und 4 °C zentrifugiert, der Überstand abgenommen und auf ein Kissen von 1.2 ml CsCl-Lösung (5.7 M) geschichtet. Zentrifugiert wurde in einem SW55Ti Rotor bei 45000 rpm und 20 °C für mindestens 12 h. Der Überstand wurde dekantiert und Polysaccharide durch zweimaliges Waschen des Pellets mit je 100 ml Na-Actetat (3 M, pH 4.8) entfernt. Das RNA Pellet wurde in 200 ml H₂O aufgenommen und die RNA durch Zugabe von 1/20stel Volumen1 M Essigsäure und 1 Volumen 100%igem Ethanol gefällt (mind. 2h,- 20 °C). Nach 10 minütiger Zentrifugation bei 4 °C wurde das Pellet mit 75% Ethanol gewaschen, getrocknet und in 100 bis 200 ml H₂O resuspendiert. Die Konzentrationsbestimmung der Gesamt-RNA erfolgte spektrophotometrisch.

### Beispiel (iv)

### Differential Display Technik

Der systematische Vergleich der Genexpressionsmuster in Wildtyp (Samsun NN) und transgenem Tabak wurde mittels Differential Display (Liang und Pardee 1992, Bauer *et al*., 1993) durchführt. Pflanzen wurden aus steriler Gewebekultur und aus dem Gewächshaus benutzt. Gesamt-RNA wurde aus nicht infizierten Pflanzen und aus Pflanzen ein, drei und sechs Tage nach TMV-Inokulation sowohl aus den infizierten Blättern (5. und 6. Blatt) als auch aus den nicht infizierten Folgeblättern (7. und 8. Blatt) der beiden Pflanzentypen isoliert. Die RNAs wurden nach den Anweisungen des Differential Display Kits Version 1.0 und 1.1 der Firma Display Systems Tandil Ltd., London, England untersucht. In frühen Experimenten wurde abweichend von diesen Anweisungen auch Primer, die am 3'-Ende binden, vereinigt und gemeinsam eingesetzt, so wie bei Liang *et al*. (1993) beschrieben. Die vereinigten Primer hatten die Sequenz (T)₁₂ A/C/G-A sowie (T)₁₂ A/C/G-G und (T)₁₂ A/C/G-C.

Die cDNA Synthese erfolgte mit M-MLV-Reverser Transktriptase (Kat. Nr. 28025-013, Fa. Gibco BRL, Eggenstein, Deutschland). Die Polymerasekettenreaktionen (DD-PCR) wurden unter Verwendung der nativen Taq-DNA-Polymerase der Fa. Perkin Elmer, Vaterstetten, Deutschland (Kat.-Nr. 801-0043) oder Gibco BRL (Kat.Nr. 18038) sowie mit Redivue alpha ³³P markiertem dATP (Fa.Amersham, Braunschweig, Deutschland) in einem GeneAmp PCR System 2400 Thermocyler (Fa. Perkin Elmer) durchgeführt (40 Zyklen, unter Bedingungen wie in der Anleitung der Fa. Tandil angegeben).

Die PCR-Produkte wurden in einem denaturierenden 6%igen Sequenziergel getrennt. Zur Auffindung reproduzierbarer Unterschiede in den Genexpressionsmustern wurden in der Regel Doppelansätze nebeneinander aufgetragen, bei denen jeder von einer unterschiedlichen RNA Präparation stammte. Für jede Primerkombination und jeden Tageswerte wurden die Expressionsmuster in infizierten und nicht infizierten Blättern von transgenen Pflanzen mit den Mustern der entsprechenden Blätter von Samsun NN (Wildtyp) verglichen. Außerdem wurden die Muster in infizierten Blättern von Samsun NN und transgenen Pflanzen mit den Mustern nicht infizierter Blätter beider Pflanzentypen verglichen.

### Beispiel (v)

### Klonierung von cDNA-Fragmenten differentiell exprimierter mRNAs

PCR-Produkte unterschiedlich exprimierter cDNA Fragmente aus dem DD wurden reamplifiziert. Dazu wurden die oben genannten Sequenziergele auf Whatman-3MM Papier 1 - 2 Stunden bei 80 °C vakuumgetrocknet. Über Nacht erfolgte die Exposition mit Röntgenfilmen (X-OMAT AR, Fa. Eastman Kodak, Rochester, NY, USA). Signale auf diesen Filmen wurden zur Lokalisierung der Fragmente benutzt und diese bei deutlichen Unterschieden ausgeschnitten. Die ausgeschnittenen Fragmente wurden in 100 ml H₂O 10 min bei Raumtemperatur quellen gelassen, 10 min bei 100 °C inkubiert und dann mit Ethanol gefällt. Das Pellet wurde in 10 ml H₂O aufgenommen. Dann wurden die cDNA Fragmente mittels PCR amplifiziert, sofort kloniert und sequenziert.

### Beispiel (vi)

### Northern-Blots

Die RNA-Proben (20 - 40 mg Gesamt RNA oder 1-5 mg polyA⁺ RNA) wurden wie bei Sambrook *et al*. (1989) beschrieben in Formaldehydgelen getrennt. Anschließend erfolgte der Kapillartransfer auf Hybond N-Membranen (Fa. Amersham). Die Hybridisierungstemperaturen wurden für jedes cDNA Fragment einzeln bestimmt (Sambrook *et al*., 1989). Die cDNA Fragmente wurden als ³²P markierte Sonden eingesetzt. Die Markierung erfolgte z.B. über in vitro-Transkription. Dabei wurden entweder die T7 RNA-Polymerase (Kat.Nr. 881-767, Fa. Boehringer Mannheim, Mannheim, Deutschland) oder die SP6 RNA-Polymerase (Kat.Nr. 810-274, Fa. Boehringer Mannheim) verwendet. Einige Sonden wurden mit dem random prime labelling kit (Kat. Nr. 1004-760, Fa. Boehringer Mannheim) markiert. Darüber hinaus wurden Sonden mittels PCR hergestellt. Quantitative Unterschiede der einzelnen radioaktiven Signale wurden mit einem radioanalytischen Imaging-System (BAS1000, Fa. Raytest, Straubenhardt, Deutschland) ermittelt. Dadurch wurde ein Maximum an Nachweisempfindlichkeit erreicht.

### Beispiel (vii)

### Bioinformatik

Bioinformatik wurde mit dem GCG Software-Paket der Universität Wisconsin, Madison, Wi, USA durchgeführt. Für die Ähnlichkeitsanalyse wurden die Programme BLAST und FASTA benutzt. Ebenso wurde das Expertensystem bioSCOUT der Firma Lion, Heidelberg, Deutschland, verwendet.

### Beispiel (viii)

### Quantitative PCR

RT-PCR wurde mit dem SuperScript OneStep RT-PCR Kit der Fa. GibcoBRL Life Technologies (Eggenstein, Deutschland, Kat.Nr. 10928-018) durchgeführt. 100 ng RNA wurden in die RT-PCR eingesetzt. Die cDNA-Synthese erfolgte bei 50 °C für 30 min. Nach einem 2 minütigem Denaturierungsschritt bei 94 °C folgten 25 - 30 Zyklen der PCR-Amplifikation: 15 s bei 94 °C, 30 s bei 55°C und 30 s bei 72 °C in einem GeneAmp PCR System 2400 Thermocyler (Fa. Perkin Elmer). Nach dem letzten Zyklus wurde die Reaktion noch 7 min bei 72 °C inkubiert.

### Beispiel (ix)

### RACE

Mit Hilfe eines RACE-Kits (Marathon™ cDNA Amplification Kit, Kat.Nr. K1802-1, Fa. Clontech, Palo Alto, CA, USA) kann ein vollständiger cDNA-Klon von dem aus dem DD Experiment stammenden cDNA-Fragment isoliert werden. Es gibt zwei mögliche RACE (random amplification of cDNA ends)-Produkte, zum einen 5' cDNA Enden ( 5'RACE) und zum anderen 3' cDNA Enden (3'RACE). Zunächst wird ausgehend von poly A ⁺ RNA eine Erst- und Zweitstrang cDNA Synthese durchgeführt. An die doppelsträngige cDNA werden dann Adaptoren ligiert. Diese - mit Adaptoren versehene - cDNA-Bank wird zur Isolierung vollständiger cDNAs verwendet.

Im nächsten Schritt wird ein genspezifischen Primer ( minimale Sequenzinformation 28 Nukleotide) an den Sinn- oder Gegensinnstrang der cDNA hybridisiert. Nach Verlängerung dieses Primers in 5'- oder in 3' -Richtung der cDNA wird eine Bindungsstelle für den entsprechenden Adaptorprimer am 5'- oder 3' - Ende erzeugt. Mit Hilfe beider Primer erfolgt dann im eigentlichen RACE PCR-Experiment die Amplifikation beider Teile der cDNA.

### Beispiel (x)

### Salicylsäure-Bestimmung

### Pflanzenanzucht und Infektion

4 Wochen alte Tabakpflanzen wurden mit TMV bzw. TSWV infiziert.

Dazu wurde jeweils das 4. Blatt mit 100 ml Virus infiziert. TSWV wurde aus einem frischen Extrakt aus einer durchinfizierten Rustika-Pflanze gewonnen (ca. 1g Blattmaterial in 50 ml Viruspuffer gemörsert). TMV wurde mit 0,075 mg/ml (Prot.äquiv.) eingesetzt.

Die Blätter (ohne Mittelrippe) von je 3 Pflanzen wurden gepoolt, in flüssigem Stickstoff schockgefroren und bis zur Aufarbeitung bei -80 °C gelagert.

### Messung der Salicylsäure

Um den Salicylsäuregehalt (SA-Gehalt) zu bestimmen, wurde ein methanolischer Extrakt hergestellt. Mittels einer Flüssig/Flüssig-Extraktion wurde die freie Salicylsäure (in der organischen Phase) und das Salicylsäure-Glycosid (in der wässrigen Phase) getrennt. Durch saure Hydrolyse wurde das Glycosid gespalten und die freie Säure durch eine zweite Extraktion von den polaren Substanzen getrennt. Die freie Salicylsäure und die aus dem Glycosid freigesetzte Säure wurden mittels HPLC und einem Fluoreszenzdetektor quantifiziert.

### Durchführung:

Ein Gramm Blattgewebe wurde mit 0,1 g Seesand in flüssigem Stickstoff gemörsert und in 3 ml 90 % Methanol aufgenommen. Nach 20 minütigem Rühren (Magnetrührer) wurde das Homogenat 20 min mit 10000 g zentrifugiert. Das Pellet wurde in 3 ml 100 % Methanol aufgenommen (20 min rühren) und anschließend zentrifugiert. Die Überstände der 1. und 2. Zentrifugation wurden vereinigt und mittels Vakuumzentrifugation eingeeingt. Die getrockneten Extrakte wurden in 1 ml 5%iger Trichloressigsäure aufgenommen; unlösliche Bestandteile wurden abzentrifugiert. Der lösliche Anteil wurde auf eine Extrelut-Säule (Fa. Merck, Darmstadt, Deutschland) aufgetragen. Die hydrophoben Bestandteile wurden mit 6 ml Ethylacetat/Cyclopentan/Isopropanol (50/50/1) eluiert. Das Eluat, welches die freie Salicylsäure enthält, wurde unter N₂ eingedampft, in 500 ml 50% Acetonitril (+ 0,06% Trifluoressigsäure) aufgenommen und steril filtriert.

Die (glykosidisch) gebundene Salicylsäure wurde wie folgt bestimmt: Nachdem die hydrophoben Bestandteile von der Extrelut-Säule eluiert worden waren, wurden die polaren Substanzen mit 6 ml Methanol eluiert. Das Eluat wurde unter N₂ eingedampft, in 1 ml 0,5 N Salzsäure aufgenommen und eine Stunde bei 100 °C hydrolysiert. Das Hydrolysat, welches die freigesetzte Salicylsäure enthält, wurde auf eine neue Extrelutsäule aufgetragen und wie oben beschrieben weiterbehandelt. Mittels HPLC und einem Fluoreszenzdetektor wurden die Menge an freier Salicylsäure und die Menge an freigesetzter Salicylsäure im Blattmaterial bestimmt.
HPLC: Series 1050 (Fa. Hewlett Packard, Böblingen, Deutschland)
Säule: Vydac 201 TP 10mm, RP18, 25 x 4,6 mm + entspr. Vorsäule (Fa. Alltech, Deerfield, Il, USA)
Laufmittel A: 0,06% TFA
Laufmittel B: 70% Acetonitril + 0,056% TFA
Flußrate: 1 ml/ min, 40 °C
Programm:

| | |
|---|---|
| 0 min | 30 % B |
| - 15 min | 30 % B |
| - 18 min | 100 % B |
| - 23 min | 100 % B |
| - 25 min | 30 % B |
| - 30 min | 30 % B |

Fluoreszenzdetektor:LS30 (Fa. Perkin Elmer)
Excitation: 300 nm
Emission: 430 nm

### Literatur:

Alexander D, Goodman RM, Gut-Rella M, Glascock C, Weymann K, Friedrich, L, Maddox D, AhlGoy P, Luntz: T, Ward E, Ryals J (1993)
   Increased tolerance to two oomycete pathogens in transgenic tobacco expressing pathogenesis-related protein 1a
   Proc. Natl. Acad. Sci. USA 90, 7327 - 7331
Bauer D, Müller H, Reich J, Riedel H, Ahrenkiel V, Warthoe P, Strauss M (1993)
   Identification of differentially expressed mRNA species by an improved display technique (DDRT-PCR)
   Nucleic Acids Research 21, 4272 - 4280
Bowling SA, Clarke JB, Liu Y, Klessig DF und Dong X (1997)
   The *cpr5* mutant of Arabidopsis expresses both NPR-1 dependent and NPR-1 independent resistance
   The Plant Cell 9, 1573-1584
Bowling SA, Guo A, Cao H, Gordon AS, Klessig DF, Dong XI (1994)
   A mutation in Arabidopsis that leads to constitutive expression of systemic acquired-resistance
   Plant Cell 6, 1845-1857
Chirgwin JM, Przybyla AE, McDonald RJ, Rutter WJ (1979)
   Isolation of biologically active ribonucleic acid from sources enriched in ribonuclease
   Biochemistry 18, 5294 - 5299
Enyedi AJ, Yalpani N, Silverman P, Raskin I (1992)
   Localization, conjugation, and function of salicylic acid in tobacco during the hypersensitive reaction to tobacco mosaic virus.
   Proc.Natl.Acad.Sci. 89, 2480 - 2484
Gianinazzi S, Kassanis B (1974)
   Virus resistance induced in plants by polyacrylic acid
   J Gen Virol 23, 1-9
Görlach J, Volrath S, Knauf-Beiter G, Hengy G, Beckhove U, Kogel KH, Oostendorp M, Staub T, Ward E, Kessmann H, Ryals J (1996)
   Benzothiadiazol, a novel class of inducers of systemic aquired resistance, activates gene expression and disease resistance
   The Plant Cell 8, 629-64
Heller WE, Gessler CJ (1986)
   Induced systemic resistance in tomato plants against Phytophtora infestans
   J. Phytophatology 116, 323-328
Jefferson RA (1987)
   Assaying chimeric genes in plants: the GUS gene fusion system
   Plant Mol. Biol. Rep. 5,387 - 405
Kogel KH, Beckhove U, Dreschers J, Münch S, Rommé Y (1994)
   Aquired resistance in barley
   Plant Physiology 106, 1269 - 1277
Liang P und Pardee AB (1992)
   Differential Display of Eukariotic Messenger RNA by Means of Polymerase Chain Reaction
   Science 257, 967-971
Madamanchi NR, Kuc J (1991)
   Iduced systemic resistance in plants
   in: The Fungal Spore and Disease Initiation in Plants and Animals
   eds.: Cole GT and Hoch HC (Plenum Publ Corp NY) 347-362
Malamy J, Carr JP, Klessig DE, Raskin J (1990)
   Salicylic acid - a likely endogenous signal in the resistance response of tobacco to virus infection
   Science 250, 1002-1004
Martin GB, Brommonschenkel SH, Chunwongse J, Fray A, Ganal MW, Spivey R, Wu T, Earle ED und Tanksley SD (1993)
   Map based Cloning of a Protein Kinase Gene Conferring Disease Resistance in Tomato
   Sience 262, 1432-1436
Mauch-Mani B and Slusarenko AJ (1994)
   Systemic aquired resistance in *Arabidopsis thaliana* induced by a predisposing infection with a pathogenic isolate of *Fusariom oxysporum*
   Molecular Plant-Microbe Interaction 7: 378 - 383
Metraux JP, Signer H, Ryals J, Ward E, Wyss-Benz M, Gardin J, Raschdorf K, Schmid E, Blum W, Inverardi B (1990)
   Increase in salicylic acid at the onset of systemic acquired resistance in cucumber
   Science 250, 1004-1006
Metraux JP, Ahl-Goy P, Staub T, Speich J, Steinemann A,
   Ryals J, Ward E (1991)
   Coordinate gene activity in response to agents that induce systemic acquired resistance
   In Advances in Molecular Genetics of Plant- Microbe interactions, Vol 1 eds.: Hennecke H and Verma DPS 432-439
Pastuglia M, Roby D, Dumas C, Cock, JM (1997)
   Rapid Induction by Wounding and Bacterial Infection of an S Gene Family Receptor-like Kinase Gene in Brassica oleracea
   Plant Cell 9, 49 - 60
Ross AF (1961)
   Systemic acquired resistance induced by a localized virus infection in plants
   Virology 14, 340-358
Sambrook J, Fritsch EF,Maniatis T (1989)
   Molecular Cloning -A Laboratory Manual- Second Edition , Cold Spring Harbor Laboratory Press, USA
Schaffrath U, Freydl E, Dudler R (1997)
   Evidence for different signal pathwys activated by inducers of aquired resistance in wheat
   MPMI 10, 779-783
Sheen J, Hwang S, Niwa Y, Kobayashi H, Galbraith DW (1995)
   Green-fluorescent protein as a new vital marker in plant cells
   Plant Journal 8 (5), 777 - 784
Sequeira L (1983)
   Mechanisms of induced resistance in plants
   Annu Rev Microbiol 37, 51-79
Smith JA, Métraux JP (1991)
   *Pseudomonas syringae* pv. *syringae* induces systemic resistance to *Pyricularia oryzae* in rice
   Physiol. Mol. Plant. Pathol. 39, 451 - 461
Tornero P, Conejero V, Vera P (1996)
   Phloem-specific expression of a plant homeobox gene during secondary phases of vascular development
   The Plant Journal 9, 639 - 648
Vernooij B, Friedrich L,Morse A, Reist R,Kolditz-Jawhar, Ward E, ukness S, Kessmann H, Ryals J (1994)
   Salicylic acid is not the translocated signal responsible for inducing systemic acqired resistance but is required in signal trasnduction
   The Plant Cell 6, 959-965
Vidal S, de Leon IP, Denecke J, Palva ET (1997)
   Salicylic acid and the plant pathogen *Erwinia caratovora* induce defence genes via antagonistic pathways
   The Plant J 11, 115-123
Ward E, Uknes SJ, Williams SC, Dincher SS, Wiederhold DL, Alexander DC, Ahl-Goy P, Metraux JP, Ryals JA (1991)
   Coordinate gene activity in response to agents that induce systemic acquired resistance
   The Plant Cell 3, 1085-1094
White RF (1979)
   Acetylsalicylic acid (Aspirin) induces resistance to tobacco mosaic virus in tobacco
   Virology 99, 410-412
Yalpani N, Silverman P, Wilson TMA, Kleier DA, Raskin I (1991)
   Salicylic Acid Is a Systemic Signal and an Inducer of Pathogenesis-Related Proteins in Virus-Infected Tobacco Plant Cell 3, 809 - 818.

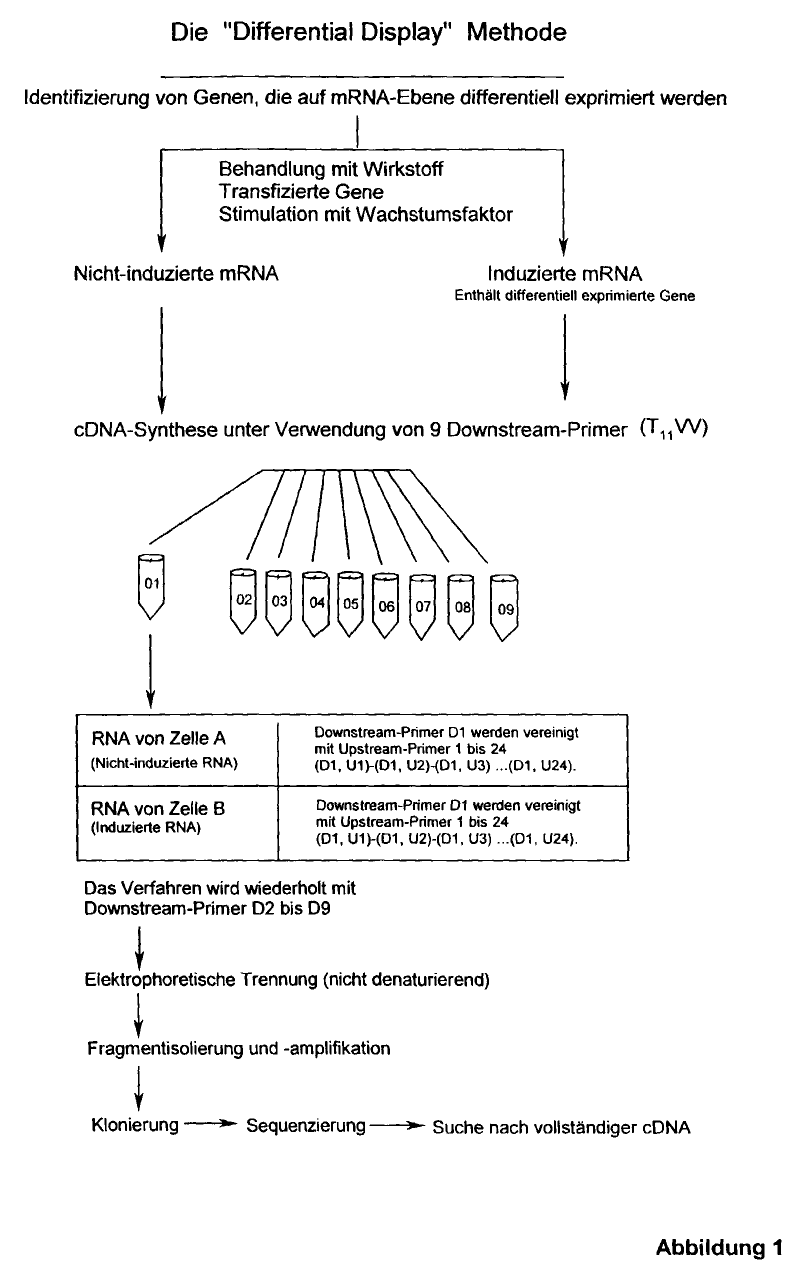

## Patentansprüche

1. Nukleinsäure umfassend eine Sequenz ausgewählt aus
(a) den Sequenzen gemäß SEQ ID NO: 1 bis SEQ ID NO: 213,
(b) zumindest 14 Basenpaare langen Teilsequenzen der unter (a) definierten Sequenzen,
(c) Sequenzen, welche an die unter (a) oder (b) definierten Sequenzen hybridisieren,
(d) Sequenzen, welche eine zumindest 70%ige Homologie zu den unter (a) definierten Sequenzen aufweisen,
(e) Sequenzen, welche zu den unter (a) definierten Sequenzen komplementär sind und
(f) Sequenzen, welche aufgrund der Degeneration des genetischen Codes für dieselbe Aminosäuresequenz kodieren wie die unter (a) bis (d) definierten Sequenzen.

2. Vektor umfassend zumindest eine Nukleinsäure gemäß Anspruch 1.

3. Vektor nach Anspruch 2, dadurch gekennzeichnet, daß das Nukleinsäuremolekül funktionell mit regulatorischen Sequenzen verknüpft ist, die die Expression der Nukleinsäure in pro- oder eukaryotischen Zellen gewährleisten.

4. Wirtszelle enthaltend eine Nukleinsäure gemäß Anspruch 1 oder einen Vektor gemäß Anspruch 2 oder 3.

5. Wirtszelle nach Anspruch 4, dadurch gekennzeichnet, daß es sich um eine pro- oder eukaryotische Zelle handelt.

6. Wirtszelle nach Anspruch 5, dadurch gekennzeichnet, daß die prokaryotische Zelle E.coli oder Agrobacterium tumefaciens ist.

7. Wirtszelle nach Anspruch 5, dadurch gekennzeichnet, daß die eukaryotische Zelle eine Pilz-, eine Pflanzen- oder eine tierische Zelle ist.

8. Verfahren zur Herstellung eines Polypeptids, das während einer induzierten Resistenz exprimiert wird, umfassend
(a) das Kultivieren einer Wirtszelle gemäß einem der Ansprüche 4 bis 7 unter Bedingungen, die die Expression der Nukleinsäure gemäß Anspruch 1 gewährleisten, und
(b) die Gewinnung des Polypeptids aus der Zelle oder dem Kulturmedium.

9. Polypeptid, welches von einer Nukleinsäure gemäß Anspruch 1 kodiert wird oder erhältlich ist nach dem Verfahren gemäß Anspruch 8.

10. Antikörper, welcher spezifisch mit dem Polypeptid gemäß Anspruch 9 reagiert.

11. Verfahren zur Herstellung einer transgenen Pflanze, einer transgenen Pflanzenzelle oder eines transgenen Pflanzengewebes umfassend das Einbringen einer Nukleinsäure gemäß Anspruch 1 oder eines Vektors gemäß Anspruch 2 oder 3.

12. Pflanzenzelle enthaltend eine Nukleinsäure gemäß Anspruch 1 oder einen Vektor gemäß Anspruch 2 oder 3 oder erhältlich nach dem Verfahren gemäß Anspruch 11.

13. Pflanze oder Pflanzengewebe enthaltend eine Pflanzenzelle gemäß Anspruch 12.

14. Nachkommen oder Ernteprodukte der Pflanze gemäß Anspruch 13.

15. Verfahren zur Herstellung einer Hukleinsäure gemäß Anspruch 1 umfassend die folgenden Schritte:
(a) Vollständige chemische Synthese auf an sich bekannte Weise oder
(b) chemische Synthese von Oligonukleotiden, Markieren der Oligonukleotide, Hybridisieren der Oligonukleotide an DNA einer Pflanzen-cDNA-Bank, Selektieren von positiven Klonen und Isolieren der hybridisierenden DNA aus positiven Klonen oder
(c) chemische Synthese von Oligonukleotiden und Amplifizierung der Ziel-DNA mittels PCR.

16. Regulatorische Region, welche natürlicherweise die Transkription einer Nukleinsäure gemäß Anspruch 1 in pflanzlichen Zellen kontrolliert, im Zusammenhang mit einer induzierten Resistenz steht und eine spezifische Expression gewährleistet.

17. Rekombinantes DNA-Molekül umfassend eine regulatorische Region gemäß Anspruch 16.

18. Rekombinantes DNA-Molekül nach Anspruch 17, dadurch gekennzeichnet, daß die regulatorische Region funktionell mit einer heterologen DNA-Sequenz verknüpft ist.

19. Rekombinantes DNA-Molekül nach Anspruch 17 oder 18, dadurch gekennzeichnet, daß die heterologe DNA-Sequenz ein Peptid, Polypeptid, Antisense-RNA und/oder Ribozym kodiert.

20. Rekombinantes DNA-Molekül nach Anspruch 18 oder 19, dadurch gekennzeichnet, daß die heterologe DNA-Sequenz für einen selektierbaren und/oder nachweisbaren Marker kodiert.

21. Vektor enthaltend eine regulatorische Region nach Anspruch 16 oder ein rekombinates DNA-Molekül gemäß einem der Ansprüche 17 bis 20.

22. Wirtszelle, welche transformiert ist mit einer regulatorischen Region nach Anspruch 16, mit einem rekombinaten DNA-Molekül nach einem der Ansprüche 17 bis 20 oder mit einem Vektor nach Anspruch 21.

23. Verfahren zur Identifizierung von neuen Wirkstoffen für den Pflanzenschutz, insbesondere von neuen Wirkstoffen, die Resistenzinduktionen auslösen, umfassend die folgenden Schritte:
(a) Bereitstellen einer Zelle, welche ein rekombinantes DNA-Molekül gemäß Anspruch 20 umfaßt,
(b) Kultivieren der Zelle in der Gegenwart einer Verbindung oder einer Probe, welche eine Vielzahl von Verbindungen umfaßt, unter Bedingungen, die die Expression des selektierbaren und/oder nachweisbaren Markers erlauben, und
(c) Identifizieren oder Nachweisen einer Verbindung oder Probe, die zur Suppression oder Aktivierung und/oder Verstärkung der Expression des selektierbaren und/oder nachweisbaren Markers in der Zelle führt.

24. Verfahren zur Identifizierung einer Verbindung, die als Inhibitor oder Aktivator eines Polypeptids geeignet ist, das bei induzierter Resistenz eine Rolle spielt, umfassend die Schritte:
(a) Inkontaktbringen einer Wirtszelle gemäß einem der Ansprüche 4 bis 7 oder eines Polypeptids nach Anspruch 9 mit einer Verbindung oder einem Gemisch von Verbindungen unter Bedingungen, die die Interaktion der Verbindung(en) mit dem Polypeptid oder der Wirtszelle erlauben,
(b) Bestimmen der Verbindung(en), die die Aktivität des Polypeptids spezifisch beeinflussen.

25. Verfahren zur Herstellung eines Pflanzenschutzmittels umfassend die Schritte (a) bis (c) gemäß Anspruch 23 oder die Schritte (a) und (b) des Verfahrens gemäß Anspruch 24 und als weiteren Schritt die Formulierung der in diesem Verfahren identifizierten Verbindung(en) in einer Form, die die Aufnahme der Verbindung(en) und gegebenenfalls deren Metabolisierung in Organismen, insbesondere Pflanzen, phytopathogenen Pilzen, Nematoden und Schadeninsekten gewährleisten.

26. Verbindungen erhältlich nach einem der Verfahren 23 oder 24.

27. Pflanzenschutzmittel erhältlich nach dem Verfahren gemäß Anspruch 25.

28. Verwendung zumindest einer Nukleinsäure gemäß Anspruch 1, eines Vektors gemäß Anspruch 2 oder 3, einer regulatorischen Region gemäß Anspruch 16, eines rekombinanten DNA-Moleküls gemäß einem der Ansprüche 17 bis 20, eines Vektors gemäß Anspruch 21, einer Verbindung gemäß Anspruch 26 oder eines Polypeptids gemäß Anspruch 9 zur Verbesserung der Pathogenabwehr von Pflanzen oder zur Herstellung von transgenen Pflanzen.

29. Verwendung zumindest einer Nukleinsäure gemäß Anspruch 1, eines Vektors gemäß Anspruch 2 oder 3, einer regulatorischen Region gemäß Anspruch 16, eines rekombinanten DNA-Moleküls gemäß einem der Ansprüche 17 bis 20, eines Vektors gemäß Anspruch 21, einer Verbindung gemäß Anspruch 26, einer Wirtszelle gemäß einem der Ansprüche 4 bis 7, einer Pflanzenzelle gemäß Anspruch 12, einer Pflanze oder eines Pflanzengewebes gemäß Anspruch 13, einer Wirtszelle gemaß Anspruch 22, eines Polypeptids gemäß Anspruch 9 oder eines Antikörpers gemäß Anspruch 10 zum Auffinden neuer Wirkstoffe für den Pflanzenschutz oder zum Auffinden von Genen, die an der Induktion von Resistenz in Pflanzen beteiligt sind.

30. Diagnostisches Mittel enthaltend einen Vektors gemäß Anspruch 2 oder 3, eine regulatorische Region gemäß Anspruch 16, ein rekombinantes DNA-Molekül gemäß einem der Ansprüche 17 bis 20, einen Vektor gemäß Anspruch 21, eine Verbindung gemäß Anspruch 26, ein Polypeptid gemäß Anspruch 9 oder einen Antikörper gemäß Anspruch 10 und gegebenenfalls geeignete Mittel zum Nachweis.
